# EUROPEAN PATENT APPLICATION

(11) **EP 4 545 566 A2**
(43) Date of publication of application: **30.04.2025**
(21) Application number: 24220782.7
(22) Date of filing: 01.03.2021
(51) Int. Cl.: C07K 16/46

(54) **SELECTIVE MODULATION OF TRANSFORMING GROWTH FACTOR BETA SUPERFAMILY SIGNALING VIA MULTI-SPECIFIC ANTIBODIES**

(30) Priority: 28.02.2020 US 202062983374 P
(62) Divisional of application: 21760431.3
(71) Applicant: The Brigham and Women's Hospital Inc., Boston, MA 02115 (US)
(72) Inventor: YU, Paul B., Boston, 02114 (US); TRONCONE, Luca, Brookline, 02446 (US); KIM, Stephanie, Brookline, 02446 (US)
(74) Representative: Forresters IP LLP

(57) **Abstract**

Compositions and methods for selective targeting of BMP/TGFβ signaling using heteromeric complexes, preferably comprising single chain variable domain (scFv) antibodies (Abs) targeting the extracellular domains of BMP type I and type II receptors.

## Description

### CLAIM OF PRIORITY

This application claims the benefit of U.S. Provisional Patent Application Serial No. 62/983,374, filed on February 28, 2020. The entire contents of the foregoing are hereby incorporated by reference.

### FEDERALLY SPONSORED RESEARCH OR DEVELOPMENT

This invention was made with Government support under Grant Nos. AR057374 and HL131910 awarded by the National Institutes of Health. The Government has certain rights in the invention.

### TECHNICAL FIELD

Described herein are compositions and methods for selective targeting of bone morphogenic protein (BMP) / transforming growth factor-beta (TGF-β) signaling, optionally using heteromeric complexes of single chain variable domain (scFv) antibodies (Abs) targeting the extracellular domains of BMP type I and type II receptors.

### BACKGROUND

The BMP/TGF-beta/activin/growth differentiation factor (GDF) signaling pathway broadly regulates developmental patterning, and postnatal tissue remodeling (Waite and Eng, Nat Rev Genet. 2003 Oct;4(10):763-73). The 33 known ligands of this pathway interact with 5 constitutively active type II and 7 conditionally active type I receptors on the cell surface to initiate downstream signaling. This two-receptor signaling system confers combinatorial diversity (at least 5 x 7 potential heterotetrameric complexes) in receptor pairs each of which respond to limited numbers of ligands. The physiologic consequences of this signaling are highly dependent on the tissue and spatiotemporal context, and may include the modulation of bone formation or fibrosis, and more generally the regulation of cellular plasticity, cellular hypertrophy, cell proliferation, and apoptosis. Recombinant BMP/TGF-beta/activin/GDF ligands might be used therapeutically for the treatment of various disease conditions, or for tissue engineering applications. However, many of these ligands may have a short half-life in circulation, or may be sequestered in the extracellular matrix via heparin binding domains, making delivery into target tissues difficult.

TGF-beta signaling requires the cross-linking of type I and type II receptors in a multimeric complex to initiate signaling. A dimeric ligand molecule facilitates the assembly of a heteromeric complex of type II and type I receptors, wherein the constitutively active kinase domain of the type II receptor trans-phosphorylates and activates the kinase domain of the type I receptor. The type I receptor is then able to imitate signaling via multiple signaling cascades, including the SMADs. SMADs are a family of proteins similar to the gene products of the Drosophila gene 'mothers against decapentaplegic' (Mad) and the C. elegans gene Sma; the SMADs include effector proteins that mediate functions of the BMP, activin, GDF, and TGF-ligands. These SMADS, which include SMADs 2 and 3 downstream of TGF-beta and many activin and GDF ligands, and SMADs 1, 5, and 9 downstream of most BMP ligands and some GDF ligands, are phosphorylated at their C-termini by type I receptor kinases and are then selectively retained in the cell nucleus to modulate gene transcriptional activity. Other signaling pathways targeted by the BMP/TGF-beta/activin/GDF receptor complex include the MAP kinases, PI3K/Akt, protein kinase G, and other signal transduction cascades. Broadly, these ligands, receptors, and their downstream effector signals function to coordinate cellular growth, apoptosis, differentiation, and plasticity. These ligands, their receptors, co-receptors, and antagonists serve to encode spatio-temporal gradients essential for developmental patterning and tissue remodeling. These ligands and receptors also function to regulate physiology in mature organisms, including iron homeostasis. These ligands and receptors are considered multi-functional growth factor cytokines.

### SUMMARY

The BMP/TGFβ combinatorial two-receptor system permits functional specificity and spatiotemporal regulation of cellular signaling. The functions are determined by specific pairings of type II and type I receptors, which determine the activation of SMAD (SMAD1/5/9 vs. SMAD2/3) and non-SMAD (i.e., mitogen-activated protein kinase (MAPK), Phosphoinositide 3-kinase (PI3K), and/or protein kinase G (PKG)) effector pathways. Expression of type II and type I receptors is tissue-specific and dynamically regulated to permit context-sensitive function. Described herein are compositions and methods for selective targeting of BMP/TGFβ signaling using heteromeric complexes of antibodies or antigen binding fragments thereof, preferably single chain fragment variable (scFv) antibodies, targeting the extracellular domains of type I and type II receptors of the BMP/TGF-beta/activin/GDF family.

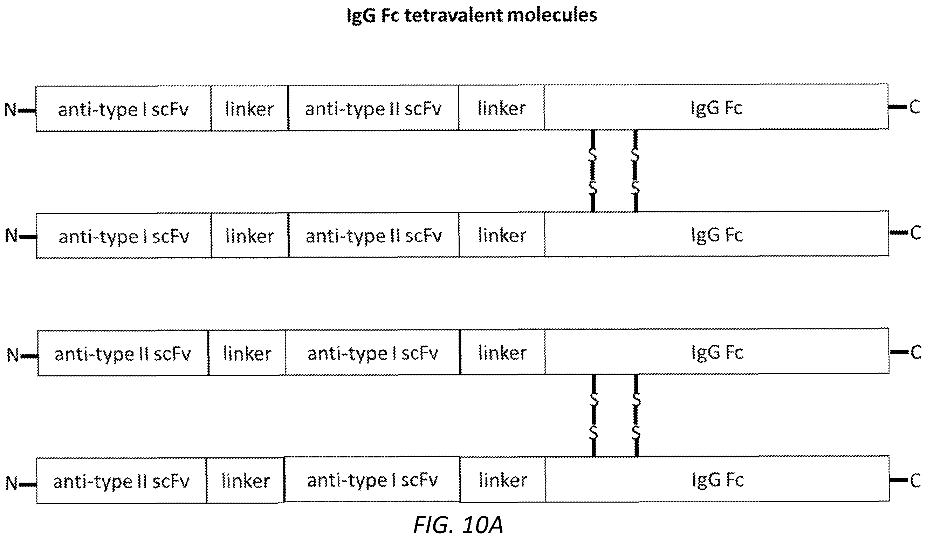

Thus, provided herein are multi- or bi-specific antibody molecules comprising at least: a first antigen binding domain that binds to a bone morphogenetic protein receptor (BMPR) type I (BMPRI); and a second antigen binding domain that binds to a bone morphogenetic protein receptor (BMPR) type II (BMPRII), wherein BMPRI or BMPRII can be taken to refer to any of the type I and type II receptors respectively of the BMP/TGF-beta/activin/GDF signaling pathway, and wherein the first and second antigen binding domains are linked together by a flexible linker, wherein each antigen binding domain can agonise the BMPR to which it binds, and preferably wherein each antigen binding domain is an scFv. The first and second antigen binding domains can be present in any order in the molecule, e.g., the molecule can comprise N-terminus- first domain - linker - second domain - C terminus, or N-terminus- second domain - linker - first domain - C terminus (see, e.g., FIGs. 10A-D). In some embodiments, the BMPRII antigen binding domain is N-terminal to the BMPRI antigen binding domain. Tetrameric or larger (e.g., hexamers, octamers, decamers, or larger) multimeric arrays are also provided comprised of first and second antigen binding domains separated by flexible linker domains, with antigen binding domains present in any order within the molecule. In general the domains are present in pairs, e.g., one BMPRI binding domain for each BMPRII binding domain, but the ratios can also vary, e.g., from 1:1 to 2:1 to 3:1 or more (in either direction).

In some embodiments, binding of the antibody molecule to a cell initiates BMP/TGF-beta/activin/GDF signaling.

In some embodiments, the first antigen binding domain binds to a BMPRI, e.g., selected from the group consisting of ALK1 (also known as activin A receptor like type 1, or ACVRL1); ALK2 (ACVR1A); ALK3 (BMPR1A); ALK4 (ACVR1B); ALK5 (TGFBR1); ALK6 (BMPR1B); or ALK7 (ACVR1C).

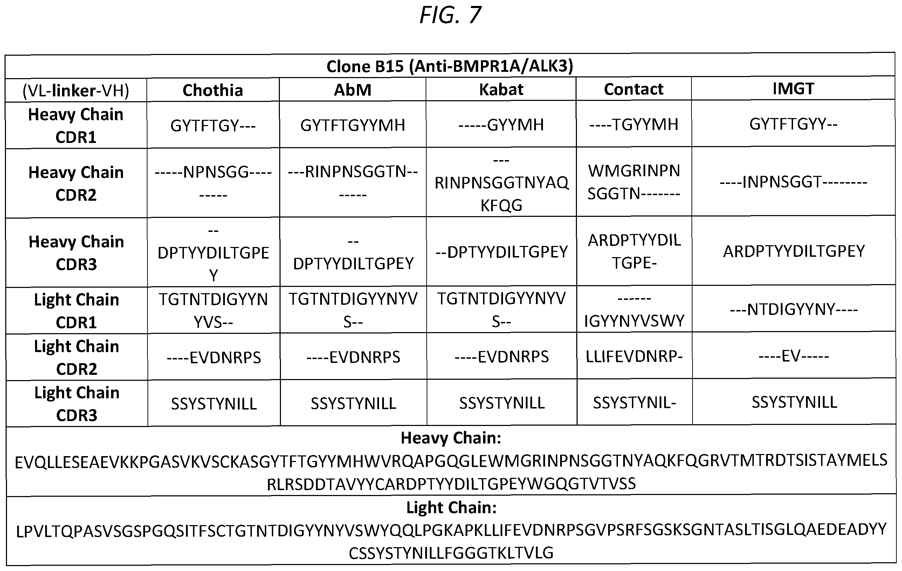

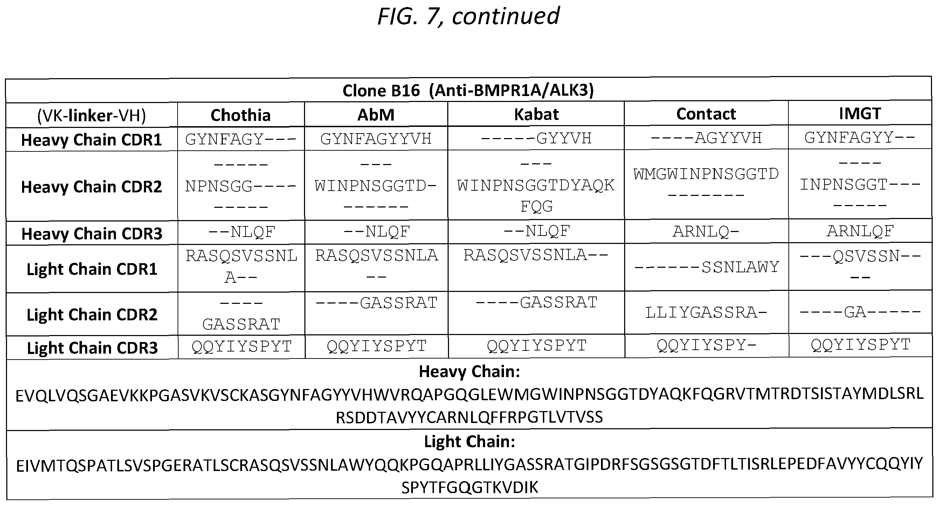

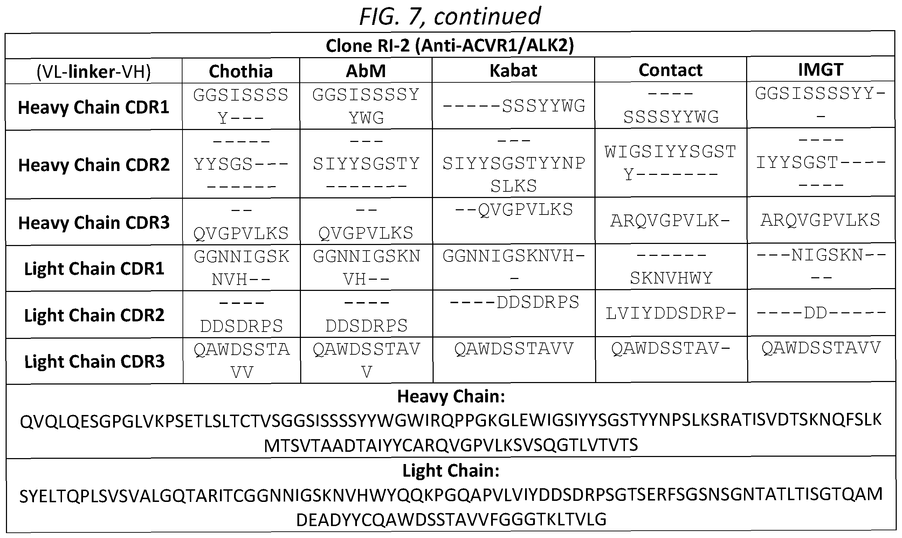

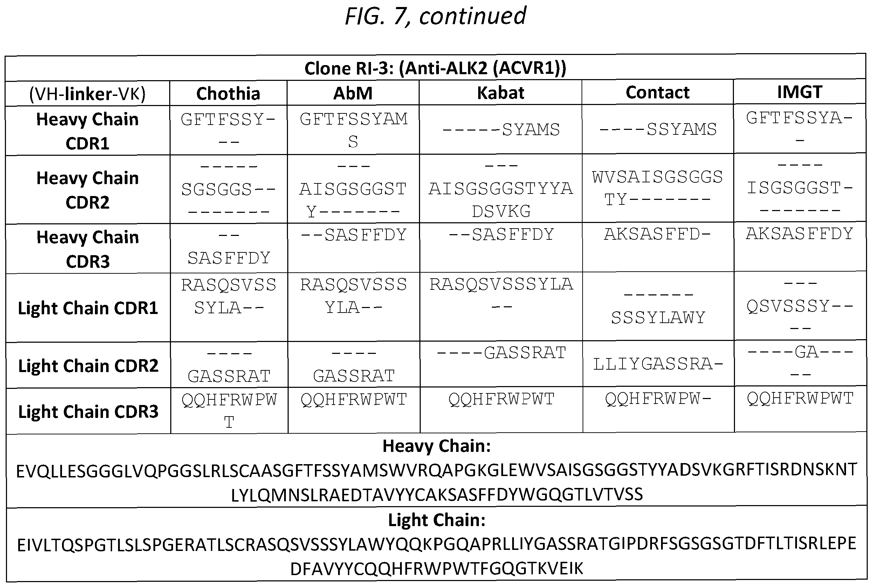

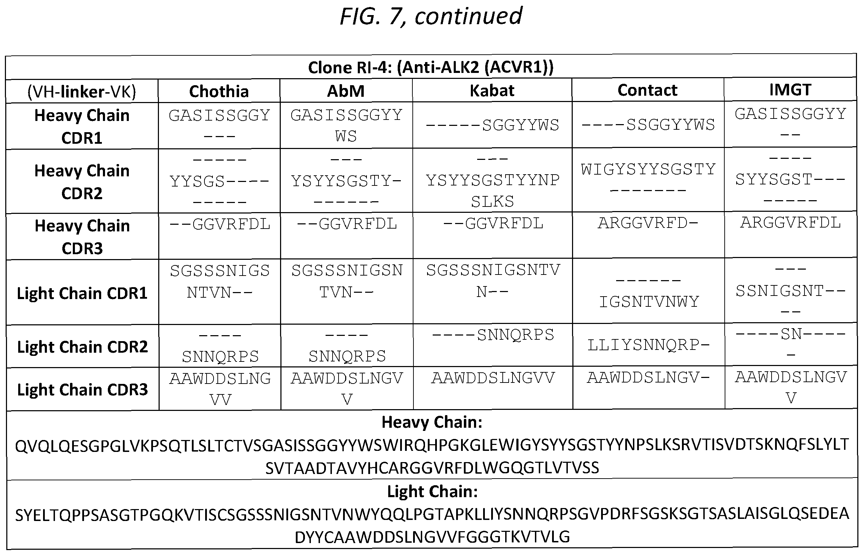

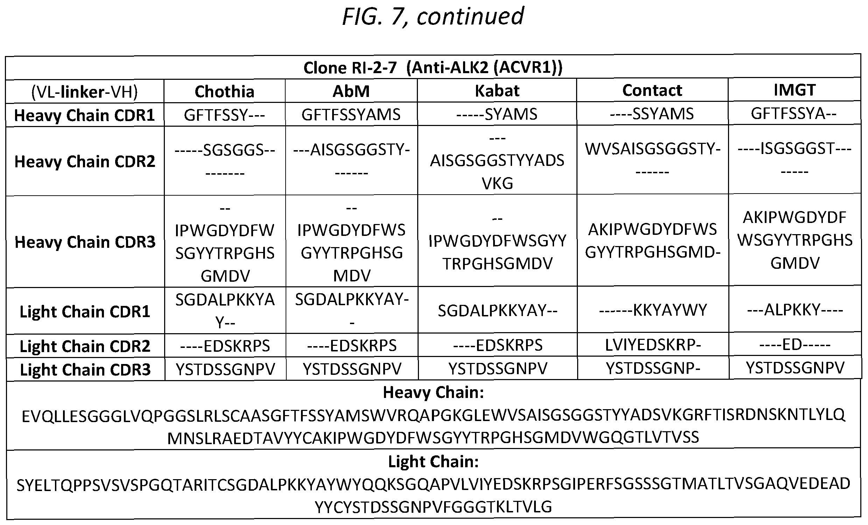

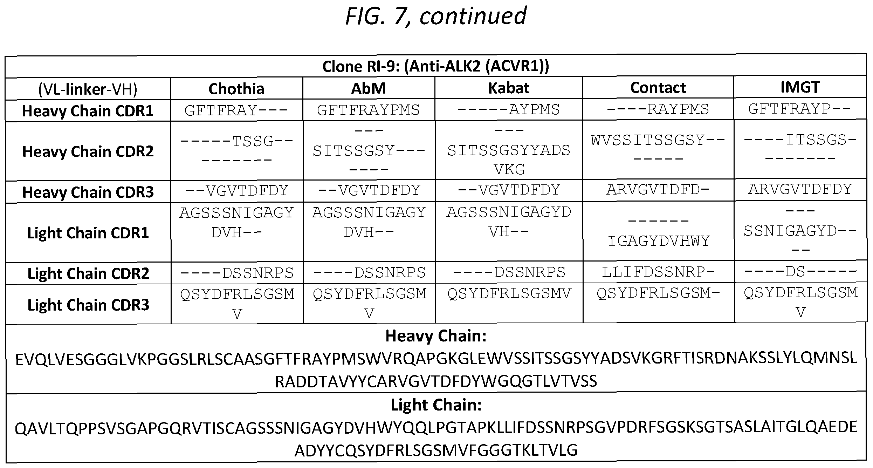

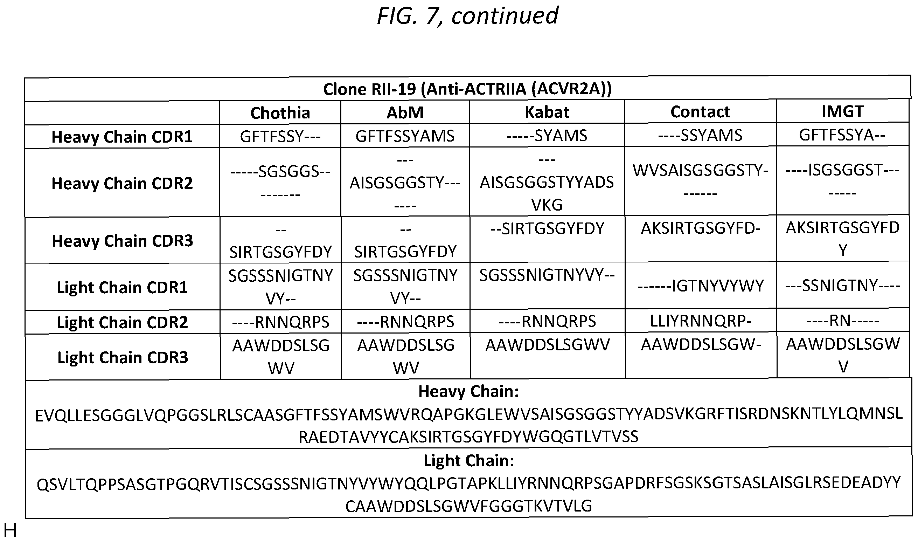

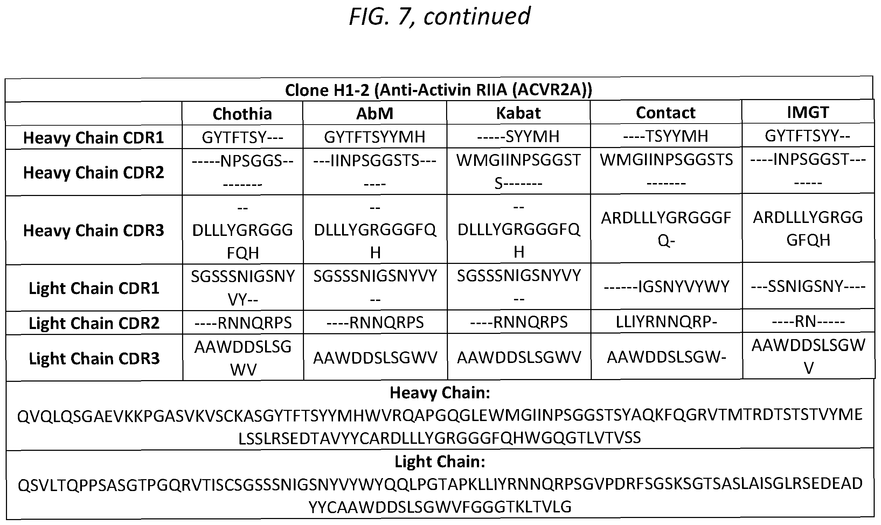

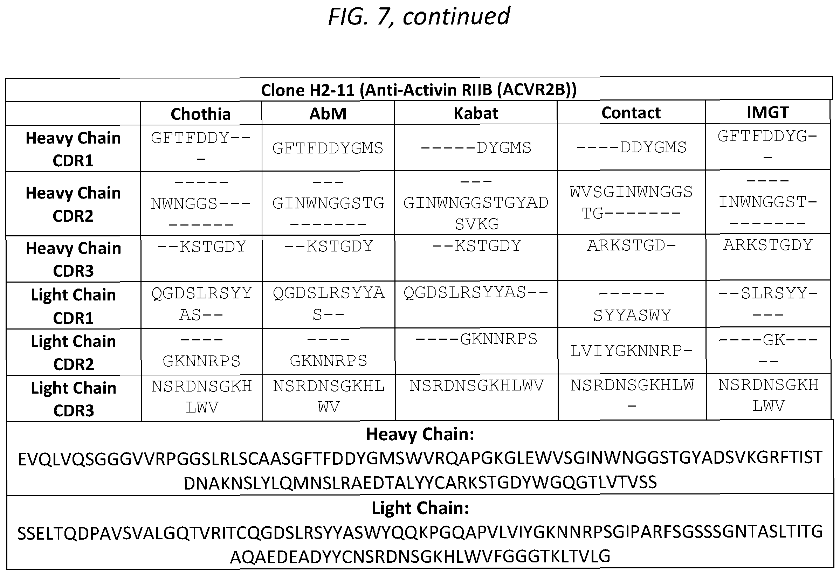

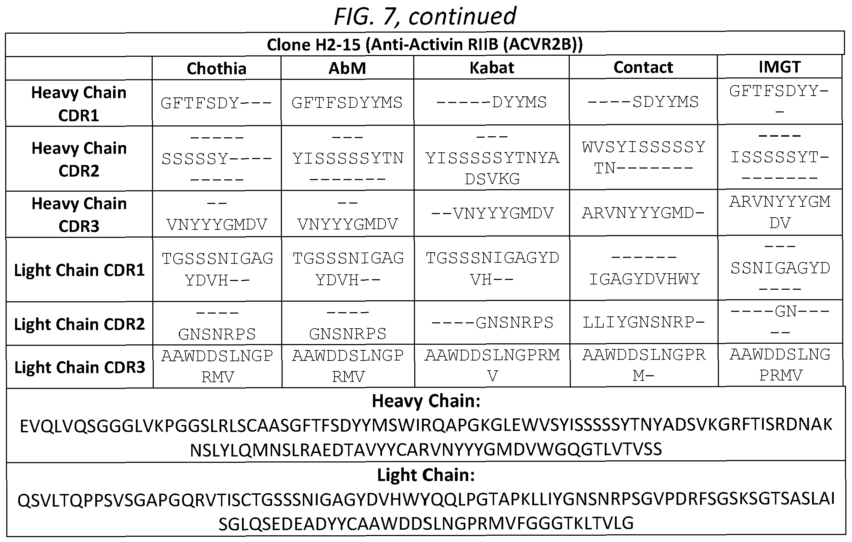

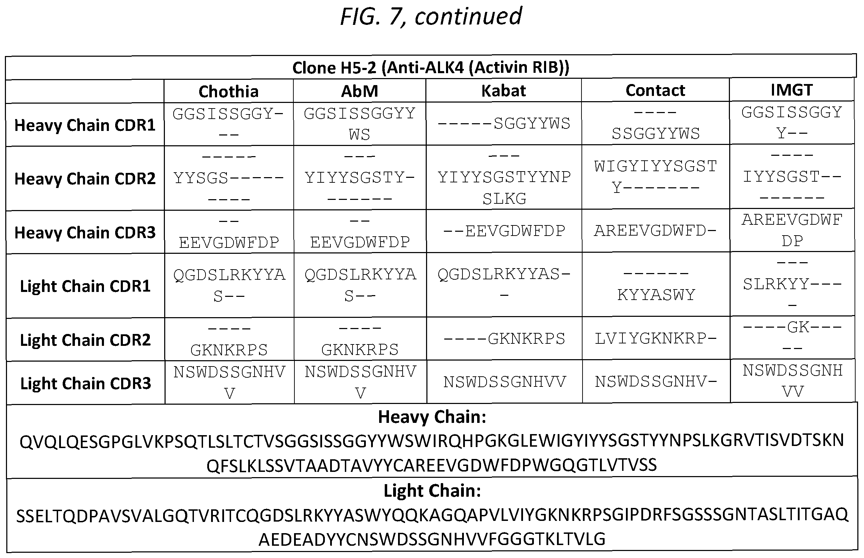

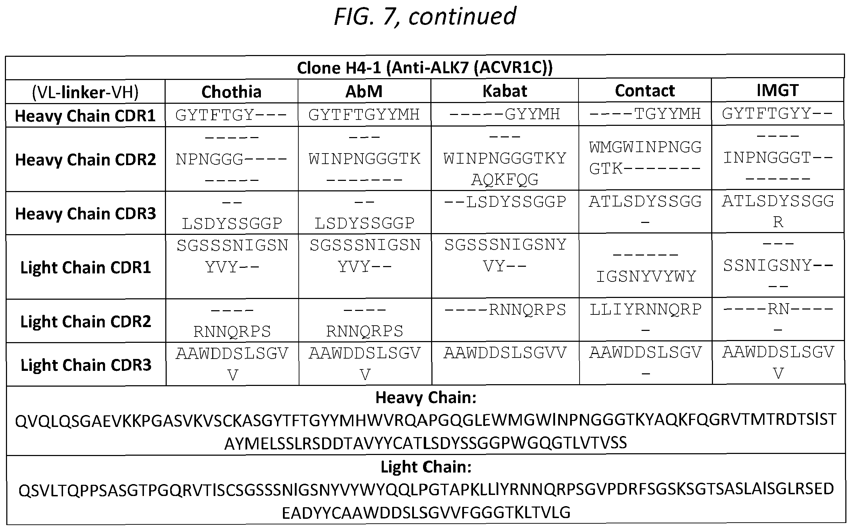

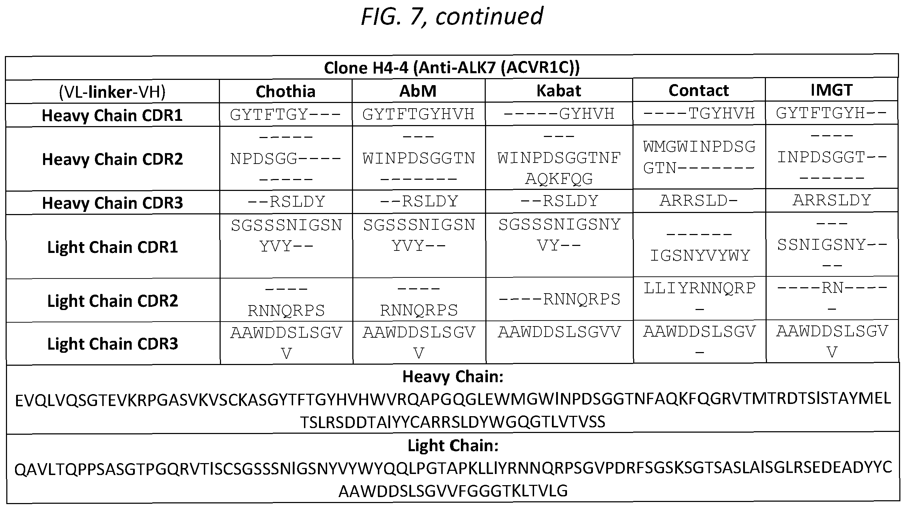

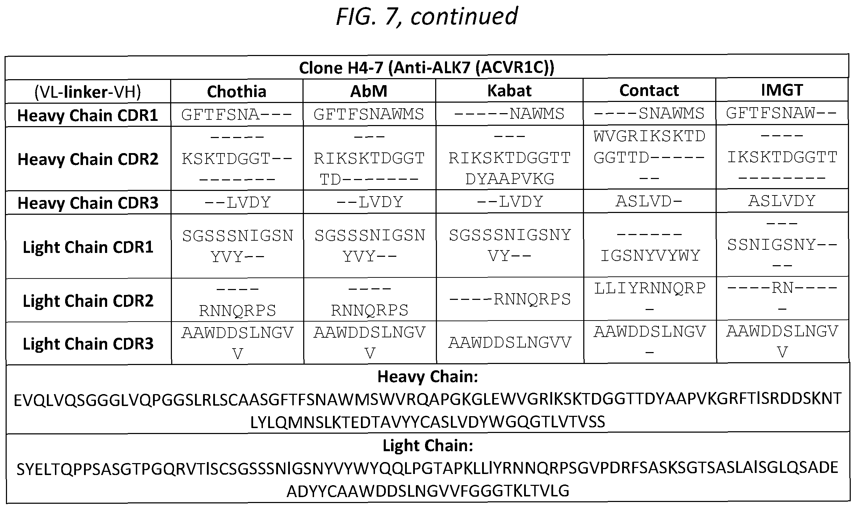

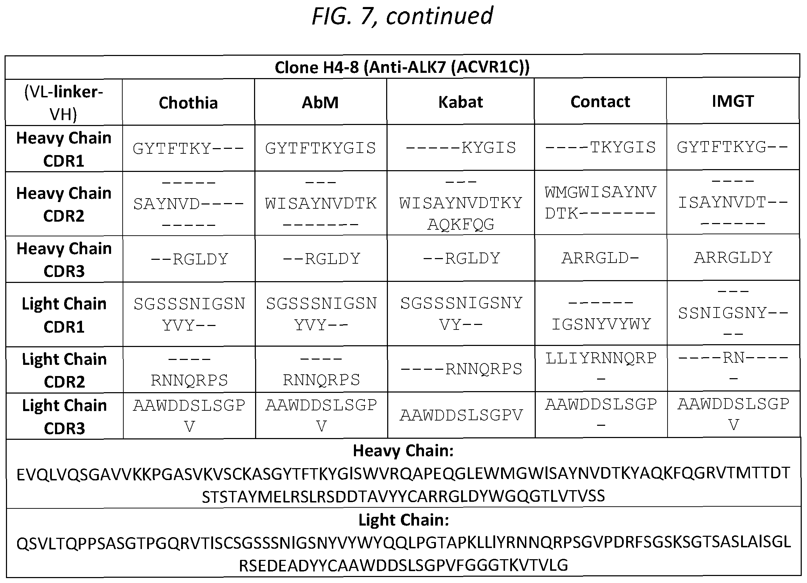

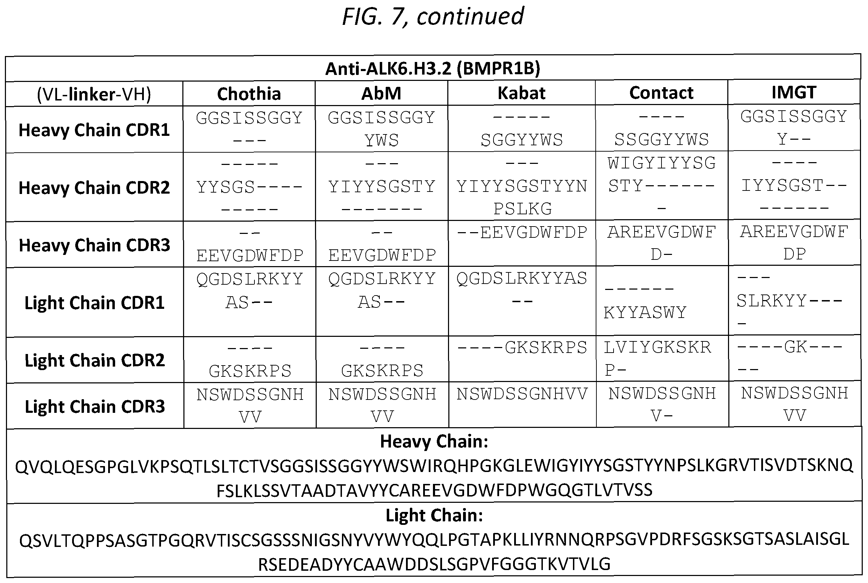

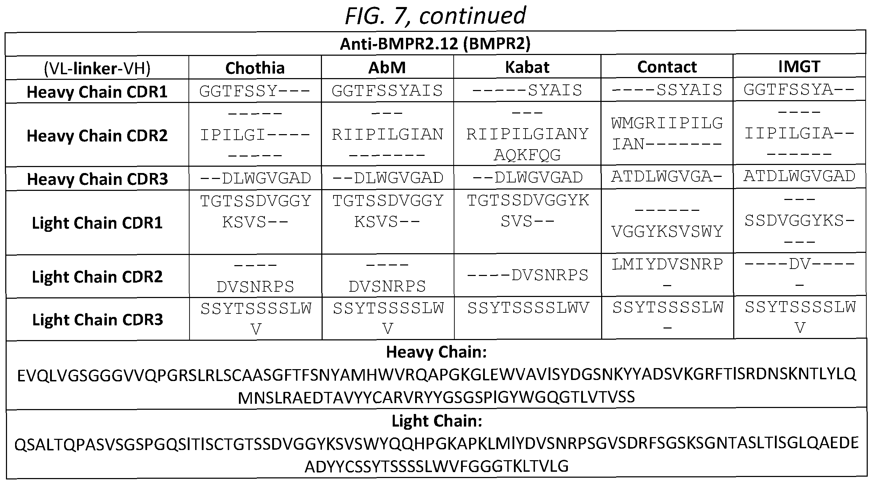

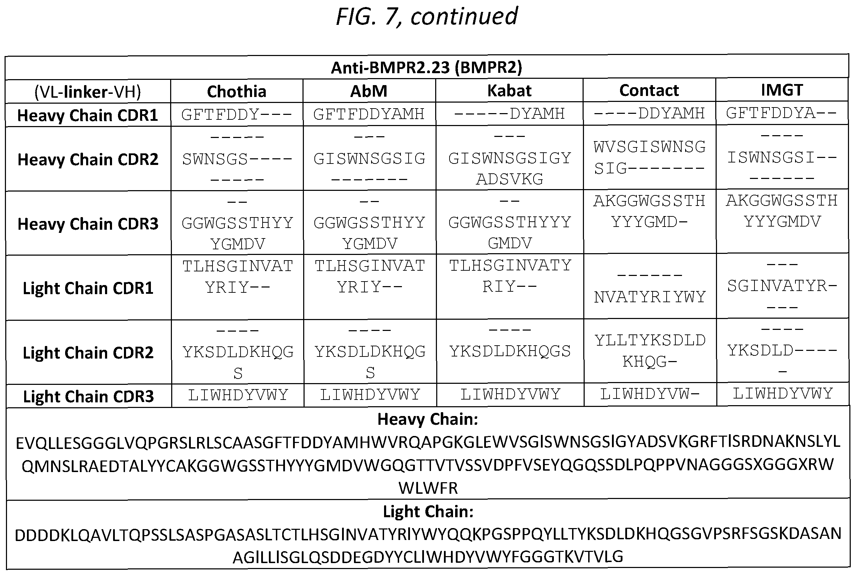

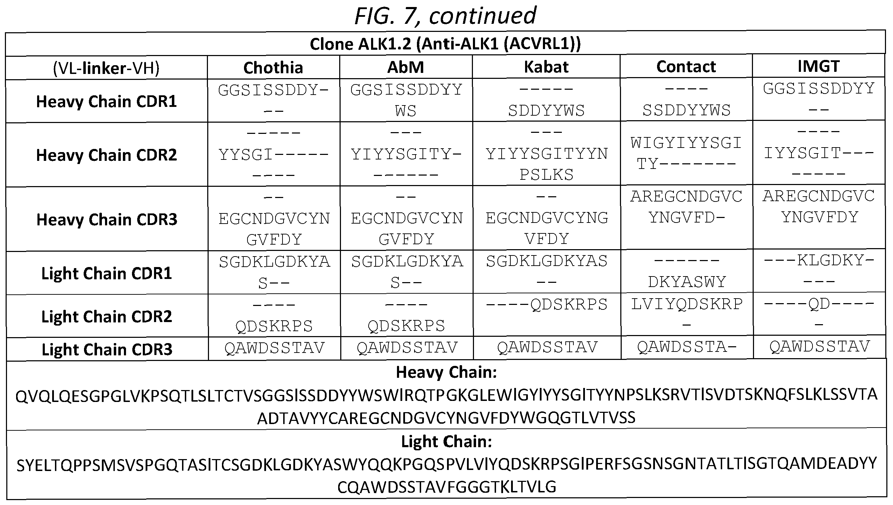

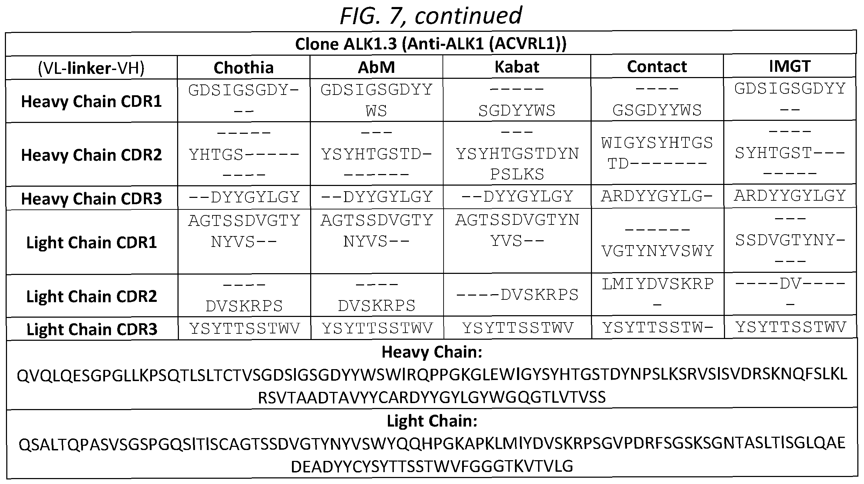

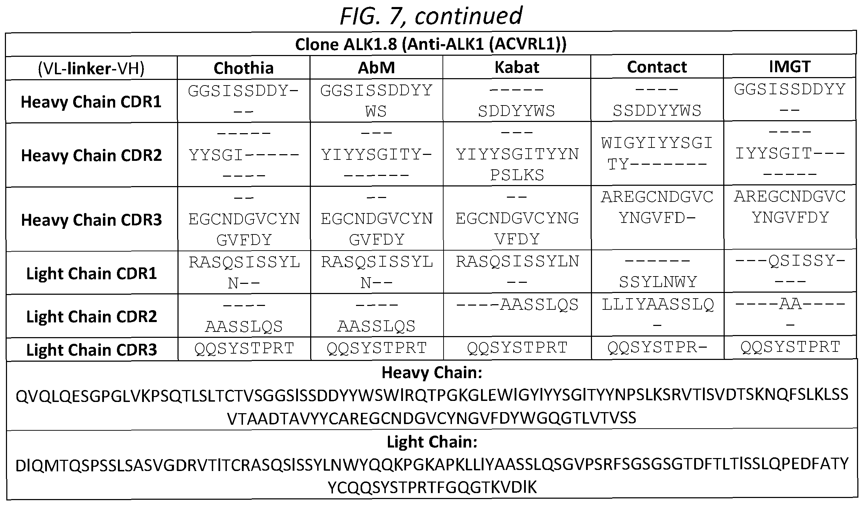

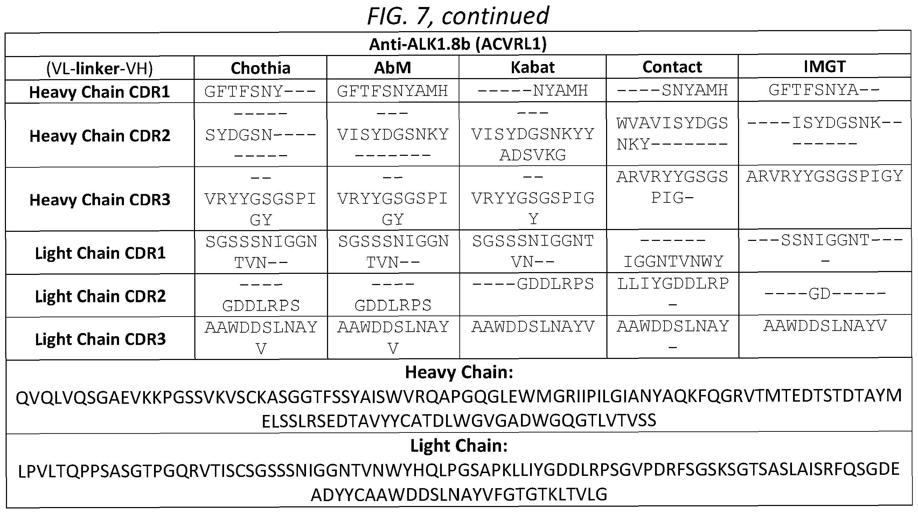

In some embodiments, the first antigen binding domain binds to ALK1 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 146, 148, 150, or 152; comprises VH and/or VL sequences at least 95% identical to sequences in Fig. 7; or comprises a sequence at least 95% identical to SEQ ID NO: 146, 148, 150, or 152.

In some embodiments, the first antigen binding domain binds to ALK2 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 160, 162, 164, 166, or 168; comprises VH and/or VL sequences at least 95% identical to sequences in Fig. 7; or comprises a sequence at least 95% identical to SEQ ID NO: 160, 162, 164, 166, or 168.

In some embodiments, the first antigen binding domain binds to ALK3 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 156 or 158; comprises VH and/or VL sequences at least 95% identical to sequences in Fig. 7; or comprises a sequence at least 95% identical to SEQ ID NO:156 or 158.

In some embodiments, the first antigen binding domain binds to ALK4 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 178; comprises VH and/or VL sequences at least 95% identical to sequences in Fig. 7; or comprises a sequence at least 95% identical to SEQ ID NO: 178.

In some embodiments, the first antigen binding domain binds to ALK6 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:180; comprises VH and/or VL sequences at least 95% identical to sequences in Fig. 7; or comprises a sequence at least 95% identical to SEQ ID NO: 180.

In some embodiments, the first antigen binding domain binds to ALK7 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 182, 184, 186, or 188; comprises VH and/or VL sequences at least 95% identical to sequences in Fig. 7; or comprises a sequence at least 95% identical to SEQ ID NOs: 182, 184, 186, or 188.

In some embodiments, the second antigen binding domain binds to a BMPRII selected from the group consisting of ACTRIIA (ACVR2A); ACTRIIB (ACVR2B); BMPRII (BMPR2); TGFBRII (TGFBR2); or AMHRII (AMHR2).

In some embodiments, the second antigen binding domain binds to BMPR2 and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:154 or 212; comprises VH and/or VL sequences at least 95% identical to sequences in Fig. 7; or comprises a sequence at least 95% identical to SEQ ID NO: 154 or 212.

In some embodiments, the second antigen binding domain binds to ACTRIIA (ACVR2A) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 170 and 172; comprises VH and/or VL sequences at least 95% identical to sequences in Fig. 7; or comprises a sequence at least 95% identical to SEQ ID NOs: 170 or 172.

In some embodiments, the second antigen binding domain binds to ACTRIIB (ACVR2B) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 174 or176; comprises VH and/or VL sequences at least 95% identical to sequences in Fig. 7; or comprises a sequence at least 95% identical to SEQ ID NOs: 174 or 176.

In some embodiments, the first and second antigen binding domains comprise any of the potential 5 x 7 = 35 combinations, e.g., BMPR2 and ALK1, ALK2, ALK3, ALK4, ALK5, ALK6, or ALK7; ACVR2A and ALK1, ALK2, ALK3, ALK4, ALK5, ALK6, or ALK7; ACVR2B and ALK1, ALK2, ALK3, ALK4, ALK5, ALK6, or ALK7; TGFBR2 and ALK1, ALK2, ALK3, ALK4, ALK5, ALK6, or ALK7; or AMHR2 and ALK1, ALK2, ALK3, ALK4, ALK5, ALK6, or ALK7.

In some embodiments:
(i) the first antigen binding domain binds to BMPR2 and comprises CDR sequences at least 95% identical to CDR sequences in Table 1 or Fig. 7, and/or the second antigen binding domain binds to ALK1, ALK2, ALK3, ALK4, ALK5, ALK6, and ALK7and comprises CDR sequences at least 95% identical to CDR sequences in Table 1 or Fig. 7;
(ii) the first antigen binding domain binds to ACVR2A and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7;
(iii) the first antigen binding domain binds to ACVR2B and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7;
(iv) the first antigen binding domain binds to TGFBR2 and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7;
(v) the first antigen binding domain binds to AMHR2 and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7.

Also provided herein are antibodies or antigen binding portions thereof that bind to ALK1 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 146, 148, 150, or 152; comprises VH and/or VL sequences at least 95% identical to sequences in Fig. 7; or comprises a sequence at least 95% identical to SEQ ID NOs: 146, 148, 150, or 152.

Also provided herein are antibodies or antigen binding portions thereof that bind to ALK2 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 160, 162, 164, 166, or 168; comprises VH and/or VL sequences at least 95% identical to sequences in Fig. 7; or comprises a sequence at least 95% identical to SEQ ID NOs: 160, 162, 164, 166, or 168.

Also provided herein are antibodies or antigen binding portions thereof that bind to ALK3 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 156 or 158; comprises VH and/or VL sequences at least 95% identical to sequences in Fig. 7; or comprises a sequence at least 95% identical to SEQ ID NO:156 or 158.

Also provided herein are antibodies or antigen binding portions thereof that bind to ALK4 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 178; comprises VH and/or VL sequences at least 95% identical to sequences in Fig. 7; or comprises a sequence at least 95% identical to SEQ ID NO: 178.

Also provided herein are antibodies or antigen binding portions thereof that bind to ALK6 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:180; comprises VH and/or VL sequences at least 95% identical to sequences in Fig. 7; or comprises a sequence at least 95% identical to SEQ ID NO: 180.

Also provided herein are antibodies or antigen binding portions thereof that bind to ALK7 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 182, 184, 186, or 188; comprises VH and/or VL sequences at least 95% identical to sequences in Fig. 7; or comprises a sequence at least 95% identical to SEQ ID NO: 182, 184, 186, or 188.

Also provided herein are antibodies or antigen binding portions thereof that bind to BMPR2 and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 154 or 212; comprises VH and/or VL sequences at least 95% identical to sequences in Fig. 7; or comprises a sequence at least 95% identical to SEQ ID NO: 154 or 212.

Also provided herein are antibodies or antigen binding portions thereof that bind to ACTRIIA (ACVR2A) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 170 or 172; comprises VH and/or VL sequences at least 95% identical to sequences in Fig. 7; or comprises a sequence at least 95% identical to SEQ ID NO: 170 and 172.

Also provided herein are antibodies or antigen binding portions thereof that bind to ACTRIIB (ACVR2B) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 174 or 176; comprises VH and/or VL sequences at least 95% identical to sequences in Fig. 7; or comprises a sequence at least 95% identical to SEQ ID NO: 174 or 176.

Additionally provided are pharmaceutical composition comprising the antibody molecules and antibodies or antigen binding portions thereof as described herein, optionally with a pharmaceutically acceptable carrier.

Also provided herein are methods for using the antibody molecules and antibodies or antigen binding portions thereof (or compositions) described herein for treating a vascular condition in a subject, optionally wherein the vascular condition is pulmonary arterial hypertension (PAH) or hereditary hemorrhagic telangiectasia (HHT) syndrome; for treating pulmonary hypertension (PH) more generally; for treating a pulmonary vascular leak syndrome, optionally wherein the pulmonary vascular leak syndrome is Acute Respiratory Distress Syndrome (ARDS) or Acute Lung Injury (ALI); and/or for treating liver fibrosis in a subject with deficient intrahepatic BMP9 signaling. In some embodiments, the first antigen binding domain binds to ALK1 and the second antigen binding domain binds to BMPR2. The methods can include administering a therapeutically effective amount of the antibody molecules described herein to a subject in need thereof.

Further, provided herein are nucleic acids encoding the antibody molecules described herein, host cells comprising the nucleic acids and optionally expressing the antibody molecules.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

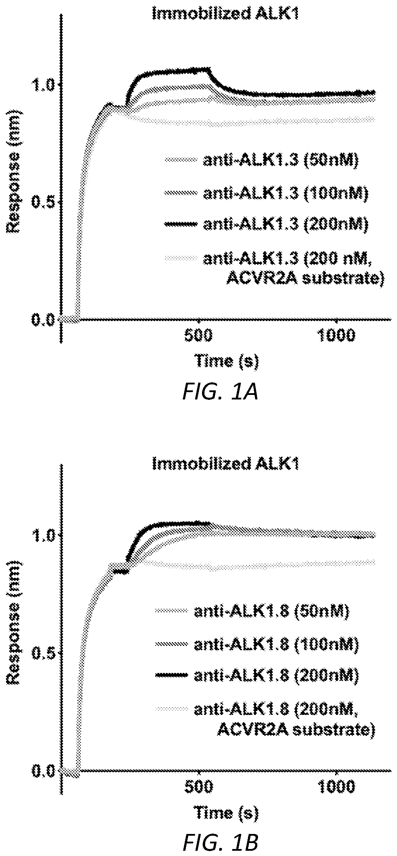

**FIGs. 1A-D****. Binding of scFv proteins to ALK1 and BMPR2.** (A-B) Octet Red Bio-Layer Interferometry (BLI) reveals binding of scFv clones ALK1.3 and ALK1.8 to immobilized ALK1. (C-D) BLI reveals binding of scFv clones BMPR2.12 and BMPR2.23 to immobilized BMPR2.

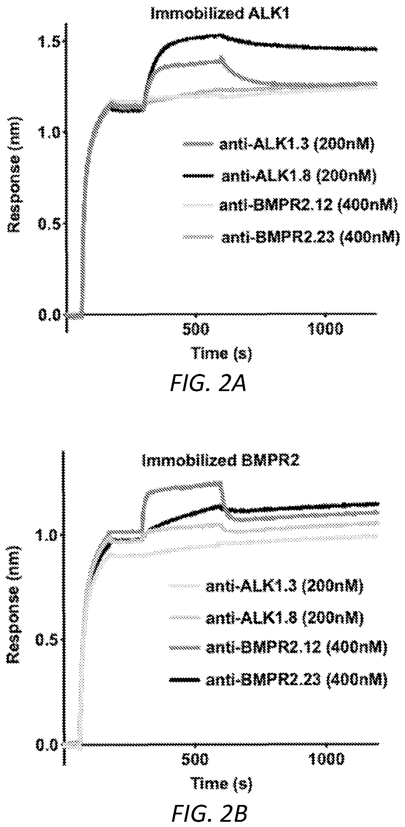

**FIGs. 2A-C****. Specific binding of scFv proteins to ALK1 and BMPR2.** (A) Octet Red Bio-Layer Interferometry (BLI) reveals binding of clones ALK1.3 and ALK1.8 to ALK1, but not clones BMPR2.12 or BMPR2.23. (B) Octet Red Bio-Layer Interferometry (BLI) reveals binding of clones BMPR2.12 and BMPR2.23 to BMPR2, but not clones ALK1.3 or ALK1.8; (C) None of clones ALK1.3, ALK1.8, BMPR2.12, or BMPR2.23, bind to immobilized ACVR2A.

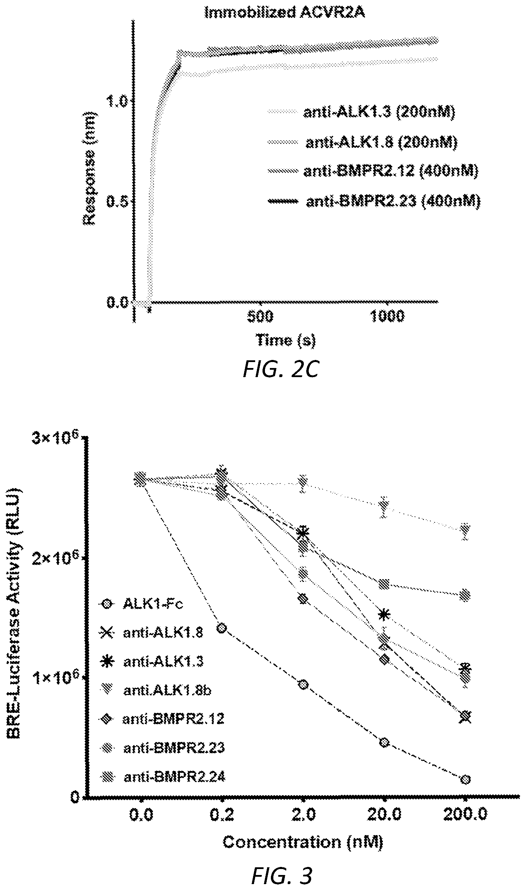

**FIG. 3****. scFv specific for ALK1 and BMPR2 inhibit BMP9 binding in endothelial cells.** Various scFvs exhibited dose-dependent inhibition of BMP9 (1 ng/mL for 18 h) mediated transcriptional activity, measured by BMP response element (BRE-luciferase) reporter activity in TIME (human telomerase immortalized microvascular endothelial cells), as compared with BMP9 ligand trap ALK1-Fc.

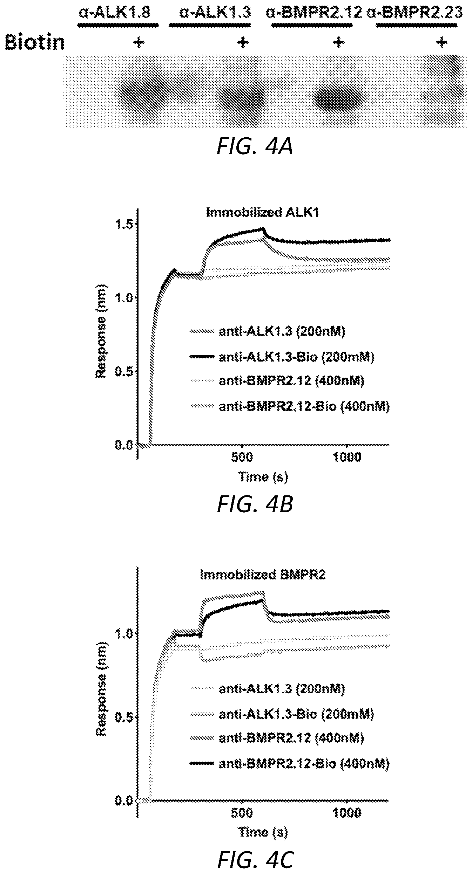

**FIGs. 4A-D****. Biotinylated scFv clones retain selective binding to ALK1 and BMPR2.** (A) Immunoblot of various scFv proteins with streptavidin-HRP confirms biotinylation of 25 Kd species. (B) Using BLI, both unmodified and biotinylated scFv ALK1.3 exhibit binding to immobilized ALK1, whereas unmodified and biotinylated scFv BMPR2.12 do not. (C) Using BLI, both unmodified and biotinylated scFv BMPR2.12 exhibit binding to immobilized BMPR2, whereas unmodified and biotinylated scFv ALK1.3 do not. (D) Using BLI, both unmodified and biotinylated scFv ALK1.8 exhibit binding to immobilized ALK1, whereas unmodified and biotinylated scFv BMPR2.23 do not.

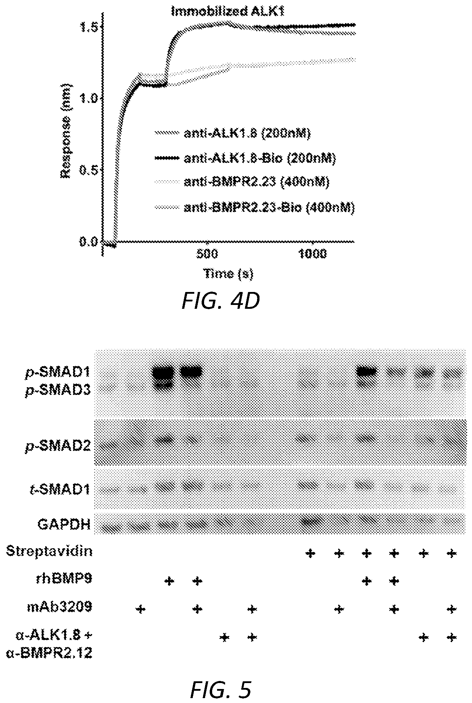

**FIG. 5****. Assembled streptavidin complexes of biotinylated scFV clones ALK1.8 and BMPR2.12 induce BMP signaling in microvascular endothelial cells.** Immunoblot reveals activation of SMADs 1 and 3, and to a lesser extent SMAD2, following stimulation of human pulmonary microvascular endothelial cells (PMVEC) with BMP9 (1 ng/mL, 30', 37C), with inhibition by co-treatment with monoclonal anti-BMP9 neutralizing antibody (mAb3209, 10 ng/mL). Treatment of cells with an equimolar mixture of biotinylated scFv clones ALK1.8 and BMPR2.12 (1 nm each, 30', 37C) induce activation of SMADs 1 and 3 in the presence but not the absence of streptavidin (1 µg/mL). The activation of SMAD1/3 by streptavidin-assembled biotinylated ALK1.8:BMPR2.12 complexes was not inhibited by co-treatment with anti-BMP9 neutralizing antibody, suggesting ligand-independent signaling activity.

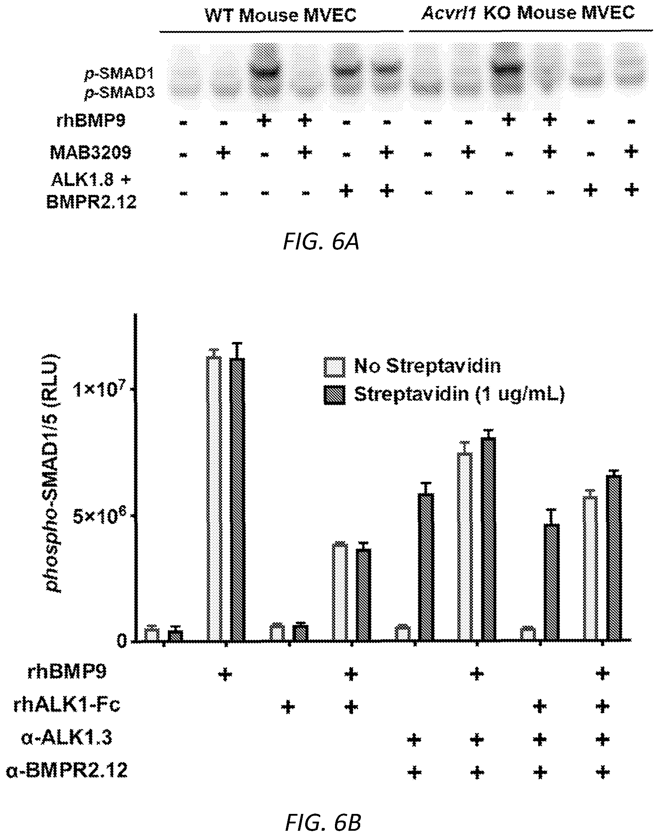

**FIGs. 6A-B****. Streptavidin-assembled complexes of biotinylated scFv clones ALK1.8 and BMPR2.12 induce SMAD1/3 signaling in pulmonary microvascular endothelial cells in a manner requiring ALK1 but not BMP9 ligand.** (A) Treatment of wild-type (WT), but not *Acvrl1-*/*-* (KO) cultured murine pulmonary microvascular endothelial cells (PMVEC) with streptavidin-assembled (1 µg/mL) biotinylated ALK1.8:BMPR2.12 clones (1 nM each) resulted in activation of SMAD1/3 (30', 37C) assayed by immunoblot, consistent with a requirement of ALK1 expression. Recombinant human BMP9 (rhBMP9, 1 ng/mL, 30', 37C) induced activation of SMAD1/3 in wild-type (WT) and *Acvrl1* KO cells, in a manner inhibited by mAb3209. In contrast, the activity of streptavidin-assembled biotinylated ALK1.8:BMPR2.12 complexes was not inhibited by mAb3209 (10 ng/mL), again consistent with ligand-independent signaling. (B) Quantitative measurement of phosphorylated SMAD1/5 using an In-Cell Western assay in cultured human pulmonary microvascular endothelial cells (PMVEC) revealed activation of SMAD1/5 by recombinant human BMP9 (rhBMP9), and streptavidin-assembled (1 µg/mL) biotinylated ALK1.8:BMPR2.12 (1 nM each) complexes. Treatment of cells with BMP9/BMP10 ligand trap ALK1-Fc inhibited BMP9-mediated but not streptavidin-assembled ALK1.8/BMPR2.12 complex-mediated SMAD1/5 activation.
**FIG. 7****. CDR Sequences.** Shown are heavy and light chain sequences for each of the exemplary antibodies described herein, with CDR sequences identified using different definitions as described herein.

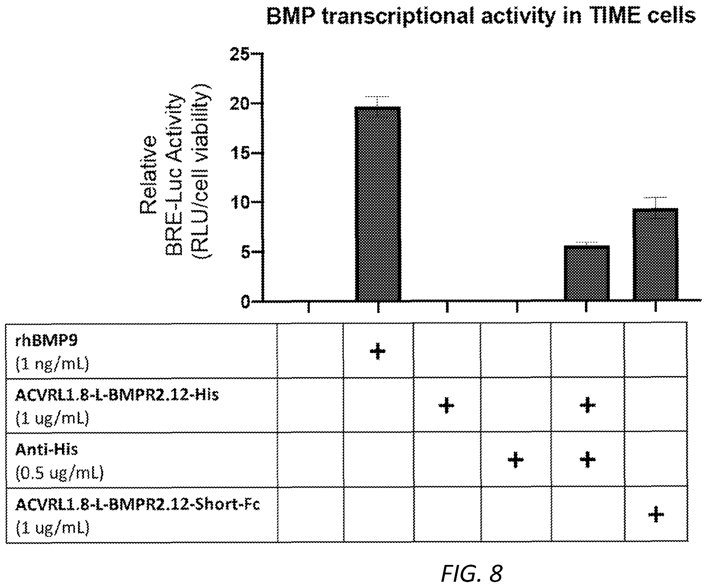

**FIG. 8****. BMP transcriptional activity in TIME cells.** *In vitro* activity assay data for the indicated bispecific and tetraspecific constructs. The His-tagged bispecifics did not generate activity unless they were cross linked to form a tetramer. The IgG Fc fusion bispecifics, when expressed as a disulfide linked homodimer to form a tetramer of antigen binding domains, elicited signaling just as they were.

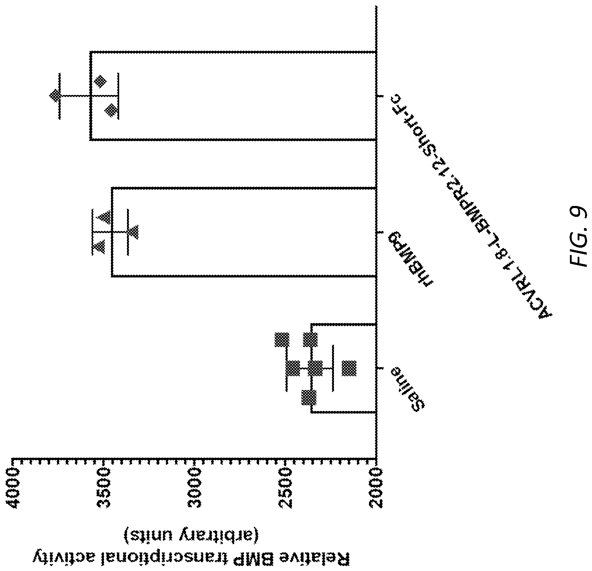

**FIG. 9****. Intravenous recombinant human BMP9 and tetravalent Fc Fusion molecules elicit BMP transcriptional activity in vivo in mice.** BMP signaling activity based on Id1 gene transcriptional activity was assayed in whole lung tissues from 8-week old mice 24 hours after tail vein injection of saline (50 uL), recombinant human BMP9 (rhBMP9, 150 ug/kg in 50 uL), or recombinant tetrameric Fc fusion protein ACVRL1. 8-L-BMPR2.12-Short-Fc (150 ug/kg in 50 uL). The injection of BMP9 or ACVRL1.8-L-BMPR2.12-Short-Fc elicited an increase in BMP transcriptional activity as compared to saline in whole lung tissues.
**FIGs. 10A-F****. Schematic illustration of exemplary constructs.** (A) Tetravalent or multivalent signaling molecules can include an anti-type I BMP receptor antigen binding region and an anti-type II BMP receptor antibody binding region separated by a flexible linker regions, and expressed as fusion molecules with the immunoglobulin G constant domain (IgG Fc). The antigen binding regions can be alternating, and form a functional heterotetramer by the homodimeric combination of two identical IgG Fc fusion molecules via disulfide bonds. Alternately, the heterotetramer can include the heterodimeric combination of two dissimilar IgG Fc fusion molecules (B), each expressing unique combinations of type I and type II antigen binding regions. Larger multivalent molecules can include more than two antigen binding regions, occurring in various recurring or non-recurring sequences, and in different numbers, likely but not necessarily with an even number of antibody binding regions (C). These molecules can include the homodimeric combination of identical IgG Fc fusion molecules, or can include the heterodimeric combination of two dissimilar IgG Fc fusion molecules (D).

### DETAILED DESCRIPTION

The BMP/TGFβ signaling pathway, which also includes ligands of the activin and growth and differentiation factor (GDF) family, serves as a critical regulator of organogenesis, embryonic pattern formation, and postnatal tissue remodeling and regeneration. Endogenous ligands of the BMP/TGFβ/activin/GDF signaling pathway exhibit promiscuity, frequently binding to several type II and type I receptors and forming diverse ligand-receptor signaling complexes with distinct functional consequences. These ligands are frequently sequestered in extracellular matrix by virtue of their heparin binding domains, and thus exhibit poor pharmacokinetics as therapeutic molecules. Simplistic gain- and loss-of-function studies targeting individual type I receptors have confirmed general roles of individual ligands and receptors in chondrogenic differentiation, osteogenic differentiation and bone mass regulation, adipogenic differentiation, fibrosis, and tenogenic differentiation,^{4,5} but with contradictory results and a lack of clear data on which signaling complexes are engaged in which tissues to generate these outcomes. The combinatorial diversity of ligand and receptor pairings in this family has confounded efforts to comprehensively map BMP/TGFβ family signaling inputs to cellular functions.

Described herein are engineered bispecific antibodies recognizing the extracellular domains of surface BMP/TGF-b/activin/GDF receptors to promote their heterodimerization and initiate signaling. In some embodiments, such antibodies can be expressed with or without C-terminal IgG Fc domains in order to have more limited or more extended half-life in circulation, and can be delivered through the circulation to target tissues, or be bound to homing peptides or other homing molecules to target specific tissues, or adsorbed or bound to solid substrates to encourage engraftment or growth of specific tissues.

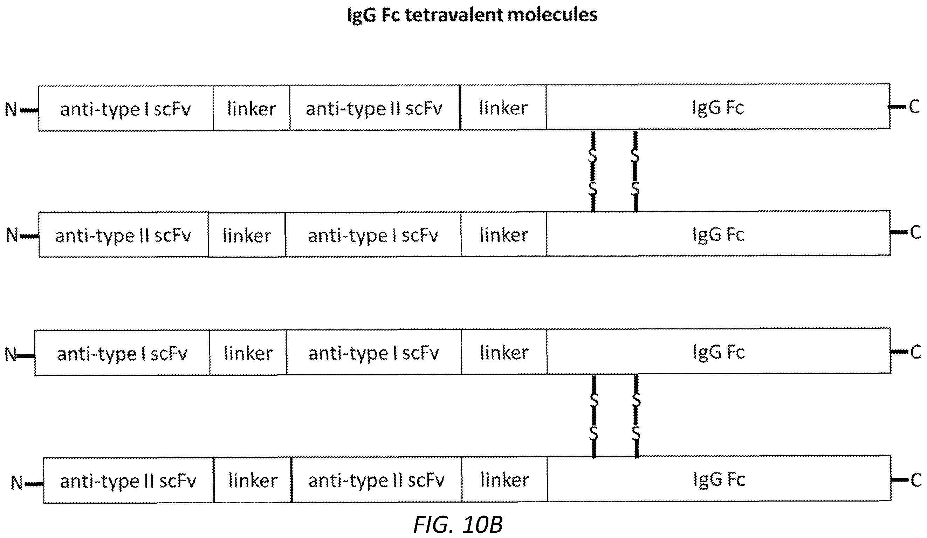

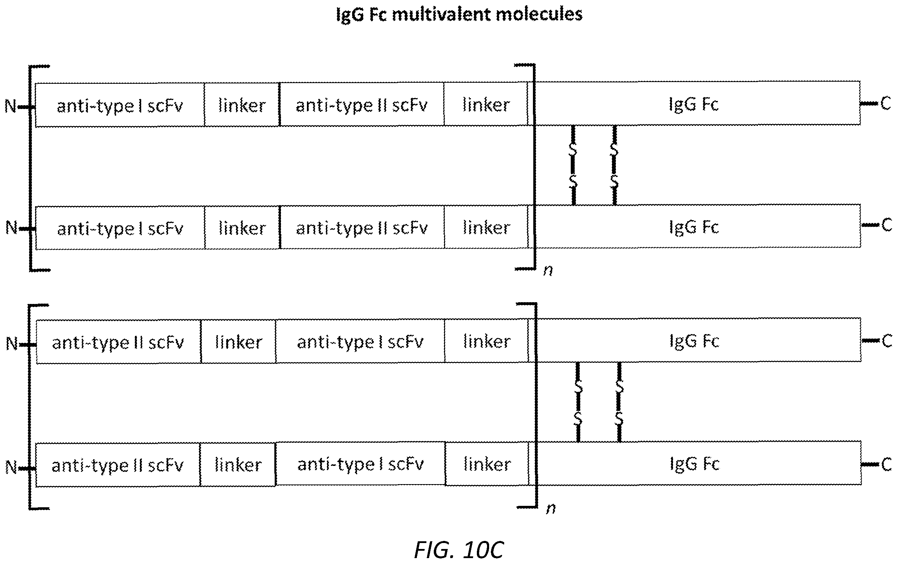

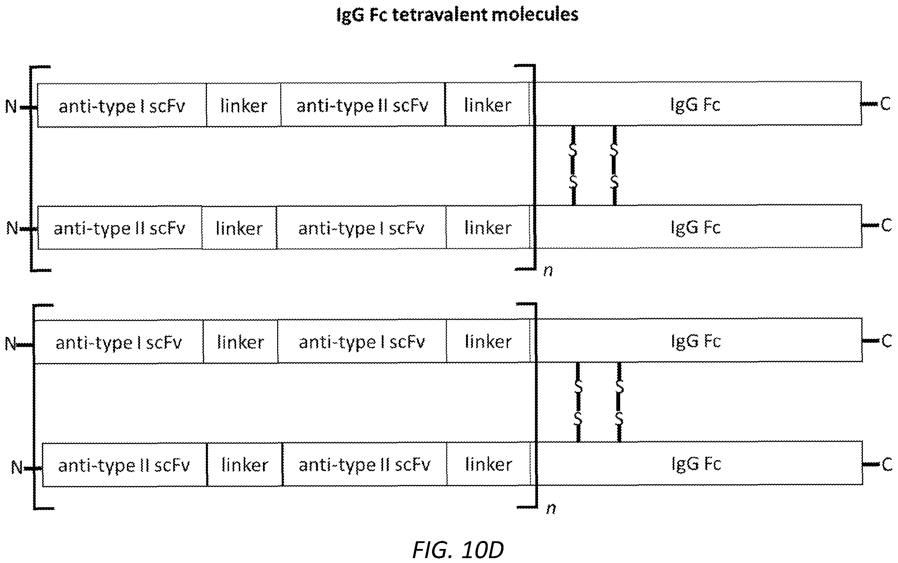

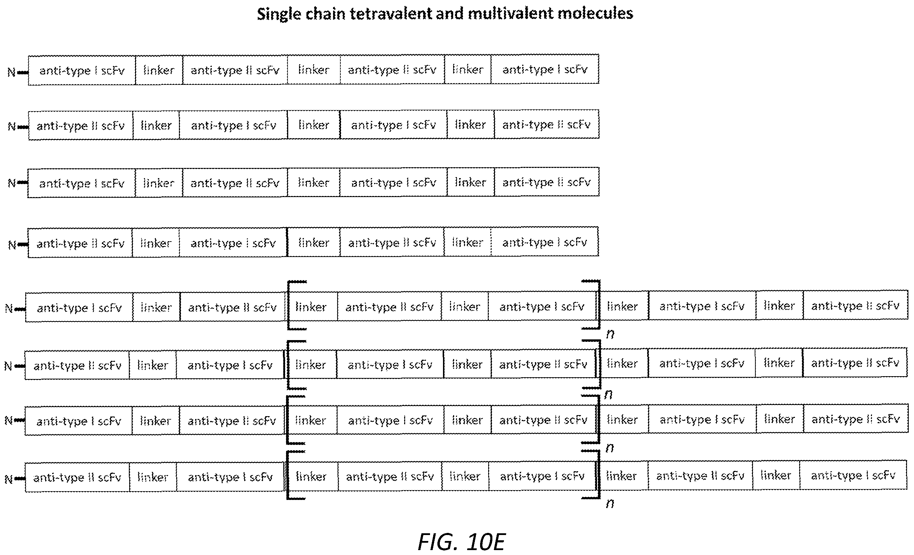

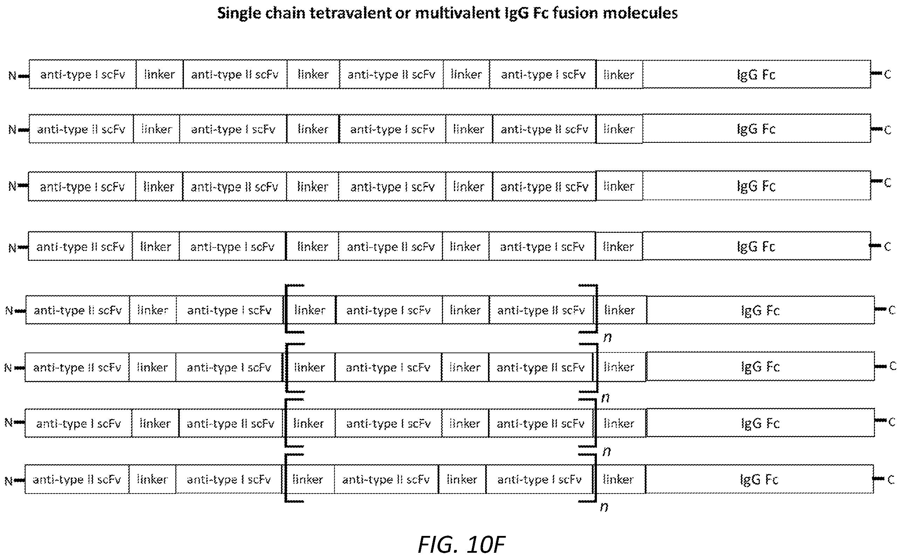

Also described are tetravalent or multivalent signaling molecules that can include an anti-type I BMP receptor antigen binding region and an anti-type II BMP receptor antibody binding region separated by a flexible linker regions, and expressed as fusion molecules with the immunoglobulin G constant domain (IgG Fc, wherein the Fc domain can be from either wild-type IgG1, IgG2, IgG3, or IgG4 sequences, or from sequences modified to alter binding to Fcy-receptor, to alter complement fixation, to alter antibody-dependent cellular cytotoxicity, to alter dimerization, or other functional properties). Methods for engineering Fc domains are known in the art, see, e.g., Yang et al., Front Immunol. 2018 Jan 8;8:1860. The antigen binding regions can be alternating and form a functional heterotetramer by the homodimeric combination of two identical IgG Fc fusion molecules via disulfide bonds (FIG. 10A). Alternately, the heterotetramer can include a heterodimeric combination of two dissimilar IgG Fc fusion molecules (FIG. 10B), each expressing unique combinations of type I and type II antigen binding regions. Larger multivalent molecules can include more than two antigen binding regions, occurring in various recurring or non-recurring sequences, and in different numbers, likely but not necessarily with an even number of antibody binding regions (FIG. 10C). These molecules can include the homodimeric combination of identical IgG Fc fusion molecules, or can include the heterodimeric combination of two dissimilar IgG Fc fusion molecules (FIG. 10D). Single chain multimeric signaling molecules can include anti-type I and anti-type II antigen binding regions, in alternating, successive, or palindromic configurations, and in recurring or non-recurring sequence, as bivalent or multivalent but more typically tetravalent molecules (FIG. 10E). Multimeric single chain signaling molecules can include more than 2 or 4 signaling molecules, in either alternating, successive, pandromic, or non-recurring sequence. These molecules will likely but not necessarily include an even number of antibody binding regions. These single chain multimeric signaling molecules can also be expressed as monomeric IgG Fc fusion proteins (FIG. 10F).

Thus thee molecules described herein can include an Fc region. IgG Fc CH3 is required for homodimerization, but it can be modified to prevent this for specific clinical applications. Methods for engineering Fc domains are known in the art, see, e.g., Yang et al., Front Immunol. 2018 Jan 8;8:1860. Alternatively or in addition, the molecules herein can include other domains to facilitate their targeting to particular cells, tissues, or functional contexts, such as causing the molecule to be retained in tissues expressing changes due to the presence of inflammation, proliferation or neoplastic changes, or other disease-associated changes in protein expression, pH, extracellular fluid composition with respect to any solutes or ion, extracellular matrix expression, glycosylation, or glycocalyx structure or composition.

Thus the tetramers can be a single molecule without an Fc region, e.g., with a short half-life if that is preferred. Homodimeric Fc fusions are also described, where each Fc chain has a bispecific pair of scFvs. An Fc region can also be appended to any tetramer, 6mer, 8 mer, etc. Also descried are disulfide linked tetramers with Fc.

Also described herein are methods of selective agonism, signaling activation, or signaling modulation of specific members of the bone morphogenetic protein (BMP), activin, growth and differentiation factor (GDF), and transforming growth factor (TGF)-beta signaling superfamily using engineered bispecific or multi-specific antibodies that recognize receptors of this pathway.

### I. Definitions

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

As used herein, the term "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids and does not refer to a specific length of the product. Thus, peptides, dipeptides, tripeptides, oligopeptides, "protein," "amino acid chain," or any other term used to refer to a chain or chains of two or more amino acids, are included within the definition of "polypeptide," and the term "polypeptide" may be used instead of, or interchangeably with any of these terms.

The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis.

A polypeptide described herein may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded. As used herein, the term glycoprotein refers to a protein coupled to at least one carbohydrate moiety that is attached to the protein via an oxygen-containing or a nitrogen-containing side chain of an amino acid residue, e.g., a serine residue or an asparagine residue.

By an "isolated" polypeptide or a fragment, variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for purposes described herein, as are native or recombinant polypeptides which have been separated, fractionated, or partially or substantially purified by any suitable technique.

Also included as polypeptides are fragments, derivatives, analogs or variants of the foregoing polypeptides, and any combination thereof. The terms "fragment," "variant," "derivative" and "analog" when referring to antibodies or antibody polypeptides described herein include any polypeptides which retain at least some of the antigen-binding properties of the corresponding native binding molecule, antibody, or polypeptide. Fragments of polypeptides described herein include proteolytic fragments, as well as deletion fragments, in addition to specific antibody fragments discussed elsewhere herein. Variants of antibodies and antibody polypeptides described herein include fragments as described above, and also polypeptides with altered amino acid sequences due to amino acid substitutions (e.g., conservative or non-conservative amino acid substitutions), deletions, or insertions. Variants may occur naturally or be non-naturally occurring. Non-naturally occurring variants may be produced using art-known mutagenesis techniques. Variant polypeptides may comprise conservative or non-conservative amino acid substitutions (e.g., conservative or non-conservative amino acid substitutions), deletions or additions. Derivatives of BMPRI/BMPRII specific binding molecules, e.g., antibodies and antibody polypeptides described herein, are polypeptides which have been altered so as to exhibit additional features not found on the native polypeptide. Examples include fusion proteins. Variant polypeptides may also be referred to herein as "polypeptide analogs". As used herein a "derivative" of a binding molecule or fragment thereof, an antibody, or an antibody polypeptide refers to a subject polypeptide having one or more residues chemically derivatized by reaction of a functional side group. Also included as "derivatives" are those peptides which contain one or more naturally occurring amino acid derivatives of the twenty standard amino acids. For example, 4-hydroxyproline may be substituted for proline; 5-hydroxylysine may be substituted for lysine; 3-methylhistidine may be substituted for histidine; homoserine may be substituted for serine; and ornithine may be substituted for lysine.

The term "polynucleotide" is intended to encompass a singular nucleic acid as well as plural nucleic acids, and refers to an isolated nucleic acid molecule or construct, e.g., messenger RNA (mRNA) or plasmid DNA (pDNA). A polynucleotide may comprise a conventional phosphodiester bond or a non-conventional bond (e.g., an amide bond, such as found in peptide nucleic acids (PNA)). The term "nucleic acid" refers to any one or more nucleic acid segments, e.g., DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding an antibody contained in a vector is considered isolated for the purposes described herein. Further examples of an isolated polynucleotide include recombinant polynucleotides maintained in heterologous host cells or purified (partially or substantially) polynucleotides in solution. Isolated RNA molecules include in vivo or in vitro RNA transcripts of polynucleotides described herein. Isolated polynucleotides or nucleic acids described herein further include such molecules produced synthetically. In addition, polynucleotide or a nucleic acid may be or may include a regulatory element such as a promoter, ribosome binding site, or a transcription terminator.

As used herein, a "coding region" is a portion of a nucleic acid that consists of codons translated into amino acids. Although a "stop codon" (TAG, TGA, or TAA) is not translated into an amino acid, it may be considered to be part of a coding region, but any flanking sequences, for example promoters, ribosome binding sites, transcriptional terminators, introns, and the like, are not part of a coding region. Two or more coding regions described herein can be present in a single polynucleotide construct, e.g., on a single vector, or in separate polynucleotide constructs, e.g., on separate (different) vectors. Furthermore, any vector may contain a single coding region, or may comprise two or more coding regions, e.g., a single vector may separately encode an immunoglobulin heavy chain variable region and an immunoglobulin light chain variable region. In addition, a vector, polynucleotide, or nucleic acid described herein may encode heterologous coding regions, either fused or unfused to a nucleic acid encoding a binding molecule, an antibody, or fragment, variant, or derivative thereof. Heterologous coding regions include without limitation specialized elements or motifs, such as a secretory signal peptide or a heterologous functional domain.

In some embodiments, the polynucleotide or nucleic acid is DNA. In the case of DNA, a polynucleotide comprising a nucleic acid which encodes a polypeptide normally may include a promoter and/or other transcription or translation control elements operably associated with one or more coding regions. An operable association is when a coding region for a gene product, e.g., a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" or "operably linked" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein.

A variety of transcription control regions are known to those skilled in the art. These include, without limitation, transcription control regions which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as lymphokine-inducible promoters (e.g., promoters inducible by interferons or interleukins).

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

In some embodiments, a polynucleotide described herein is RNA, for example, in the form of messenger RNA (mRNA).

Polynucleotide and nucleic acid coding regions described herein may be associated with additional coding regions that encode secretory or signal peptides, which direct the secretion of a polypeptide encoded by a polynucleotide described herein. According to the signal hypothesis, proteins secreted by mammalian cells have a signal peptide or secretory leader sequence that is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the complete or "full-length" polypeptide to produce a secreted or "mature" form of the polypeptide. In some embodiments, the native signal peptide, e.g., an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence can be substituted with the leader sequence of human tissue plasminogen activator (TPA) or mouse β-glucuronidase.

Unless stated otherwise, the terms "disorder" and "disease" are used interchangeably herein.

A "binding molecule" as used herein relates primarily to antibodies, and fragments thereof, but may also refer to other non-antibody molecules that bind to BMPRI/BMPRII including but not limited to hormones, receptors, ligands, major histocompatibility complex (MHC) molecules, chaperones such as heat shock proteins (HSPs) as well as cell-cell adhesion molecules such as members of the cadherin, intergrin, C-type lectin and immunoglobulin (Ig) superfamilies. Thus, for the sake of clarity only and without restricting the scope of the disclosure most of the following embodiments are discussed with respect to antibodies and antibody-like molecules which represent a specific embodiment of binding molecules for the development of therapeutic and diagnostic agents.

The terms "antibody" and "immunoglobulin" are used interchangeably herein. An antibody or immunoglobulin is a BMPRI/BMPRII-binding molecule which comprises at least the variable domain of a heavy chain, and normally comprises at least the variable domains of a heavy chain and a light chain. Basic immunoglobulin structures in vertebrate systems are relatively well understood; see, e.g., Harlow et al., Antibodies: A Laboratory Manual, (Cold Spring Harbor Laboratory Press, 2nd ed. 1988). As used herein, the term "antibody" refers to any antigen-binding molecule that contains at least one (e.g., one, two, three, four, five, or six) complementary determining region (CDR) (e.g., any of the three CDRs from an immunoglobulin light chain or any of the three CDRs from an immunoglobulin heavy chain) and is capable of specifically binding to an epitope. Non-limiting examples of antibodies include: monoclonal antibodies, polyclonal antibodies, multi-specific antibodies (e.g., bi-specific antibodies), single-chain antibodies, chimeric antibodies, human antibodies, and humanized antibodies. In some embodiments, an antibody can contain an Fc region of a human antibody. The term antibody also includes derivatives, e.g., bi-specific antibodies, single-chain antibodies, diabodies, linear antibodies, and multi-specific antibodies formed from antibody fragments.

As will be discussed in more detail below, the term "immunoglobulin" comprises various broad classes of polypeptides that can be distinguished biochemically. Those skilled in the art will appreciate that heavy chains are classified as gamma, mu, alpha, delta, or epsilon, (γ, µ, α, δ, ε) with some subclasses among them (e.g., γ1-γ4). It is the nature of this chain that determines the "class" of the antibody as IgG, IgM, IgA IgG, or IgE, respectively. The immunoglobulin subclasses (isotypes) e.g., IgG1, IgG2, IgG3, IgG4, IgA1, etc. are well characterized and are known to confer functional specialization. Modified versions of each of these classes and isotypes are readily discernable to the skilled artisan in view of the instant disclosure and, accordingly, are within the scope of the disclosure. All immunoglobulin classes are clearly within the scope of the disclosure, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain.

Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention, the numbering of the constant region domains increases as they become more distal from the antigen-binding site or amino-terminus of the antibody. The N-terminal portion is a variable region and at the C-terminal portion is a constant region; the CH3 and CL domains actually comprise the carboxy-terminus of the heavy and light chain, respectively.

As indicated above, the variable region allows the antibody to selectively recognize and specifically bind epitopes on antigens. That is, the VL domain and VH domain, or subset of the complementarity determining regions (CDRs), of an antibody combine to form the variable region that defines a three-dimensional antigen-binding site. This quaternary antibody structure forms the antigen-binding site present at the end of each arm of the Y. More specifically, the antigen-binding site is defined by three CDRs on each of the VH and VL chains. Any antibody or immunoglobulin fragment that contains sufficient structure to specifically bind to BMPRI/BMPRII is denoted herein interchangeably as a "binding fragment" or an "immunospecific fragment."

In naturally occurring antibodies, an antibody comprises six hypervariable regions, sometimes called "complementarity determining regions" or "CDRs" present in each antigen-binding domain, which are short, non-contiguous sequences of amino acids that are specifically positioned to form the antigen-binding domain as the antibody assumes its three dimensional configuration in an aqueous environment. The "CDRs" are flanked by four relatively conserved "framework" regions or "FRs" which show less inter-molecular variability. The framework regions largely adopt a β-sheet conformation and the CDRs form loops which connect, and in some cases form part of, the β-sheet structure. Thus, framework regions act to form a scaffold that provides for positioning the CDRs in correct orientation by inter-chain, non-covalent interactions. The antigen-binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined; see, "Sequences of Proteins of Immunological Interest," Kabat, E., et al., U.S. Department of Health and Human Services, (1983); and Chothia and Lesk, J. Mol. Biol., 196 (1987), 901-917, which are incorporated herein by reference in their entireties.

In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al., U.S. Dept. of Health and Human Services, "Sequences of Proteins of Immunological Interest" (1983) and by Chothia and Lesk, J. Mol. Biol., 196 (1987), 901-917, which are incorporated herein by reference, where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers that encompass a particular CDR will vary depending on the sequence and size of the CDR. Those skilled in the art can routinely determine which residues comprise a particular hypervariable region or CDR of the human IgG subtype of antibody given the variable region amino acid sequence of the antibody.

**Table A: Exemplary CDR Definitions¹**

| | **Kabat** | **Chothia** |
|---|---|---|
| VH CDR1 | 31-35 | 26-32 |
| VH CDR2 | 50-65 | 52-58 |
| VH CDR3 | 95-102 | 95-102 |
| VL CDR1 | 24-34 | 26-32 |
| VL CDR2 | 50-56 | 50-52 |
| VL CDR3 | 89-97 | 91-96 |

| | | |
|---|---|---|
| ¹Numbering of all CDR definitions in Table A is according to the numbering conventions set forth by Kabat et al. (see below). | | |

Kabat et al. also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody or antigen-binding fragment, variant, or derivative thereof described herein are according to the Kabat numbering system, which however is theoretical and may not equally apply every antibody described herein. For example, depending on the position of the first CDR the following CDRs might be shifted in either direction.

For the avoidance of confusion, where the term CDR is used without specifying the method used to identify the CDRs, the term refers to CDRs identified using the Paratome prediction method, e.g., as shown in Table 1.

Antibodies or antigen-binding fragments, immunospecific fragments, variants, or derivatives thereof described herein include, but are not limited to, monoclonal, bispecific, multispecific, human, humanized, primatized, murinized or chimeric antibodies, single chain antibodies, epitope-binding fragments, e.g., Fab, Fab' and F(ab')2, Fd, Fvs, single-chain Fvs (scFv), single-chain antibodies, disulfide-linked Fvs (sdFv), fragments comprising either a VL or VH domain, fragments produced by a Fab expression library, and anti-idiotypic (anti-Id) antibodies (including, e.g., anti-Id antibodies to antibodies disclosed herein). ScFv molecules are known in the art and are described, e.g., in US patent 5,892,019. Immunoglobulin or antibody molecules described herein can be of any type (e.g., IgG, IgE, IgM, IgD, IgA, and IgY), class (e.g., IgG1, IgG2, IgG3, IgG4, IgA1 and IgA2) or subclass of immunoglobulin molecule.

In some embodiments, the antibody is not IgM or a derivative thereof with a pentavalent structure. Particular, in specific applications, especially therapeutic use, IgMs are less useful than IgG and other bivalent antibodies or corresponding binding molecules since IgMs due to their pentavalent structure and lack of affinity maturation often show unspecific cross-reactivities and very low affinity.

In some embodiments, the antibody is an IgM or a derivative thereof with a pentavalent structure.

In some embodiments, the antibody is not a polyclonal antibody, i.e. it substantially consists of one particular antibody species rather than being a mixture obtained from a plasma immunoglobulin sample.

Antibody fragments, including single-chain antibodies, may comprise the variable region(s) alone or in combination with the entirety or a portion of the following: hinge region, CH1, CH2, and CH3 domains. Also provided herein are BMPRI/BMPRII-binding fragments also comprising any combination of variable region(s) with a hinge region, CH1, CH2, and CH3 domains. Antibodies or immunospecific fragments thereof described herein may be from any animal origin including birds and mammals. In some embodiments, the antibodies are human, murine, donkey, rabbit, goat, guinea pig, camel, llama, horse, or chicken antibodies. In another embodiment, the variable region may be condricthoid in origin (e.g., from sharks).

In some embodiments, the antibody is a human monoclonal antibody isolated from a human. Optionally, the framework region of the human antibody is aligned and adopted in accordance with the pertinent human germ line variable region sequences in the database; see, e.g., Vbase (vbase.mrc-cpe.cam.ac.uk) hosted by the MRC Centre for Protein Engineering (Cambridge, UK). For example, amino acids considered to potentially deviate from the true germ line sequence could be due to the PCR primer sequences incorporated during the cloning process. Compared to artificially generated human-like antibodies such as single chain antibody fragments (scFvs) from a phage displayed antibody library or xenogeneic mice the human monoclonal antibody described herein is characterized by (i) being obtained using the human immune response rather than that of animal surrogates, i.e. the antibody has been generated in response to natural BMPRI/BMPRII in its relevant conformation in the human body, (ii) having protected the individual or is at least significant for the presence of BMPRI/BMPRII, and (iii) since the antibody is of human origin the risks of cross-reactivity against self-antigens is minimized. Thus, as used herein, the terms "human monoclonal antibody", "human monoclonal autoantibody", "human antibody" and the like are used to denote a BMPRI/BMPRII binding molecule which is of human origin, i.e. which has been isolated from a human cell such as a B cell or hybridoma thereof or the cDNA of which has been directly cloned from mRNA of a human cell, for example a human memory B cell. A human antibody is still "human" even if amino acid substitutions are made in the antibody, e.g., to improve binding characteristics.

Antibodies derived from human immunoglobulin libraries or from animals transgenic for one or more human immunoglobulins and that do not express endogenous immunoglobulins, as described infra and, for example in, US patent no 5,939,598 by Kucherlapati et al., are denoted human-like antibodies in order distinguish them from truly human antibodies described herein.

For example, the pairing of heavy and light chains of human-like antibodies such as synthetic and semi-synthetic antibodies typically isolated from phage display do not necessarily reflect the original pairing as it occurred in the original human B cell. Accordingly Fab and scFv fragments obtained from recombinant expression libraries as commonly used in the prior art can be considered as being artificial with all possible associated effects on immunogenicity and stability.

As used herein, the term "heavy chain portion" includes amino acid sequences derived from an immunoglobulin heavy chain. A polypeptide comprising a heavy chain portion comprises at least one of: a CH1 domain, a hinge (e.g., upper, middle, and/or lower hinge region) domain, a CH2 domain, a CH3 domain, or a variant or fragment thereof. For example, a binding polypeptide for use in a method described herein may comprise a polypeptide chain comprising a CH1 domain; a polypeptide chain comprising a CH1 domain, at least a portion of a hinge domain, and a CH2 domain; a polypeptide chain comprising a CH1 domain and a CH3 domain; a polypeptide chain comprising a CH1 domain, at least a portion of a hinge domain, and a CH3 domain, or a polypeptide chain comprising a CH1 domain, at least a portion of a hinge domain, a CH2 domain, and a CH3 domain. In another embodiment, a polypeptide described herein comprises a polypeptide chain comprising a CH3 domain. Further, a binding polypeptide for use in a method described herein may lack at least a portion of a CH2 domain (e.g., all or part of a CH2 domain). As set forth above, it will be understood by one of ordinary skill in the art that these domains (e.g., the heavy chain portions) may be modified such that they vary in amino acid sequence from the naturally occurring immunoglobulin molecule.

In certain antibodies, or antigen-binding fragments, variants, or derivatives thereof disclosed herein, the heavy chain portions of one polypeptide chain of a multimer are identical to those on a second polypeptide chain of the multimer. Alternatively, heavy chain portion-containing monomers described herein are not identical. For example, each monomer may comprise a different target binding site, forming, for example, a bispecific antibody or diabody.

In some embodiments, the antibodies, or antigen-binding fragments, variants, or derivatives thereof disclosed herein are composed of a single polypeptide chain such as scFvs and are to be expressed intracellularly (intrabodies) for potential in vivo therapeutic and diagnostic applications.

The heavy chain portions of a binding polypeptide for use in the diagnostic and treatment methods disclosed herein may be derived from different immunoglobulin molecules. For example, a heavy chain portion of a polypeptide may comprise a CH1 domain derived from an IgG1 molecule and a hinge region derived from an IgG3 molecule. In another example, a heavy chain portion can comprise a hinge region derived, in part, from an IgG1 molecule and, in part, from an IgG3 molecule. In another example, a heavy chain portion can comprise a chimeric hinge derived, in part, from an IgG1 molecule and, in part, from an IgG4 molecule.

As used herein, the term "light chain portion" includes amino acid sequences derived from an immunoglobulin light chain. In some embodiments, the light chain portion comprises at least one of a VL or CL domain.

The minimum size of a peptide or polypeptide epitope for an antibody is thought to be about four to five amino acids. Peptide or polypeptide epitopes may contain at least seven, at least nine or between at least about 15 to about 30 amino acids. Since a CDR can recognize an antigenic peptide or polypeptide in its tertiary form, the amino acids comprising an epitope need not be contiguous, and in some cases, may not even be on the same peptide chain. In some embodiments, a peptide or polypeptide epitope recognized by antibodies described herein contains a sequence of at least 4, at least 5, at least 6, at least 7, at least 8, at least 9, at least 10, at least 15, at least 20, at least 25, or between about 5 to about 30, about 10 to about 30 or about 15 to about 30 contiguous or non-contiguous amino acids of a BMPRI/BMPRII.

By "specifically binding", or "specifically recognizing", used interchangeably herein, it is generally meant that a binding molecule, e.g., an antibody binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. A skilled artisan understands that an antibody may specifically bind to, or specifically recognize an isolated polypeptide comprising, or consisting of, amino acid residues corresponding to a linear portion of a non-contiguous epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D".

Where present, the term "immunological binding characteristics," or other binding characteristics of an antibody with an antigen, in all of its grammatical forms, refers to the specificity, affinity, cross-reactivity, and other binding characteristics of an antibody.

By "preferentially binding", it is meant that the binding molecule, e.g., antibody specifically binds to an epitope more readily than it would bind to a related, similar, homologous, or analogous epitope. Thus, an antibody which "preferentially binds" to a given epitope would more likely bind to that epitope than to a related epitope, even though such an antibody may cross-react with the related epitope.

By way of non-limiting example, a binding molecule, e.g., an antibody may be considered to bind a first epitope preferentially if it binds said first epitope with a dissociation constant (K_{D}) that is less than the antibody's K_{D} for the second epitope. In another non-limiting example, an antibody may be considered to bind a first antigen preferentially if it binds the first epitope with an affinity that is at least one order of magnitude less than the antibody's K_{D} for the second epitope. In another non-limiting example, an antibody may be considered to bind a first epitope preferentially if it binds the first epitope with an affinity that is at least two orders of magnitude less than the antibody's K_{D} for the second epitope.

In another non-limiting example, a binding molecule, e.g., an antibody may be considered to bind a first epitope preferentially if it binds the first epitope with an off rate (k(off)) that is less than the antibody's k(off) for the second epitope. In another non-limiting example, an antibody may be considered to bind a first epitope preferentially if it binds the first epitope with an affinity that is at least one order of magnitude less than the antibody's k(off) for the second epitope. In another non-limiting example, an antibody may be considered to bind a first epitope preferentially if it binds the first epitope with an affinity that is at least two orders of magnitude less than the antibody's k(off) for the second epitope.

A binding molecule, e.g., an antibody is said to competitively inhibit binding of a reference antibody to a given epitope if it preferentially binds to that epitope to the extent that it blocks, to some degree, binding of the reference antibody to the epitope. Competitive inhibition may be determined by any method known in the art, for example, competition ELISA assays. An antibody may be said to competitively inhibit binding of the reference antibody to a given epitope by at least 90%, at least 80%, at least 70%, at least 60%, or at least 50%. A skilled artisan understands that the binding of an antibody to its epitope may also be competitively inhibited by a binding molecule that is not an antibody.

As used herein, the term "affinity" refers to a measure of the strength of the binding of an individual epitope with the CDR of a binding molecule, e.g., an immunoglobulin molecule; see, e.g., Harlow et al., Antibodies: A Laboratory Manual, Cold Spring Harbor Laboratory Press, 2nd ed. (1988) at pages 27-28. As used herein, the term "avidity" refers to the overall stability of the complex between a population of immunoglobulins and an antigen, that is, the functional combining strength of an immunoglobulin mixture with the antigen; see, e.g., Harlow at pages 29-34. Avidity is related to both the affinity of individual immunoglobulin molecules in the population with specific epitopes, and also the valencies of the immunoglobulins and the antigen. For example, the interaction between a bivalent monoclonal antibody and an antigen with a highly repeating epitope structure, such as a polymer, would be one of high avidity. The affinity or avidity of an antibody for an antigen can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. General techniques for measuring the affinity of an antibody for an antigen include ELISA, RIA, and surface plasmon resonance. The measured affinity of a particular antibody-antigen interaction can vary if measured under different conditions, e.g., salt concentration, pH. Thus, measurements of affinity and other antigen-binding parameters, e.g., K_{D}, IC50, are preferably made with standardized solutions of antibody and antigen, and a standardized buffer.

Binding molecules, e.g., antibodies or antigen-binding fragments, variants or derivatives thereof described herein may also be described or specified in terms of their cross-reactivity. As used herein, the term "cross-reactivity" refers to the ability of an antibody, specific for one antigen, to react with a second antigen; a measure of relatedness between two different antigenic substances. Thus, an antibody is cross reactive if it binds to an epitope other than the one that induced its formation. The cross reactive epitope generally contains many of the same complementary structural features as the inducing epitope, and in some cases, may actually fit better than the original.

For example, certain antibodies have some degree of cross-reactivity, in that they bind related, but non-identical epitopes, e.g., epitopes with at least 95%, at least 90%, at least 85%, at least 80%, at least 75%, at least 70%, at least 65%, at least 60%, at least 55%, and at least 50% identity (as calculated using methods known in the art and described herein) to a reference epitope. An antibody may be said to have little or no cross-reactivity if it does not bind epitopes with less than 95%, less than 90%, less than 85%, less than 80%, less than 75%, less than 70%, less than 65%, less than 60%, less than 55%, and less than 50% identity (as calculated using methods known in the art and described herein) to a reference epitope. An antibody may be deemed "highly specific" for a certain epitope, if it does not bind any other analog, ortholog, or homolog of that epitope.

Binding molecules, e.g., antibodies or antigen-binding fragments, variants or derivatives thereof described herein may also be described or specified in terms of their binding affinity to a BMPRI/BMPRII.

As previously indicated, the subunit structures and three dimensional configuration of the constant regions of the various immunoglobulin classes are well known. As used herein, the term "VH domain" includes the amino terminal variable domain of an immunoglobulin heavy chain and the term "CH1 domain" includes the first (most amino terminal) constant region domain of an immunoglobulin heavy chain. The CH1 domain is adjacent to the VH domain and is amino terminal to the hinge region of an immunoglobulin heavy chain molecule.

As used herein the term "CH2 domain" includes the portion of a heavy chain molecule that extends, e.g., from about residue 244 to residue 360 of an antibody using conventional numbering schemes (residues 244 to 360, Kabat numbering system; and residues 231-340, EU numbering system; see Kabat EA et al. op. cit), in the Fc domain. The CH2 domain is unique in that it is not closely paired with another domain. Rather, two N-linked branched carbohydrate chains are interposed between the two CH2 domains of an intact native IgG molecule. It is also well documented that the CH3 domain extends from the CH2 domain to the C-terminal of the IgG molecule and comprises approximately 108 residues.

As used herein, the term "hinge region" includes the portion of a heavy chain molecule that joins the CH1 domain to the CH2 domain. This hinge region comprises approximately 25 residues and is flexible, thus allowing the two N-terminal antigen-binding regions to move independently. Hinge regions can be subdivided into three distinct domains: upper, middle, and lower hinge domains; see Roux et al., J. Immunol. 161 (1998), 4083.

As used herein the term "disulfide bond" includes the covalent bond formed between two sulfur atoms. The amino acid cysteine comprises a thiol group that can form a disulfide bond or bridge with a second thiol group. In most naturally occurring IgG molecules, the CH1 and CL regions are linked by a disulfide bond and the two heavy chains are linked by two disulfide bonds at positions corresponding to 239 and 242 using the Kabat numbering system (position 226 or 229, EU numbering system).

As used herein, the terms "linked", "fused" or "fusion" are used interchangeably. These terms refer to the joining together of two more elements or components, by whatever means including chemical conjugation or recombinant means. An "in-frame fusion" refers to the joining of two or more polynucleotide open reading frames (ORFs) to form a continuous longer ORF, in a manner that maintains the correct translational reading frame of the original ORFs. Thus, a recombinant fusion protein is a single protein containing two or more segments that correspond to polypeptides encoded by the original ORFs (which segments are not normally so joined in nature). Although the reading frame is thus made continuous throughout the fused segments, the segments may be physically or spatially separated by, for example, in-frame linker sequence. For example, polynucleotides encoding the CDRs of an immunoglobulin variable region may be fused, in-frame, but be separated by a polynucleotide encoding at least one immunoglobulin framework region or additional CDR regions, as long as the "fused" CDRs are co-translated as part of a continuous polypeptide.

The term "expression" as used herein refers to a process by which a gene produces a biochemical, for example, an RNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product, and the translation of such mRNA into polypeptide(s). If the final desired product is a biochemical, expression includes the creation of that biochemical and any precursors. Expression of a gene produces a "gene product." As used herein, a gene product can be either a nucleic acid, e.g., a messenger RNA produced by transcription of a gene, or a polypeptide which is translated from a transcript. Gene products described herein further include nucleic acids with post transcriptional modifications, e.g., polyadenylation, or polypeptides with post translational modifications, e.g., methylation, glycosylation, the addition of lipids, association with other protein subunits, proteolytic cleavage, and the like.

As used herein, the term "sample" refers to any biological material obtained from a subject or patient. In one aspect, a sample can comprise blood, plasma or urine. In other aspects, a sample can comprise whole blood, plasma, B cells enriched from blood samples, and cultured cells (e.g., B cells from a subject). A sample can also include a biopsy or tissue sample including neural tissue. In still other aspects, a sample can comprise whole cells and/or a lysate of the cells. Blood samples can be collected by methods known in the art. In one aspect, the pellet can be resuspended by vortexing at 4°C in 200 µl buffer (20 mM Tris, pH. 7.5, 0.5% Nonidet, 1 mM EDTA, 1 mM PMSF, 0.1M NaCl, IX Sigma Protease Inhibitor, and IX Sigma Phosphatase Inhibitors 1 and 2). The suspension can be kept on ice for 20 minutes with intermittent vortexing. After spinning at 15,000 x g for 5 minutes at about 4°C, aliquots of supernatant can be stored at about -70°C.

As used herein, the terms "treat" or "treatment" refer to both therapeutic treatment and prophylactic or preventative measures, wherein the object is to prevent or slow down (lessen) an undesired physiological change or disorder described herein. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the manifestation of the condition or disorder is to be prevented.

By "subject" or "individual" or "animal" or "patient" or "mammal," is meant any subject, particularly a mammalian subject, e.g., a human patient, for whom diagnosis, prognosis, prevention, or therapy is desired.

A composition or method described herein that comprises components or steps can also consist essentially of, or consist of, those components or steps.

### II. Antibodies

Provided are anti-BMPRI/II antibodies and antigen-binding fragments thereof, e.g., antigen binding domains, and fusions/multimers thereof. Also provided herein are derivatives or variants of the anti-BMPRI/II antibodies or antigen-binding fragments thereof. Also provided herein are conjugates comprising such antibodies or BMPRI/BMPRII-binding fragments thereof or derivatives or variants thereof. In some embodiments, an antibody or BMPRI/BMPRII-binding fragment thereof described herein demonstrates the binding characteristics and/or biological properties as outlined for the antibodies illustrated in the Examples section below.

The antibodies and antigen-binding fragments thereof, e.g., antigen binding domains, may be characterized by comprising in their variable region, e.g., binding domain, at least one complementarity determining region (CDR) of the VH and/or VL variable region comprising any one of the amino acid sequences depicted herein. Exemplary corresponding nucleotide sequences encoding the above-identified variable regions are set forth herein (see below). An exemplary set of CDRs of the above amino acid sequences of the VH and/or VL region is provided below. Exemplary corresponding framework regions for the CDRs are also provided herein. However, as discussed in the following the person skilled in the art is well aware of the fact that in addition or alternatively CDRs may be used, which differ in their amino acid sequence from those set forth herein by one, two, three or even more amino acids in case of CDR2 and CDR3.

In some embodiments, an antibody or antigen-binding fragment thereof, e.g., antigen binding domain, described herein comprises at least one CDR comprising, or consisting of an amino acid sequence shown in the Examples, Table 1, or FIG. 7. In some embodiments, an antibody or antigen-binding fragment thereof described herein comprises one, two, three, four, five or six CDRs comprising, or consisting of an amino acid sequence shown in the Examples, Table 1, or FIG. 7. The following table provides exemplary scFv sequences.

| | **Clone** | **Scfv SEQ ID NO:** |
|---|---|---|
| **BMPRI target** | | |
| ALK1 (ACVRL1); | Clone ALK1.2 (anti-ACVRL1/ALK1) | 146 |
| | Clone ALK1.3 (anti-ACVRL1/ALK1) | 148 |
| | Clone ALK1.8 (anti-ACVRL1/ALK1)* | 150 |
| | Clone ALK1.8b (anti-ACVRL1/ALK1) | 152 |
| | | |
| ALK2 (ACVR1A); | Clone RI-2 (Anti-ACVR1/ALK2) | 160 |
| | Clone RI-3 (Anti-ACVR1/ALK2) | 162 |
| | Clone RI-4 (Anti-ACVR1/ALK2) | 164 |
| | Clone RI-2-7 (Anti- ACVR1/ALK2) | 166 |
| | Clone RI-9 (Anti-ACVR1/ALK2) | 168 |
| | | |
| ALK3 (BMPR1A); | Clone B15 (Anti-BMPR1A/ALK3) | 156 |
| | Clone B16 (Anti-BMPR1A/ALK3) | 158 |
| | | |
| ALK4 (ACVR1B); | Clone H5-2 (Anti-ACVR1B/ALK4) | 178 |
| | | |
| ALK5 (TGFBR1); | | -- |
| | | |
| ALK6 (BMPR1B); | Clone H3-2 (Anti-BMPR1B/ALK6) | 180 |
| | | |
| ALK7 (ACVR1C). | Clone H4-1 (Anti-ACVR1C/ALK7) | 182 |
| | Clone H4-4 (Anti-ACVR1C/ALK7) | 184 |
| | Clone H4-7 (Anti-ACVR1C/ALK7)) | 186 |
| | Clone H4-8 (Anti-ACVR1C/ALK7) | 188 |
| | | |

| **BMPRII target** | | |
|---|---|---|
| ACTRIIA (ACVR2A); | Clone RII-19 (Anti-ACVR2A/ACTRIIA) | 170 |
| | Clone H1-2 (Anti-ACVR2A/ACTRIIA) | 172 |
| | | |
| ACTRIIB (ACVR2B); | Clone H2-11 (Anti-ACVR2B/ACTRIIB) | 174 |
| | Clone H2-15 (Anti-ACVR2B/ACTRIIB) | 176 |
| | | |
| BMPRII (BMPR2); | Clone BMPR2.12 (anti-BMPR2/BMPRII) | 154 |
| | Clone BMPR2.23 (anti-BMPR2/BMPRII) | 212 |

In some embodiments, the antibody or antigen-binding fragment thereof, e.g., antigen binding domain, comprises a heavy chain variable region (VH) complementarity determining region 1 (CDR1), a VH CDR2, and a VH CDR3 comprising amino acid sequences as shown herein, e.g., in the Examples, FIG. 7, and/or Table 1, and optionally further comprises a light chain variable region (VL) CDR1, a VL CDR2, and a VL CDR3 amino acid sequences as shown herein, e.g., in the Examples, FIG. 7, and/or Table 1.

In some embodiments, an antibody or antigen-binding fragment thereof, e.g., antigen binding domain, described herein comprises a VH comprising a heavy chain framework region 1 (FR1) as set forth herein (i.e., some or all of the framework portions of SEQ ID NOs: 146, 148, 150, 152, 154, 160, 162, 164, 166, 168, 156, 158, 178, 180, 182, 184, 186, 188, 170, 172, 174, 176, or 212.

In some embodiments, an antibody or antigen-binding fragment thereof, e.g., antigen binding domain, described herein comprises CDR sequences at least 95% identical to CDR sequences in the Examples, FIG. 7, and/or Table 1; comprises VH CDRs 1, 2, 3 and VL CDRs 1, 2, 3 that are identical to complementary determining regions in the Examples, FIG. 7, and/or Table 1; comprises VH and/or VL sequences at least 95% identical to sequences in Fig. 7; or comprises a sequence at least 95% identical to sequences in the Examples, FIG. 7, and/or Table 1.

In some embodiments, an antigen binding domain, antibody or antigen-binding fragment thereof described herein comprises a VH comprising a VH CDR1, CDR2, or CDR2, or VL CDR1, CDR2, or CDR3 comprising or consisting of a sequence that has at least 80%, at least 85%, at least 90%, at least 95%, at least 98%, or 100% sequence identity to an amino acid sequence as set forth herein.

In some embodiments, the antibody or antigen-binding fragment thereof, e.g., antigen binding domain, comprises sequences with zero or 1 to 10 (e.g., up to 1, 2, 3, 4, 5, 6, 7, 8, 9, or 10) alterations, i.e., amino acid substitutions, additions, or deletions relative to the reference amino acid sequence, e.g., in the VH or LH. In some embodiments, the amino acid substitutions are conservative amino acid substitutions. In some embodiments, the alterations are not in a CDR; in some embodiments, there are 1 or 2 alterations in one of the CDRs, e.g., up to 2 alterations in one CDR, e.g., one light chain CDR. Such variants retain the ability to bind the same antigen as the parent antibody or antigen-binding fragment thereof.

A "conservative amino acid substitution" is one in which the amino acid residue is replaced with an amino acid residue having a side chain with a similar charge. Families of amino acid residues having side chains with similar charges have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains ( e.g., threonine, valine, isoleucine) and aromatic side chains (e.g., tyrosine, phenylalanine, tryptophan, histidine).

In some embodiments, a variant of an antibody described herein (e.g., an antibody or antigen-binding fragment thereof having amino acid substitutions, additions, or deletions relative to the VH and/or VL of the amino acid sequences set forth herein retains the ability to bind BMPRI/BMPRII and/or retains one or more characteristics of an antibody described in the examples below.

In some embodiments, an antibody or antigen-binding fragment thereof described herein comprises a heavy chain variable region (VH) comprising, or consisting of an amino acid sequence set forth herein. In some embodiments, an antibody or antigen-binding fragment thereof described herein comprises a light chain variable region (VL) comprising, or consisting of the amino acid sequence set forth herein. In some embodiments, an antibody or antigen-binding fragment thereof described herein comprises a heavy chain variable region (VH) comprising, or consisting of an amino acid sequence set forth herein, and further comprises a light chain variable region (VL) comprising, or consisting of the amino acid sequence set forth herein. In some embodiments, the antibody or antigen-binding fragment thereof comprises a VH and a VL of a sequence set forth herein.

In some embodiments, an antibody described herein comprises a heavy chain comprising a VH described herein. In some instances, the heavy chain comprises an IgG constant region. In some instances, the heavy chain comprises a human IgG constant region.

In some embodiments, an antibody described herein comprises a light chain comprising a VL described herein. In some instances, the light chain comprises a kappa constant region. In some instances, the light chain comprises a human kappa constant region. In some instances, the light chain comprises a lambda constant region. In some instances, the light chain comprises a human lambda constant region.

In some embodiments, an antibody thereof described herein comprises a heavy chain comprising a VH described herein and a light chain comprising a VL described herein, wherein the heavy chain and/or light chain comprises an IgG (e.g., a human IgG) constant region.

Alternatively, the antibody described herein is an antibody or antigen-binding fragment, derivative or variant thereof, which competes for binding to BMPRI/BMPRII with an antibody having VH CDRs 1-3 set forth herein. Alternatively, the antibody described herein is an antibody or antigen-binding fragment, derivative, or variant thereof, which competes for binding to BMPRI/BMPRII with an antibody having VL CDRs 1-3 set forth herein. Alternatively, the antibody described herein is an antibody or antigen-binding fragment, derivative, or variant thereof, which competes for binding to BMPRI/BMPRII with an antibody having VH CDRs 1-3 and VL CDRs 1-3 set forth herein. Alternatively, the antibody described herein is an antibody or antigen-binding fragment, derivative, or variant thereof, which competes for binding to BMPRI/BMPRII with an antibody having the VH and/or VL set forth herein. Those antibodies may be human, rodent (e.g., murine), chimeric, or humanized, in particular for therapeutic applications.

Alternatively, the antibody described herein is an antibody or antigen-binding fragment, derivative or variant thereof, which binds to the same epitope as an anti-BMPRI/BMPRII antibody comprising a VH and a VL as set forth herein.

Competition between antibodies is determined by an assay in which the immunoglobulin under test inhibits specific binding of a reference antibody to a common antigen, such as BMPRI/BMPRII. Numerous types of competitive binding assays are known, for example: solid phase direct or indirect radioimmunoassay (RIA), solid phase direct or indirect enzyme immunoassay (EIA), sandwich competition assay; see Stahli et al., Methods in Enzymology 9 (1983), 242-253; solid phase direct biotin-avidin EIA; see Kirkland et al., J. Immunol. 137 (1986), 3614-3619 and Cheung et al., Virology 176 (1990), 546-552; solid phase direct labeled assay, solid phase direct labeled sandwich assay; see Harlow and Lane, Antibodies, A Laboratory Manual, Cold Spring Harbor Press (1988); solid phase direct label RIA using I125 label; see Morel et al, Molec. Immunol. 25 (1988), 7-15 and Moldenhauer et al., Scand. J. Immunol. 32 (1990), 77-82. Typically, such an assay involves the use of purified BMPRI/BMPRII or aggregates thereof bound to a solid surface or cells bearing either of these, an unlabeled test immunoglobulin and a labeled reference immunoglobulin, i.e. the human monoclonal antibody described herein. Competitive inhibition is measured by determining the amount of label bound to the solid surface or cells in the presence of the test immunoglobulin. Usually the test immunoglobulin is present in excess. In some embodiments, the competitive binding assay is performed under conditions known in the art. Antibodies identified by competition assay (competing antibodies) include antibodies binding to the same epitope as the reference antibody and antibodies binding to an adjacent epitope sufficiently proximal to the epitope bound by the reference antibody for steric hindrance to occur. Usually, when a competing antibody is present in excess, it will inhibit specific binding of a reference antibody to a common antigen by at least 50% or 75%. Hence, the present invention is further drawn to an antibody, or antigen-binding fragment, variant or derivatives thereof, where the antibody competitively inhibits a reference antibody comprising a VH and a VL of SEQ ID as set forth herein from binding to BMPRI/BMPRII.

In some embodiments, provided herein are isolated polypeptides comprising an immunoglobulin heavy chain variable region (VH), where at least one of the VH-CDRs of the heavy chain variable region or at least two of the VH-CDRs of the heavy chain variable region are at least 80%, 85%, 90%, 95%, 96%, 97%, 98% or 99% identical to reference heavy chain VH-CDR1, VH-CDR2 or VH-CDR3 amino acid sequences from an antibody disclosed herein (see, e.g., Table 1 and the Examples for VH CDR sequences). Alternatively, the VH-CDR1, VH-CDR2 and VH-CDR3 regions of the VH are at least 80%, 85%, 90%, 95%, 96%, 97%, 98% or 99% identical to reference heavy chain VH -CDR1, VH-CDR2 and VH-CDR3 amino acid sequences from an antibody disclosed herein (see, e.g., Table 1, Fig. 7, and the Examples for VH CDR sequences). Thus, a heavy chain variable region described herein can have VH-CDR1, VH-CDR2 and VH-CDR3 polypeptide sequences related to sequences of Table 1, FIG. 7, and the Examples.

CDRs are defined by a variety of methods/systems by those skilled in the art. These systems and/or definitions have been developed and refined over a number of years and include Kabat, Chothia, IMGT, AbM, and Contact. The Kabat definition is based on sequence variability and generally is the most commonly used. The Chothia definition is based on the location of the structural loop regions. The IMGT system is based on sequence variability and location within the structure of the variable domain. The AbM definition is a compromise between Kabat and Chothia. The Contact definition is based on analyses of the available antibody crystal structures. An Exemplary system is a combination of Kabat and Chothia. Software programs (e.g., abYsis (bioinf.org.uk/abysis/sequence_input/key_annotation/key_annotation.cgi) and Paratome, Kunik et al. PLoS Comput Biol 8(2): e1002388 (2012); Kunik et al., Nucleic Acids Res. 2012 Jul;40(Web Server issue):W521-4 (2012)) are available and known to those of skill in the art for analysis of antibody sequences and determination of CDRs.

The specific CDR sequences defined in Table 1 are generally based on Paratome predictions. However, it will be understood that reference to a heavy chain CDR or CDRs and/or a light chain CDR or CDRs of a specific antibody will encompass all CDR definitions as known to those of skill in the art.

While Table 1 shows CDRs defined by the Paratome system, other CDR definitions, e.g., CDRs defined by the Kabat, AbM, Contact, IMGT, or Chothia system as shown in FIG. 7, are also included in the present invention, and can be easily identified by a person of ordinary skill in the art using the sequences presented in FIG. 7 and the Examples.

Isolated polypeptides described herein can comprise an immunoglobulin heavy chain variable region (VH) in which the VH-CDR1, VH-CDR2 and VH-CDR3 regions have polypeptide sequences that are identical to the VH-CDR1, VH-CDR2 and VH-CDR3 sequences shown herein.

Further, the isolated polypeptides can comprise an immunoglobulin heavy chain variable region (VH) in which the VH-CDR1, VH-CDR2 and VH-CDR3 regions have polypeptide sequences which are identical to the VH-CDR1, VH-CDR2 and VH-CDR3 sequences shown in Table 1 or the Examples, except for one, two, three, four, five, six, seven, eight, nine, or ten amino acid substitutions in any one VH-CDR. In some embodiments, the amino acid substitutions are conservative.

The isolated polypeptides can comprise an immunoglobulin light chain variable region (VL), where at least one of the VL-CDRs of the light chain variable region or at least two of the VL-CDRs of the light chain variable region are at least 80%, 85%, 90%, 95%, 96%, 97%, 98% or 99% identical to reference light chain VL-CDR1, VL-CDR2 or VL-CDR3 amino acid sequences from an antibody disclosed herein (see, e.g., the Examples, Table 1, and FIG. 7 for VL CDR sequences). Alternatively, the VL-CDR1, VL-CDR2 and VL-CDR3 regions of the VL can be at least 80%, 85%, 90%, 95%, 96%, 97%, 98% or 99% identical to reference light chain VL-CDR1, VL-CDR2 and VL-CDR3 amino acid sequences from an antibody disclosed herein (see e.g., the Examples, Table 1, and FIG. 7 for VL CDR sequences). Thus, the light chain variable region can have VL-CDR1, VL-CDR2 and VL-CDR3 polypeptide sequences related to the sequences of Table 1 or the Examples. While Table 1 shows VL-CDRs, other CDR definitions, e.g., VL-CDRs defined by the Kabat or Chothia system, are also included in the present invention.

Also provided herein are isolated polypeptides comprising an immunoglobulin light chain variable region (VL) in which the VL-CDR1, VL-CDR2 and VL-CDR3 regions have polypeptide sequences that are identical to the VL-CDR1, VL-CDR2 and VL-CDR3 sequences of Table 1 and FIG. 7.

The isolated polypeptides can also comprise an immunoglobulin light chain variable region (VL) in which the VL-CDR1, VL-CDR2 and VL-CDR3 regions have polypeptide sequences that are identical to the VL-CDR1, VL-CDR2 and VL-CDR3 sequences of Table 1 or FIG. 7, except for one, two, three, four, five, six, seven, eight, nine, or ten amino acid substitutions in any one VL-CDR. In some embodiments, the amino acid substitutions are conservative.

In general each of the antigen binding domains will include all of the CDRs from a single clone as listed in Table 1 or FIG. 7. In some embodiments, the antigen binding domains can include CDRs from multiple clones, so long as they retain antigen-binding ability.

An immunoglobulin or its encoding cDNA can be modified. Thus, the methods described herein can include any one of the step(s) of producing a chimeric antibody, humanized antibody, single-chain antibody, Fab-fragment, bi-specific antibody, fusion antibody, labeled antibody or an analog of any one of those. Corresponding methods are known to the person skilled in the art and are described, e.g., in Harlow and Lane "Antibodies, A Laboratory Manual", CSH Press, Cold Spring Harbor (1988). When derivatives of said antibodies are obtained by the phage display technique, surface plasmon resonance as employed in the BIAcore system can be used to increase the efficiency of phage antibodies that bind to the same epitope as that of any one of the antibodies described herein (Schier, Human Antibodies Hybridomas 7 (1996), 97-105; Malmborg, J. Immunol. Methods 183 (1995), 7-13). The production of chimeric antibodies is described, for example, in international application WO89/09622. Methods for the production of humanized antibodies are described in, e.g., European application EP-A1 0 239 400 and international application WO90/07861. A further source of antibodies to be utilized in accordance with the present invention are so-called xenogeneic antibodies. The general principle for the production of xenogeneic antibodies such as human-like antibodies in mice is described in, e.g., international applications WO91/10741, WO94/02602, WO96/34096 and WO 96/33735. As discussed above, the antibody described herein may exist in a variety of forms besides complete antibodies; including, for example, Fv, Fab and F(ab)2, as well as in single chains; see e.g. international application WO88/09344. Antibodies made by such methods are also provided herein.

The antibodies described herein or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Modifications of the antibody described herein include chemical and/or enzymatic derivatizations at one or more constituent amino acids, including side chain modifications, backbone modifications, and N- and C-terminal modifications including acetylation, hydroxylation, methylation, amidation, and the attachment of carbohydrate or lipid moieties, cofactors, and the like. Likewise, the present invention encompasses the production of chimeric proteins, which comprise the described antibody or some fragment thereof at the amino terminus fused to heterologous molecule such as an immunostimulatory ligand at the carboxyl terminus; see, e.g., international application WO00/30680 for corresponding technical details.

Additionally, provided herein are peptides including those containing a binding molecule as described above, for example containing the CDR3 region of the variable region of any one of the mentioned antibodies, in particular CDR3 of the heavy chain since it has frequently been observed that heavy chain CDR3 (HCDR3) is the region having a greater degree of variability and a predominant participation in antigen-antibody interaction. Such peptides may easily be synthesized or produced by recombinant means to produce a binding agent as described herein. Such methods are well known to those of ordinary skill in the art. Peptides can be synthesized for example, using automated peptide synthesizers which are commercially available. The peptides can also be produced by recombinant techniques by incorporating the DNA expressing the peptide into an expression vector and transforming cells with the expression vector to produce the peptide.

Hence, described herein are binding molecules, e.g., an antibody or binding fragment thereof that is bind the anti-BMPRI/BMPRII antibodies described herein and display the mentioned properties, i.e., that specifically recognize (bind to) BMPRI/BMPRII. Such antibodies and binding molecules can be tested for their binding specificity and affinity by ELISA and Western Blot and immunohistochemisty as described herein, see, e.g., the Examples.

As an alternative to obtaining immunoglobulins directly from the culture of immortalized B cells or B memory cells, the immortalized cells describd herein can be used as a source of rearranged heavy chain and light chain loci for subsequent expression and/or genetic manipulation. Rearranged antibody genes can be reverse transcribed from appropriate mRNAs to produce cDNA. If desired, the heavy chain constant region can be exchanged for that of a different isotype or eliminated altogether. The variable regions can be linked to encode single chain Fv regions. Multiple Fv regions can be linked to confer binding ability to more than one target or chimeric heavy and light chain combinations can be employed. Once the genetic material is available, design of analogs as described above which retain both their ability to bind the desired target is straightforward. Methods for the cloning of antibody variable regions and generation of recombinant antibodies are known to the person skilled in the art and are described, for example, Gilliland et al., Tissue Antigens 47 (1996), 1-20; Doenecke et al., Leukemia 11 (1997), 1787-1792.

Once the appropriate genetic material is obtained and, if desired, modified to encode an analog, the coding sequences, including those that encode, at a minimum, the variable regions of the heavy and light chain, can be inserted into expression systems contained on vectors which can be transfected into standard recombinant host cells. A variety of such host cells may be used; for efficient processing, however, mammalian cells may be considered. Typical mammalian cell lines useful for this purpose include, but are not limited to, CHO cells, HEK 293 cells, or NSO cells.

The production of the antibody or analog is then undertaken by culturing the modified recombinant host under culture conditions appropriate for the growth of the host cells and the expression of the coding sequences. The antibodies are then recovered by isolating them from the culture. The expression systems are designed to include signal peptides so that the resulting antibodies are secreted into the medium; however, intracellular production is also possible.

In accordance with the above, the present invention also relates to a polynucleotide encoding the antibody or equivalent binding molecule described herein. In some embodiments, the polynucleotide encodes at least a variable region of an immunoglobulin chain of the antibody described above. Typically, said variable region encoded by the polynucleotide comprises at least one complementarity determining region (CDR) of the VH and/or VL of the variable region of the said antibody.

A person skilled in the art will readily appreciate that the variable domain of an antibody having an above-described variable domains can be used for the construction of other polypeptides or antibodies of desired specificity and biological function. Thus, provided herein are polypeptides and antibodies comprising at least one or more CDR, e.g., all of the CDRs, of the above-described variable domains and that advantageously have substantially the same or similar binding properties as the antibody described in the appended examples. The person skilled in the art knows that binding affinity can be enhanced by making amino acid substitutions within the CDRs or within the hypervariable loops (Chothia and Lesk, J. Mol. Biol. 196 (1987), 901-917) which partially overlap with the CDRs as defined by Kabat; see, e.g., Riechmann, et al, Nature 332 (1988), 323-327. Thus, antibodies wherein one or more of the mentioned CDRs comprise one or more, or not more than two amino acid substitutions, also provided herein. In some embodiments, the antibody described herein comprises in one or both of its immunoglobulin chains two or all three CDRs of the variable regions as set forth in Table 1.

Binding molecules, e.g., antibodies, or antigen-binding fragments, variants, or derivatives thereof described herein, as known by those of ordinary skill in the art, can comprise a constant region, which mediates one or more effector functions. For example, binding of the C1 component of complement to an antibody constant region may activate the complement system. Activation of complement is important in the opsonization and lysis of cell pathogens. The activation of complement also stimulates the inflammatory response and may also be involved in autoimmune hypersensitivity. Further, antibodies bind to receptors on various cells via the Fc region, with a Fc receptor binding site on the antibody Fc region binding to a Fc receptor (FcR) on a cell. There are a number of Fc receptors which are specific for different classes of antibody, including IgG (gamma receptors), IgE (epsilon receptors), IgA (alpha receptors) and IgM (mu receptors). Binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell-mediated cytotoxicity, or ADCC), release of inflammatory mediators, placental transfer and control of immunoglobulin production.

Accordingly, some embodiments described herein include an antibody, or antigen-binding fragment, variant, or derivative thereof, in which at least a fraction of one or more of the constant region domains has been deleted or otherwise altered so as to provide desired biochemical characteristics such as reduced effector functions, the ability to non-covalently dimerize, increased ability to localize at the site of BMPRI/BMPRII, reduced serum half-life, or increased serum half-life when compared with a whole, unaltered antibody of approximately the same immunogenicity. For example, some antibodies for use in the diagnostic and treatment methods described herein are domain deleted antibodies which comprise a polypeptide chain similar to an immunoglobulin heavy chain, but which lack at least a portion of one or more heavy chain domains. For instance, in certain antibodies, one entire domain of the constant region of the modified antibody will be deleted, for example, all or part of the CH2 domain will be deleted. In other embodiments, certain antibodies for use in the diagnostic and treatment methods described herein have a constant region, e.g., an IgM heavy chain constant region, which is altered to eliminate glycosylation, referred to elsewhere herein as aglycosylated or "agly" antibodies. Such "agly" antibodies may be prepared enzymatically as well as by engineering the consensus glycosylation site(s) in the constant region. While not being bound by theory, it is believed that "agly" antibodies may have an improved safety and stability profile in vivo. Methods of producing aglycosylated antibodies, having desired effector function are found for example in international application WO2005/018572, which is incorporated by reference in its entirety.

In certain antibodies, or antigen-binding fragments, variants, or derivatives thereof described herein, the Fc portion may be mutated to decrease effector function using techniques known in the art. For example, the deletion or inactivation (through point mutations or other means) of a constant region domain may reduce Fc receptor binding of the circulating modified antibody thereby increasing BMPRI/BMPRII localization. In other cases it may be that constant region modifications consistent with the instant invention moderate complement binding and thus reduce the serum half life and nonspecific association of a conjugated cytotoxin. Yet other modifications of the constant region may be used to modify disulfide linkages or oligosaccharide moieties that allow for enhanced localization due to increased antigen specificity or antibody flexibility. The resulting physiological profile, bioavailability and other biochemical effects of the modifications, such as BMPRI/BMPRII localization, biodistribution and serum half-life, may easily be measured and quantified using well know immunological techniques without undue experimentation.

In some antibodies, or antigen-binding fragments, variants, or derivatives thereof described herein, the Fc portion may be mutated or exchanged for alternative protein sequences to increase the cellular uptake of antibodies by way of example by enhancing receptor-mediated endocytosis of antibodies via Fcγ receptors, LRP, or Thy1 receptors or by 'SuperAntibody Technology', which is said to enable antibodies to be shuttled into living cells without harming them (Expert Opin. Biol. Ther. (2005), 237-241). For example, the generation of fusion proteins of the antibody-binding region and the cognate protein ligands of cell surface receptors or bi- or multi-specific antibodies with a specific sequences biding to BMPRI/BMPRII as well as a cell surface receptor may be engineered using techniques known in the art.

In some antibodies, or antigen-binding fragments, variants, or derivatives thereof described herein, the Fc portion may be mutated or exchanged for alternative protein sequences or the antibody may be chemically modified to increase its blood brain barrier penetration.

Modified forms of antibodies, or antigen-binding fragments, variants, or derivatives thereof described herein can be made from whole precursor or parent antibodies using techniques known in the art. Exemplary techniques are discussed in more detail herein. Antibodies, or antigen-binding fragments, variants, or derivatives thereof described herein can be made or manufactured using techniques that are known in the art. In some embodiments, antibody molecules or fragments thereof are "recombinantly produced," i.e., are produced using recombinant DNA technology. Exemplary techniques for making antibody molecules or fragments thereof are discussed in more detail elsewhere herein.

Antibodies, or antigen-binding fragments, variants, or derivatives thereof described herein also include derivatives that are modified, e.g., by the covalent attachment of any type of molecule to the antibody such that covalent attachment does not prevent the antibody from specifically binding to its cognate epitope. For example, but not by way of limitation, the antibody derivatives include antibodies that have been modified, e.g., by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

In some embodiments, antibodies, or antigen-binding fragments, variants, or derivatives thereof described herein will not elicit a deleterious immune response in the animal to be treated, e.g., in a human. In some embodiments, binding molecules, e.g., antibodies, or antigen-binding fragments thereof described herein are derived from a patient, e.g., a human patient, and are subsequently used in the same species from which they are derived, e.g., human, alleviating or minimizing the occurrence of deleterious immune responses.

De-immunization can also be used to decrease the immunogenicity of an antibody. As used herein, the term "de-immunization" includes alteration of an antibody to modify T cell epitopes; see, e.g., international applications WO98/52976 and WO00/34317. For example, VH and VL sequences from the starting antibody are analyzed and a human T cell epitope "map" from each V region showing the location of epitopes in relation to complementarity determining regions (CDRs) and other key residues within the sequence. Individual T cell epitopes from the T cell epitope map are analyzed in order to identify alternative amino acid substitutions with a low risk of altering activity of the final antibody. A range of alternative VH and VL sequences are designed comprising combinations of amino acid substitutions and these sequences are subsequently incorporated into a range of binding polypeptides, e.g., BMPRI/BMPRII-specific antibodies or immunospecific fragments thereof for use in the diagnostic and treatment methods disclosed herein, which are then tested for function. Typically, between 12 and 24 variant antibodies are generated and tested. Complete heavy and light chain genes comprising modified V and human C regions are then cloned into expression vectors and the subsequent plasmids introduced into cell lines for the production of whole antibody. The antibodies are then compared in appropriate biochemical and biological assays, and the optimal variant is identified.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, Cold Spring Harbor Laboratory Press, 2nd ed. (1988); Hammerling et al., in: Monoclonal Antibodies and T-Cell Hybridomas Elsevier, N.Y., 563-681 (1981), said references incorporated by reference in their entireties. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Thus, the term "monoclonal antibody" is not limited to antibodies produced through hybridoma technology.

In a well-known hybridoma process (Kohler et al., Nature 256 (1975), 495) the relatively short-lived, or mortal, lymphocytes from a mammal, e.g., B cells derived from a murine subject as described herein, are fused with an immortal tumor cell line (e.g.,. a myeloma cell line), thus, producing hybrid cells or "hybridomas" which are both immortal and capable of producing the genetically coded antibody of the B cell. The resulting hybrids are segregated into single genetic strains by selection, dilution, and regrowth with each individual strain comprising specific genes for the formation of a single antibody. They produce antibodies, which are homogeneous against a desired antigen and, in reference to their pure genetic parentage, are termed "monoclonal".

Hybridoma cells thus prepared are seeded and grown in a suitable culture medium that contain one or more substances that inhibit the growth or survival of the unfused, parental myeloma cells. Those skilled in the art will appreciate that reagents, cell lines and media for the formation, selection and growth of hybridomas are commercially available from a number of sources and standardized protocols are well established. Generally, culture medium in which the hybridoma cells are growing is assayed for production of monoclonal antibodies against the desired antigen. The binding specificity of the monoclonal antibodies produced by hybridoma cells is determined by in vitro assays such as immunoprecipitation, radioimmunoassay (RIA) or enzyme-linked immunoabsorbent assay (ELISA) as described herein. After hybridoma cells are identified that produce antibodies of the desired specificity, affinity and/or activity, the clones may be subcloned by limiting dilution procedures and grown by standard methods; see, e.g., Goding, Monoclonal Antibodies: Principles and Practice, Academic Press, pp 59-103 (1986). It will further be appreciated that the monoclonal antibodies secreted by the subclones may be separated from culture medium, ascites fluid or serum by conventional purification procedures such as, for example, protein-A, hydroxylapatite chromatography, gel electrophoresis, dialysis or affinity chromatography.

In some embodiments, lymphocytes can be selected by micromanipulation and the variable genes isolated. For example, peripheral blood mononuclear cells can be isolated from an immunized or naturally immune mammal, e.g., a human, and cultured for about 7 days in vitro. The cultures can be screened for specific immunoglobulins that meet the screening criteria. Cells from positive wells can be isolated. Individual Ig-producing B cells can be isolated by FACS or by identifying them in a complement-mediated hemolytic plaque assay. Ig-producing B cells can be micromanipulated into a tube and the VH and VL genes can be amplified using, e.g., RT-PCR. The VH and VL genes can be cloned into an antibody expression vector and transfected into cells (e.g., eukaryotic or prokaryotic cells) for expression.

Alternatively, antibody-producing cell lines may be selected and cultured using techniques well known to the skilled artisan. Such techniques are described in a variety of laboratory manuals and primary publications. In this respect, techniques suitable for use in the methods and compositions as described below are described in Current Protocols in Immunology, Coligan et al., Eds., Green Publishing Associates and Wiley-Interscience, John Wiley and Sons, New York (1991) which is herein incorporated by reference in its entirety, including supplements.

Antibody fragments that recognize specific epitopes may be generated by known techniques. For example, Fab and F(ab')2 fragments may be produced recombinantly or by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Such fragments are sufficient for use, for example, in immunodiagnostic procedures involving coupling the immunospecific portions of immunoglobulins to detecting reagents such as radioisotopes.

In some embodiments, an antibody described herein comprises at least one heavy or light chain CDR of an antibody molecule. In another embodiment, an antibody described herein comprises at least two CDRs from one or more antibody molecules. In another embodiment, an antibody described herein comprises at least three CDRs from one or more antibody molecules. In another embodiment, an antibody described herein comprises at least four CDRs from one or more antibody molecules. In another embodiment, an antibody described herein comprises at least five CDRs from one or more antibody molecules. In another embodiment, an antibody described herein comprises at least six CDRs from one or more antibody molecules. Exemplary antibody molecules comprising at least one CDR that can be included in the subject antibodies are described herein.

Antibodies described herein can be produced by any method known in the art for the synthesis of antibodies, in particular, by chemical synthesis or by recombinant expression techniques as described herein.

In some embodiments, an antibody, or antigen-binding fragment, variant, or derivative thereof described herein comprises a synthetic constant region wherein one or more domains are partially or entirely deleted ("domain-deleted antibodies"). In some embodiments compatible modified antibodies will comprise domain deleted constructs or variants wherein the entire CH2 domain has been removed. For other embodiments a short connecting peptide may be substituted for the deleted domain to provide flexibility and freedom of movement for the variable region. Those skilled in the art will appreciate that such constructs are particularly preferred due to the regulatory properties of the CH2 domain on the catabolic rate of the antibody. Domain deleted constructs can be derived using a vector encoding an IgG1 human constant domain, see, e.g., international applications WO02/060955 and WO02/096948A2. This vector is engineered to delete the CH2 domain and provide a synthetic vector expressing a domain deleted IgG1 constant region.

In some embodiments, antibodies, or antigen-binding fragments, variants, or derivatives thereof described herein are minibodies. Minibodies can be made using methods described in the art, see, e.g., US patent 5,837,821 or international application WO 94/09817.

In some embodiments, an antibody, or antigen-binding fragment, variant, or derivative thereof described herein comprises an immunoglobulin heavy chain having deletion or substitution of a few or even a single amino acid as long as it permits association between the monomeric subunits and retains binding to BMPRI/BMPRII. For example, the mutation of a single amino acid in selected areas of the CH2 domain may be enough to substantially reduce Fc binding and thereby increase BMPRI/BMPRII localization. Similarly, it may be desirable to simply delete that part of one or more constant region domains that control the effector function (e.g. complement binding) to be modulated. Such partial deletions of the constant regions may improve selected characteristics of the antibody (serum half-life) while leaving other desirable functions associated with the subject constant region domain intact. Moreover, as alluded to above, the constant regions of the disclosed antibodies may be synthetic through the mutation or substitution of one or more amino acids that enhances the profile of the resulting construct. In this respect it may be possible to disrupt the activity provided by a conserved binding site (e.g., Fc binding) while substantially maintaining the configuration and immunogenic profile of the modified antibody. Yet other embodiments comprise the addition of one or more amino acids to the constant region to enhance desirable characteristics such as effector function or provide for more cytotoxin or carbohydrate attachment. In such embodiments, it may be desirable to insert or replicate specific sequences derived from selected constant region domains.

The present invention also provides antibodies that comprise, consist essentially of, or consist of, variants (including derivatives) of antibody molecules (e.g., the VH regions and/or VL regions) described herein, which antibodies or fragments thereof immunospecifically bind to BMPRI/BMPRII. Standard techniques known to those of skill in the art can be used to introduce mutations in the nucleotide sequence encoding an antibody, including, but not limited to, site-directed mutagenesis and PCR-mediated mutagenesis which result in amino acid substitutions. In some embodiments, the variants (including derivatives) encode less than 50 amino acid substitutions, less than 40 amino acid substitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference VH region, VH-CDR1, VH-CDR2, VH-CDR3, VL region, VL-CDR1, VL-CDR2, or VL-CDR3. Alternatively, mutations can be introduced randomly along all or part of the coding sequence, such as by saturation mutagenesis, and the resultant mutants can be screened for biological activity to identify mutants that retain activity (e.g., the ability to bind BMPRI/BMPRII).

For example, it is possible to introduce mutations only in framework regions or only in CDR regions of an antibody molecule. Introduced mutations may be silent or neutral missense mutations, e.g., have no, or little, effect on an antibody's ability to bind antigen, indeed some such mutations do not alter the amino acid sequence whatsoever. These types of mutations may be useful to optimize codon usage, or improve a hybridoma's antibody production. Codon-optimized coding regions encoding antibodies described herein are disclosed elsewhere herein. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen-binding activity or alteration in binding activity (e.g., improvements in antigen-binding activity or change in antibody specificity). Following mutagenesis, the encoded protein may routinely be expressed and the functional and/or biological activity of the encoded protein, (e.g., ability to immunospecifically bind at least one epitope of BMPRI/BMPRII) can be determined using techniques described herein or by routinely modifying techniques known in the art.

BMPRI/BMPRII-binding agents, for example, but not limited to, BMPRI/BMPRII-binding antibodies described herein may be characterized using any in vivo or in vitro models. A skilled artisan readily understands that a BMPRI/BMPRII binding agent (e.g., an antibody) described herein may be characterized in a mouse model described herein.

### III. Polynucleotides Encoding Antibodies

Also provided herein are polynucleotides encoding an antibody, or antigen-binding fragment, variant, or derivative thereof described herein. A polynucleotide encoding an antibody, or antigen-binding fragment, variant, or derivative thereof can be composed of any polyribonucleotide or polydeoxribonucleotide, which may be unmodified RNA or DNA or modified RNA or DNA. For example, a polynucleotide encoding an antibody, or antigen-binding fragment, variant, or derivative thereof can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, a polynucleotide encoding an antibody, or antigen-binding fragment, variant, or derivative thereof can be composed of triple-stranded regions comprising RNA or DNA or both RNA and DNA. A polynucleotide encoding an antibody, or antigen-binding fragment, variant, or derivative thereof may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

An isolated polynucleotide encoding a non-natural variant of a polypeptide derived from an immunoglobulin (e.g., an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCR-mediated mutagenesis. In some embodiments, conservative amino acid substitutions are made at one or more non-essential amino acid residues.

As is well known, RNA may be isolated from the original B cells, hybridoma cells or from other transformed cells by standard techniques, such as guanidinium isothiocyanate extraction and precipitation followed by centrifugation or chromatography. Where desirable, mRNA may be isolated from total RNA by standard techniques such as chromatography on oligo dT cellulose. Suitable techniques are familiar in the art. In some embodiments, cDNAs that encode the light and the heavy chains of the antibody may be made, either simultaneously or separately, using reverse transcriptase and DNA polymerase in accordance with well known methods. PCR may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as human constant region probes.

DNA, typically plasmid DNA, may be isolated from the cells using techniques known in the art, restriction mapped and sequenced in accordance with standard, well known techniques set forth in detail, e.g., in the foregoing references relating to recombinant DNA techniques. Of course, the DNA may be synthetic according to the present invention at any point during the isolation process or subsequent analysis.

In some embodiments, provided herein is an isolated polynucleotide comprising, consisting essentially of, or consisting of a nucleic acid encoding an immunoglobulin heavy chain variable region (VH), where at least one of the CDRs of the heavy chain variable region or at least two of the VH-CDRs of the heavy chain variable region are at least 80%, 85%, 90%, 95%, 96%, 97%, 98%, or 99% identical to reference heavy chain VH-CDR1, VH-CDR2, or VH-CDR3 amino acid sequences from the antibodies disclosed herein. Alternatively, the VH-CDR1, VH-CDR2, or VH-CDR3 regions of the VH are at least 80%, 85%, 90%, 95%, 96%, 97%, 98%, or 99% identical to reference heavy chain VH-CDR1, VH-CDR2, and VH-CDR3 amino acid sequences from the antibodies disclosed herein. Thus, according to this embodiment a heavy chain variable region of an antibody or antigen-binding fragment thereof described herein has VH-CDR1, VH-CDR2, or VH-CDR3 polypeptide sequences related to the polypeptide sequences shown in the Examples.

In some embodiments, provided herein is an isolated polynucleotide comprising, consisting essentially of, or consisting of a nucleic acid encoding an immunoglobulin heavy chain variable region (VH), in which the VH-CDR1, VH-CDR2 and VH-CDR3 regions have polypeptide sequences which are identical to the VH-CDR1, VH-CDR2 and VH-CDR3 groups shown in the Examples, except for one, two, three, four, five, six, seven, eight, nine, or ten amino acid substitutions in any one VH-CDR. In some embodiments, the amino acid substitutions are conservative.

In another embodiment, provided herein is an isolated polynucleotide comprising, consisting essentially of, or consisting of a nucleic acid encoding an immunoglobulin light chain variable region (VL), where at least one of the VL-CDRs of the light chain variable region or at least two of the VL-CDRs of the light chain variable region are at least 80%, 85%, 90%, 95%, 96%, 97%, 98%, or 99% identical to reference light chain VL-CDR1, VL-CDR2, or VL-CDR3 amino acid sequences from the antibodies disclosed herein. Alternatively, the VL-CDR1, VL-CDR2, or VL-CDR3 regions of the VL are at least 80%, 85%, 90%, 95%, 96%, 97%, 98%, or 99% identical to reference light chain VL-CDR1, VL-CDR2, and VL-CDR3 amino acid sequences from the antibodies disclosed herein. Thus, according to this embodiment a light chain variable region of the antibody or antigen-binding fragment thereof described herein has VL-CDR1, VL-CDR2, or VL-CDR3 polypeptide sequences related to the polypeptide sequences shown in the Examples.

In another embodiment, provided herein is an isolated polynucleotide comprising, consisting essentially of, or consisting of a nucleic acid encoding an immunoglobulin light chain variable region (VL) in which the VL-CDR1, VL-CDR2 and VL-CDR3 regions have polypeptide sequences which are identical to the VL-CDR1, VL-CDR2 and VL-CDR3 groups shown in the Examples, except for one, two, three, four, five, six, seven, eight, nine, or ten amino acid substitutions in any one VL-CDR. In some embodiments the amino acid substitutions are conservative.

In another embodiment, provided herein is an isolated polynucleotide comprising, consisting essentially of, or consisting of a nucleic acid encoding an immunoglobulin heavy chain variable region (VH) in which the VH-CDR1, VH-CDR2, and VH-CDR3 regions have polypeptide sequences that are identical to the VH-CDR1, VH-CDR2, and VH-CDR3 groups shown in the Examples.

In another embodiment, provided herein is an isolated polynucleotide comprising, consisting essentially of, or consisting of a nucleic acid encoding an immunoglobulin light chain variable region (VL) in which the VL-CDR1, VL-CDR2, and VL-CDR3 regions have polypeptide sequences which are identical to the VL-CDR1, VL-CDR2, and VL-CDR3 groups shown in the Examples.

As known in the art, "sequence identity" between two polypeptides or two polynucleotides is determined by comparing the amino acid or nucleic acid sequence of one polypeptide or polynucleotide to the sequence of a second polypeptide or polynucleotide. When discussed herein, whether any particular polypeptide is at least about 40%, 45%, 50%, 55%, 60%, 65%, 70%, 75%, 80%, 85%, 90% or 95% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2 (1981), 482-489, to find the best segment of homology between two sequences. When using BESTFIT or any other sequence alignment program to determine whether a particular sequence is, for example, 95% identical to a reference sequence described herein (e.g., an antibody or antigen-binding fragment thereof described herein), the parameters are set, of course, such that the percentage of identity is calculated over the full length of the reference polypeptide sequence and that gaps in homology of up to 5% of the total number of amino acids in the reference sequence are allowed.

In some embodiments, the polynucleotide comprises, consists essentially of, or consists of a nucleic acid having a polynucleotide sequence of the VH or VL region of an anti-BMPRI/BMPRII antibody as depicted herein. In this respect, the person skilled in the art will readily appreciate that the polynucleotides encoding at least the variable domain of the light and/or heavy chain may encode the variable domain of both immunoglobulin chains or only one. Further provided herein is a polynucleotide comprising, or consisting of a nucleotide sequence encoding the amino acid sequence of an antibody or antigen binding fragment thereof as shown herein, e.g., in the Examples, FIG. 7, or Table 1.

In some embodiments, provided herein is an isolated polynucleotide comprising, consisting essentially of, or consisting of a nucleic acid encoding an immunoglobulin heavy chain variable region at least 80%, 85%, 90%, 95%, 96%, 97%, 98%, or 99% or 95% identical to reference heavy chain VH. In some embodiments, the amino acid sequence of the reference heavy chain variable region is as shown herein.

In some embodiments, provided herein is an isolated polynucleotide comprising, consisting essentially of, or consisting of a nucleic acid encoding an immunoglobulin light chain variable region at least 80%, 85%, 90%, 95%, 96%, 97%, 98%, or 99% or 95% identical to reference light chain VL. In some embodiments, the amino acid sequence of the reference light chain variable region is as shown herein.

Also provided herein are fragments of the polynucleotides described herein, as described elsewhere. Additionally polynucleotides which encode fusion polynucleotides, Fab fragments, and other derivatives, as described herein, are also contemplated.

The polynucleotides may be produced or manufactured by any method known in the art. For example, if the nucleotide sequence of the antibody is known, a polynucleotide encoding the antibody may be assembled from chemically synthesized oligonucleotides, e.g., as described in Kutmeier et al., BioTechniques 17 (1994), 242, which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody, or antigen-binding fragment, variant, or derivative thereof may be generated from nucleic acid from a suitable source. If a clone containing a nucleic acid encoding a particular antibody is not available, but the sequence of the antibody molecule is known, a nucleic acid encoding the antibody may be chemically synthesized or obtained from a suitable source (e.g., an antibody cDNA library, or a cDNA library generated from, or nucleic acid, preferably polyA+ RNA, isolated from, any tissue or cells expressing the BMPRI/BMPRII-specific antibody, such as hybridoma cells selected to express an antibody) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the antibody, or antigen-binding fragment, variant, or derivative thereof is determined, its nucleotide sequence may be manipulated using methods well known in the art for the manipulation of nucleotide sequences, e.g., recombinant DNA techniques, site directed mutagenesis, PCR etc. (see, for example, the techniques described in Sambrook et al., Molecular Cloning, A Laboratory Manual, 2d Ed., Cold Spring Harbor Laboratory, Cold Spring Harbor, N.Y. (1990) and Ausubel et al., eds., Current Protocols in Molecular Biology, John Wiley & Sons, NY (1998), which are both incorporated by reference herein in their entireties), to generate antibodies having a different amino acid sequence, for example to create amino acid substitutions, deletions, and/or insertions.

### IV. Fusion Proteins and Conjugates

In some embodiments, the antibody polypeptide comprises an amino acid sequence or one or more moieties not normally associated with an antibody. Exemplary modifications are described in more detail below. For example, a single-chain fv antibody fragment described herein may comprise a flexible linker sequence, or may be modified to add a functional moiety (e.g., PEG, a drug, a toxin, or a label such as a fluorescent, radioactive, enzyme, nuclear magnetic, heavy metal and the like)

An antibody polypeptide described herein may comprise, consist essentially of, or consist of a fusion protein. Fusion proteins are chimeric molecules which comprise, for example, an immunoglobulin BMPRI/BMPRII-binding domain with at least one target binding site, and at least one heterologous portion, i.e., a portion with which it is not naturally linked in nature. The amino acid sequences may normally exist in separate proteins that are brought together in the fusion polypeptide or they may normally exist in the same protein but are placed in a new arrangement in the fusion polypeptide. Fusion proteins may be created, for example, by chemical synthesis, or by creating and translating a polynucleotide in which the peptide regions are encoded in the desired relationship.

The term "heterologous" as applied to a polynucleotide or a polypeptide, means that the polynucleotide or polypeptide is derived from a distinct entity from that of the rest of the entity to which it is being compared. For instance, as used herein, a "heterologous polypeptide" to be fused to an antibody, or an antigen-binding fragment, variant, or analog thereof is derived from a non-immunoglobulin polypeptide of the same species, or an immunoglobulin or non-immunoglobulin polypeptide of a different species.

As discussed in more detail elsewhere herein, antibodies, or antigen-binding fragments, variants, or derivatives thereof described herein may further be recombinantly fused to a heterologous polypeptide at the N- or C-terminus or chemically conjugated (including covalent and non-covalent conjugations) to polypeptides or other compositions. For example, antibodies may be recombinantly fused or conjugated to molecules useful as labels in detection assays and effector molecules such as heterologous polypeptides, drugs, radionuclides, or toxins; see, e.g., international applications WO92/08495; WO91/14438; WO89/12624; US patent no. 5,314,995; and European patent application EP 0 396 387.

In some instances, the antibody or antigen-binding fragment thereof is conjugated to a drug. Methods of producing such drug conjugates are known in the art and described herein (see, e.g., Examples below). In some instances, the drug is an immunosuppressive drug (see, e.g., the section "Immunosuppressive or Immunoregulatory Drugs" below for examples of immunosuppressive drugs. In some instances, the drug is taxol. In some instances, the drug directly is conjugated to the antibody or antigen-binding fragment thereof. In some instances, the drug is conjugated to the antibody or antigen-binding fragment thereof via a linker. Examples of linkers that may be used to conjugate a drug to an antibody or antigen-binding fragment thereof described herein include succinimidyl 4-(N-maleimidomethyl)cyclohexane-1-carboxylate (SMCC) or maleimidocaproyl-valine-citrulline-p- aminobenzoyloxycarbonyl (mc-vc-PAB). In some instances, the drug is covalently conjugated to the antibody or antigen-binding fragment thereof. Covalent conjugation of the drug to the antibody or antigen-binding fragment thereof may be performed as described in the Examples section below. For example, the drug may be chemically modified using, e.g., glutaraldehyde, to produce a carboxylic acid group, which is then activated using 1-ethyl-3-(3-dimethylaminopropyl)carbodiimide hydrochloride (EDC)/N-hydroxysulfosuccinimide (NHS) chemistry; the resulting drug preferentially reacts with (i.e., conjugates to) primary amines on the antibody or antigen-binding fragment thereof (e.g., such as those on lysines).

Antibodies, or antigen-binding fragments, variants, or derivatives thereof described herein can be composed of amino acids joined to each other by peptide bonds or modified peptide bonds, i.e., peptide isosteres, and may contain amino acids other than the 20 gene-encoded amino acids. Antibodies may be modified by natural processes, such as posttranslational processing, or by chemical modification techniques which are well known in the art. Such modifications are well described in basic texts and in more detailed monographs, as well as in a voluminous research literature. Modifications can occur anywhere in the antibody, including the peptide backbone, the amino acid side-chains and the amino or carboxyl termini, or on moieties such as carbohydrates. It will be appreciated that the same type of modification may be present in the same or varying degrees at several sites in a given antibody. Also, a given antibody may contain many types of modifications. Antibodies may be branched, for example, as a result of ubiquitination, and they may be cyclic, with or without branching. Cyclic, branched, and branched cyclic antibodies may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination; see, e.g., Proteins - Structure And Molecular Properties, T. E. Creighton, W. H. Freeman and Company, New York 2nd Ed., (1993); Posttranslational Covalent Modification Of Proteins, B. C. Johnson, Ed., Academic Press, New York, pgs. 1-12 (1983); Seifter et al., Meth. Enzymol. 182 (1990), 626-646; Rattan et al., Ann. NY Acad. Sci. 663 (1992), 48-62).

Also provided herein are fusion proteins comprising an antibody, or antigen-binding fragment, variant, or derivative thereof, and a heterologous polypeptide. In some embodiments, a fusion protein comprises, consists essentially of, or consists of, a polypeptide having the amino acid sequence of any one or more of the VH regions of an antibody described herein or the amino acid sequence of any one or more of the VL regions of an antibody described herein or fragments or variants thereof, and a heterologous polypeptide sequence. In another embodiment, a fusion protein for use in the diagnostic and treatment methods disclosed herein comprises, consists essentially of, or consists of a polypeptide having the amino acid sequence of any one, two, three of the VH-CDRs of an antibody, or fragments, variants, or derivatives thereof, or the amino acid sequence of any one, two, three of the VL-CDRs of an antibody, or fragments, variants, or derivatives thereof, and a heterologous polypeptide sequence. In some embodiments, the fusion protein comprises a polypeptide having the amino acid sequence of a VH-CDR3 of an antibody described herein, or fragment, derivative, or variant thereof, and a heterologous polypeptide sequence, which fusion protein specifically binds to BMPRI/BMPRII. In another embodiment, a fusion protein comprises a polypeptide having the amino acid sequence of at least one VH region of an antibody described herein and the amino acid sequence of at least one VL region of an antibody described herein or fragments, derivatives or variants thereof, and a heterologous polypeptide sequence. In some embodiments, the VH and VL regions of the fusion protein correspond to a single source antibody (or scFv or Fab fragment) that specifically binds BMPRI/BMPRII. In yet another embodiment, a fusion protein for use in the diagnostic and treatment methods disclosed herein comprises a polypeptide having the amino acid sequence of any one, two, three or more of the VH CDRs of an antibody and the amino acid sequence of any one, two, three or more of the VL CDRs of an antibody, or fragments or variants thereof, and a heterologous polypeptide sequence. In some embodiments, two, three, four, five, six, or more of the VH-CDR(s) or VL-CDR(s) correspond to single source antibody (or scFv or Fab fragment) described herein. Nucleic acid molecules encoding these fusion proteins are also provided herein.

Exemplary fusion proteins reported in the literature include fusions of the T cell receptor (Gascoigne et al., Proc. Natl. Acad. Sci. USA 84 (1987), 2936-2940; CD4 (Capon et al., Nature 337 (1989), 525-531; Traunecker et al., Nature 339 (1989), 68-70; Zettmeissl et al., DNA Cell Biol. USA 9 (1990), 347-353; and Byrn et al., Nature 344 (1990), 667-670); L-selectin (homing receptor) (Watson et al., J. Cell. Biol. 110 (1990), 2221-2229; and Watson et al., Nature 349 (1991), 164-167); CD44 (Aruffo et al., Cell 61 (1990), 1303-1313); CD28 and B7 (Linsley et al., J. Exp. Med. 173 (1991),721-730); CTLA-4 (Lisley et al., J. Exp. Med. 174 (1991), 561-569); CD22 (Stamenkovic et al., Cell 66 (1991), 1133-1144); TNF receptor (Ashkenazi et al., Proc. Natl. Acad. Sci. USA 88 (1991), 10535-10539; Lesslauer et al., Eur. J. Immunol. 27 (1991), 2883-2886; and Peppel et al., J. Exp. Med. 174 (1991), 1483-1489 (1991); and IgE receptor a (Ridgway and Gorman, J. Cell. Biol. 115 (1991), Abstract No. 1448).

As discussed elsewhere herein, antibodies, or antigen-binding fragments, variants, or derivatives thereof described herein may be fused to heterologous polypeptides to increase the in vivo half-life of the polypeptides or for use in immunoassays using methods known in the art. For example, In some embodiments, PEG can be conjugated to the antibodies described herein to increase their half-life in vivo; see, e.g., Leong et al., Cytokine 16 (2001), 106-119; Adv. in Drug Deliv. Rev. 54 (2002), 531; or Weir et al., Biochem. Soc. Transactions 30 (2002), 512.

Moreover, antibodies, or antigen-binding fragments, variants, or derivatives thereof described herein can be fused to marker sequences, such as a peptide to facilitate their purification or detection. In particular embodiments, the marker amino acid sequence is a hexa-histidine peptide (HIS), such as the tag provided in a pQE vector (QIAGEN, Inc., 9259 Eton Avenue, Chatsworth, Calif., 91311), among others, many of which are commercially available. As described in Gentz et al., Proc. Natl. Acad. Sci. USA 86 (1989), 821-824, for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37 (1984), 767) and the "flag" tag.

Fusion proteins can be prepared using methods that are well known in the art; see for example US patent nos. 5,116,964 and 5,225,538. The precise site at which the fusion is made may be selected empirically to optimize the secretion or binding characteristics of the fusion protein. DNA encoding the fusion protein is then transfected into a host cell for expression.

Antibodies described herein may be used in non-conjugated form or may be conjugated to at least one of a variety of molecules, e.g., to improve the therapeutic properties of the molecule, to facilitate target detection, or for imaging or therapy of the patient. Antibodies, or antigen-binding fragments, variants, or derivatives thereof described herein can be labeled or conjugated either before or after purification, when purification is performed. In particular, antibodies, or antigen-binding fragments, variants, or derivatives thereof described herein may be conjugated to therapeutic agents, prodrugs, peptides, proteins, enzymes, viruses, lipids, biological response modifiers, pharmaceutical agents, or PEG.

Conjugates that are immunotoxins including conventional antibodies have been widely described in the art. The toxins may be coupled to the antibodies by conventional coupling techniques or immunotoxins containing protein toxin portions can be produced as fusion proteins. The antibodies described herein can be used in a corresponding way to obtain such immunotoxins. Illustrative of such immunotoxins are those described by Byers, Seminars Cell. Biol. 2 (1991), 59-70 and by Fanger, Immunol. Today 12 (1991), 51-54.

Those skilled in the art will appreciate that conjugates may also be assembled using a variety of techniques depending on the selected agent to be conjugated. For example, conjugates with biotin are prepared e.g. by reacting a BMPRI/BMPRII binding polypeptide with an activated ester of biotin such as the biotin N-hydroxysuccinimide ester. Similarly, conjugates with a fluorescent marker may be prepared in the presence of a coupling agent, e.g. those listed herein, or by reaction with an isothiocyanate, or fluorescein-isothiocyanate. Conjugates of the antibodies, or antigen-binding fragments, variants or derivatives thereof described herein are prepared in an analogous manner.

Also provided herein are antibodies, or antigen-binding fragments, variants, or derivatives thereof described herein conjugated to a diagnostic or therapeutic agent. The antibodies can be used diagnostically to, for example, demonstrate presence of a BMPRI/BMPRII-related disease, to indicate the risk of getting a BMPRI/BMPRII-related disease, to monitor the development or progression of a BMPRI/BMPRII-related disease, i.e. as part of a clinical testing procedure to, e.g., determine the efficacy of a given treatment and/or prevention regimen. Detection can be facilitated by coupling the antibody, or antigen-binding fragment, variant, or derivative thereof to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions; see, e.g., US patent no. 4,741,900 for metal ions which can be conjugated to antibodies for use as diagnostics. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include 125I, 1311, 111In or 99Tc.

An antibody, or antigen-binding fragment, variant, or derivative thereof also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

One of the ways in which an antibody, or antigen-binding fragment, variant, or derivative thereof can be detectably labeled is by linking the same to an enzyme and using the linked product in an enzyme immunoassay (EIA) (Voller, A., "The Enzyme Linked Immunosorbent Assay (ELISA)" Microbiological Associates Quarterly Publication, Walkersville, Md., Diagnostic Horizons 2 (1978), 1-7); Voller et al., J. Clin. Pathol. 31 (1978), 507-520; Butler, Meth. Enzymol. 73 (1981), 482-523; Maggio, E. (ed.), Enzyme Immunoassay, CRC Press, Boca Raton, Fla., (1980); Ishikawa, E. et al., (eds.), Enzyme Immunoassay, Kgaku Shoin, Tokyo (1981). The enzyme, which is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. Additionally, the detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibody, or antigen-binding fragment, variant, or derivative thereof, it is possible to detect the antibody through the use of a radioimmunoassay (RIA) (see, for example, Weintraub, B., Principles of Radioimmunoassays, Seventh Training Course on Radioligand Assay Techniques, The Endocrine Society, (March, 1986)), which is incorporated by reference herein). The radioactive isotope can be detected by means including, but not limited to, a gamma counter, a scintillation counter, or autoradiography.

An antibody, or antigen-binding fragment, variant, or derivative thereof can also be detectably labeled using fluorescence emitting metals such as 152Eu, or others of the lanthanide series. These metals can be attached to the antibody using such metal chelating groups as diethylenetriaminepentacetic acid (DTPA) or ethylenediaminetetraacetic acid (EDTA).

Techniques for conjugating various moieties to an antibody, or antigen-binding fragment, variant, or derivative thereof are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. (1985); Hellstrom et al., "Antibodies For Drug Delivery", in Controlled Drug Delivery (2nd Ed.), Robinson et al. (eds.), Marcel Dekker, Inc., pp. 623-53 (1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), Academic Press pp. 303-16 (1985), and Thorpe et al., "The Preparation And Cytotoxic Properties Of Antibody-Toxin Conjugates", Immunol. Rev. 62 (1982), 119-158.

As mentioned, in some embodiments, a moiety that enhances the stability or efficacy of a binding molecule, e.g., a binding polypeptide, e.g., an antibody or immunospecific fragment thereof can be conjugated. For example, In some embodiments, PEG can be conjugated to the binding molecules described herein to increase their half-life in vivo. Leong et al., Cytokine 16 (2001), 106; Adv. in Drug Deliv. Rev. 54 (2002), 531; or Weir et al., Biochem. Soc. Transactions 30 (2002), 512.

### Bispecific and Multi-Specific Antibodies

The bispecific and multi-specific antibody reagents described herein can serve as surrogate ligands to assemble complexes of type I and type II receptors of this pathway. These reagents initiate the assembly of type I and type II receptors to phosphorylate downstream SMAD proteins, and modulate signaling in a similar manner to that of an endogenous ligand. In contrast to endogenous ligands, most of which can pair with a multitude of distinct type I and type II ligands, due to overlapping specificity of the receptors, the bispecific and multi-specific reagents of this application target specific pairs of type I and type II receptors. By targeting specific pairs of receptors, these reagents can elicit specific functions downstream of those receptor pairs, without engaging other receptor pairings normally engaged by endogenous ligands. This selective targeting of receptors can recruit the activity engendered by activation of a specific pair of receptors, while avoiding the activation of other receptor pairs that could otherwise be activated by a given endogenous ligand. This selective targeting of receptors could enrich desirable activities of a given TGF-beta superfamily ligand while eliminating undesirable effects, for therapeutic purposes.

The bispecific antibodies with specificity for various combinations of surface BMP/TGF-beta receptors as described herein can be used, e.g., therapeutically, in a wide range of contexts. Multi-specific or bi-specific antibodies recognizing type I and type II receptors of the TGF-beta superfamily can elicit specific BMP or TGF-beta pathway-specific activation of SMAD effectors, gene transcription, and cell biology and fate, similar to endogneous BMP, activin, GDF, and TGF-beta ligands.

Signaling bi-specific antibodies do not have the promiscuity of native ligands, providing a method of eliciting specific cellular effects in specific target tissues despite systemic adminstration. Instead of binding to multiple type II and multiple type I receptors, which is often the case with native ligands, bi-specifics target a single type II and type I pair. This would restrict the biological effects of signaling and the targeted tissues. Unlike native ligands, which have half-lives in circulation measuring several minutes to hours, bispecific signaling molecules have pharmacokinetics that can be adjusted depending on the desired biological effect. Bispecific IgG Fc molecules are predicted to have a half-life of several weeks, as is generally the case for therapeutic IgG molecules or other IgG Fc fusion molecules. But could also be designed to have shorter half-lives on the order of minutes or hours, if expressed as bispecific molecules without an Fc, i.e., F(ab')2 fragments, or bispecific single-chain Fv (scFv) molecules. Bispecific IgG Fc can be directed towards specific effects based on ADCC or complement fixing functions depending on the use of native IgG Fc domains, or mutant IgG Fc domains.

Unlike native ligands, which have heparan sulfate binding domains that cause them to be bound to extracellular matrix glycosoaminoglycans, and thus sequestered from circulation or from target cells, engineered bispecific signaling molecules can be administered systemically to target diverse tissues for more favorable distribution and pharmacodynamics.

Mutated protein ligands of the BMP/TGF-beta signaling pathway, a.k.a., muteins, can be designed to have more selective receptor targeting, tissue targeting, or pharmacokinetics and pharmacodynamics. Whereas muteins of the BMP/TGF-beta signaling pathway could be generated to engage some of their functions in a selective manner, these muteins will likely be immunogenic, and the anti-drug antibody response could not only neutralize the effects of those therapeutic reagents, diminishing or abrogating their effect with repeated use or exposure, but could also cause a pathogenic auto-immune response against endogenous ligand, inhibiting its essential functions and causing permanent impairment of endogenous physiologic functions.

Whereas engineered multi-specific and bi-specific antibody reagents might engender anti-drug antibody responses, similar to other therapeutic antibodies these responses, and their corresponding effects can be minimized by sequence optimization to match endogenous antibody idiotypes and peptide sequences, as well as by optimizing protein expression and protein folding, formulation and delivery systems. We expect that similar to other therapeutic antibodies, the development of an anti-drug antibody response, if present, should in many causes does not disrupt therapeutic efficacy.

Many endogenous ligands of this pathway are naturally bound by extracellular matrix proteins via heparan sulfate binding domains, or by other extracellular matrix proteins which serve to sequester these proteins at potential sites of activity pending activation by biochemical changes in those tissues which regulate activity. These interactions can also limit the ability of administered endogenous ligands to act upon target cells. As such, these proteins may not be suitable for systemic administration. Bispecific or multispecific antibody reagents targeting this pathway could be administered systemically, and could diffuse into diverse tissue compartments for therapeutic effect. Engineered bispecific signaling molecules may be administered systemically to target diverse tissues for more favorable distribution and pharmacodynamics. Bispecific or multispecific antibody reagents targeting this pathway could alternatively be targeting with homing domains to concentrate their effects in specific tissue compartments.

The term "antibody" as used herein refers to an immunoglobulin molecule or immunologically active portion thereof, i.e., an antigen-binding portion. Examples of immunologically active portions of immunoglobulin molecules include F(ab) and F(ab')₂ fragments, which retain the ability to bind antigen. Such fragments can be obtained commercially, or using methods known in the art. For example F(ab)2 fragments can be generated by treating the antibody with an enzyme such as pepsin, a non-specific endopeptidase that normally produces one F(ab)2 fragment and numerous small peptides of the Fc portion. The resulting F(ab)2 fragment is composed of two disulfide-connected Fab units. The Fc fragment is extensively degraded and can be separated from the F(ab)2 by dialysis, gel filtration or ion exchange chromatography. F(ab) fragments can be generated using papain, a non-specific thiol-endopeptidase that digests IgG molecules, in the presence of a reducing agent, into three fragments of similar size: two Fab fragments and one Fc fragment. When Fc fragments are of interest, papain is the enzyme of choice because it yields a 50,00 Dalton Fc fragment; to isolate the F(ab) fragments, the Fc fragments can be removed, e.g., by affinity purification using protein A/G. A number of kits are available commercially for generating F(ab) fragments. In addition, commercially available services for generating antigen-binding fragments can be used, e.g., Bio Express, West Lebanon, NH. The antibodies are at least bispecific (i.e., recognizing two different epitopes either on the same or on different antigens; see, e.g., Brinkmann and Kontermann, MAbs. 2017 Feb-Mar; 9(2): 182-212) or multispecific (i.e., exhibiting recogniszing more than 2 epitopes; see, e.g., Egan et al., MAbs. 2017 Jan; 9(1): 68-84).

The antibody can be de-immunized or humanized, fully human, non-human, e.g., murine, or single chain antibody. In some embodiments, the antibody has effector function and can fix complement. In some embodiments, the antibody has reduced or no ability to bind an Fc receptor. For example, the antibody can be an isotype or subtype, fragment or other mutant, that does not support binding to an Fc receptor, e.g., it has a mutagenized or deleted Fc receptor binding region.

In preferred embodiments, the antibody is a single chain antibody. A single-chain antibody (scFV) can be engineered (see, for example, Colcher et al., Ann. N. Y Acad. Sci. 880:263-80 (1999); and Reiter, Clin. Cancer Res. 2:245-52 (1996); Ahmad et al., Clin Dev Immunol. 2012;2012:980250; Brinkmann and Kontermann, MAbs. 2017 Feb-Mar; 9(2): 182-212). The single chain antibody can be dimerized or multimerized to generate multivalent antibodies having specificities for different epitopes of the same target protein.

In some embodiments, the bispecific and multi-specific reagents described herein comprise two or more different scFV, each of which binds to a different protein target; each scFv comprises variable regions of heavy (VH) and light (VL) chains joined together by an intrabody flexible peptide linker. This intrabody linker should be long enough to allow the VH and VL chains to fold and interact properly, and include hydrophilic domains. In some embodiments, the intrabody linkers comprise stretches of Gly and Ser residues, optionally with charged Glu and Lys interspersed to enhance solubility. See, e.g., Ahmad et al., Clin Dev Immunol. 2012;2012:980250.

The scFV are then linked together with flexible interbody linkers, e.g., of 5-50, 15-40, or 20-30 amino acids (e.g., at least 5, 10, 15, or 20 amino acids, up to 25, 35, 40 or 50 amino acids, and all ranges with the foregoing numbers as endpoints). The interbody linkers can include engineered linkers, e.g., short alanine linkers (Ala3), hydrophilic linkers, glycine-serine-rich linkers, helical linkers, and native linkers derived from various immunoglobulin and non-immunoglobulin molecules. Brinkmann and Kontermann, MAbs. 2017 Feb-Mar; 9(2): 182-212. In some embodiments, 20 residue (G₄S)₄ linkers or one or more instances of GGGGSG (SEQ ID NO: 103); GGGGSGGGS (SEQ ID NO: 104); GSGGGGDGGGGSG (SEQ ID NO: 194); GGGGSGGGGSGDGSS (SEQ ID NO: 195), EGKSSGSGSDSKAS (SEQ ID NO: 105) or EGKSSGSGSESKAS (SEQ ID NO: 106); SGGGGSGGGGSSGSGGGGDGGGGSG (SEQ ID NO: 192); SGGGGSGGGGSSGSGGGGDGGGGSGGT (SEQ ID NO: 107); SGGSGGGGSSGGGGSGGGGSSGGGGDGGGGSG (SEQ ID NO: 193); GSGGGGDSGGGGSGGGGSSGGGGSG (SEQ ID NO: 195); GSGGGGDSGGGGSGGSGGSGGSGGGSGGGGSG (SEQ ID NO: 196); or SGGSGGGGSSGGGGSGGGGSSGGGGDGGGGSGGT (SEQ ID NO: 108) are used. In some embodiments, one of the linkers also includes one or more, e.g., two, four, six or more, Histidine residues, e.g., GGHHHHHHHHGG (SEQ ID NO: 198); SGGGGSHHHHHHHHSGGGGS (SEQ ID NO: 199); or SGGGGSGGHHHHHHHHGGSGGGGS (SEQ ID NO:200). The bispecific scFV can be arranged VH-VL or VL-VH; higher-order multiples can likewise be arranged in any order.

Methods known in the art can be used to generate antigen-binding domains for each of the type I and type II receptors. For example, the process can include obtaining antibody-secreting immune cells (lymphocytes) from the spleen of a mammal (e.g., mouse) that has been previously immunized with the antigen of interest (e.g., all or part of a type I or II receptor) either in vivo or in vitro. The antibody-secreting lymphocytes are then fused with myeloma cells or transformed cells that are capable of replicating indefinitely in cell culture, thereby producing an immortal, immunoglobulin-secreting cell line. The resulting fused cells, or hybridomas, are cultured, and the resulting colonies screened for the production of the desired monoclonal antibodies. Colonies producing such antibodies are cloned, and grown either in vivo or in vitro to produce large quantities of antibody. A description of the theoretical basis and practical methodology of fusing such cells is set forth in Kohler and Milstein, Nature 256:495 (1975), which is hereby incorporated by reference. The sequences of the antigen-binding regions of the clones can be determined using known methods.

Exemplary sequences for human type I or II receptor proteins are shown in Table A.

**Table A - Type I and II BMPRs**

| Receptor | Type | NCBI GenBank RefSeq Acc No. |
|---|---|---|
| Activin A receptor like type 1, ALK1, ACVRL1 | I | NP_000011.2 |
| Activin A receptor type 1, ALK2, ACVR1 | I | NP_001096.1 |
| Bone morphogenetic protein receptor type 1A, ALK3, BMPR1A | I | NP_004320.2 |
| Activin A receptor type 1B, ALK4, ACVR1B | I | NP_004293.1 isoform a |
| | | NP_064732.3 isoform b |
| | | NP 064733.3 isoform c |
| Transforming growth factor beta receptor 1, ALK5, TGFBR1 | I | NP_004603.1 isoform 1 |
| | | NP_001124388.1 isoform 2 |
| | | NP 001293139.1 isoform 3 |
| Bone morphogenetic protein receptor type 1B, ALK6, BMPR1B | I | NP_001243722.1 isoform a |
| | | NP_001194.1 isoform b |
| Activin A receptor type 1C, ALK7, ACVR1C | I | NP_660302.2 isoform 1 |
| | | NP_001104501.1 Isoform 2 |
| | | NP_001104502.1 Isoform 3 |
| | | NP 001104503.1 Isoform 4 |
| Activin A receptor type 2A, ACTRIIA, ACVR2 | II | NP_001607.1 isoform 1 |
| | | NP_001265508.1 isoform 2 |
| Activin A receptor type 2B, ACTRIIB, ACVR2B | II | NP_001097.2 |
| Bone morphogenetic protein receptor type 2, BMPR2, BMPR2 | II | NP_001195.2 |
| Transforming growth factor beta receptor 2, TGFBR2, TGFBR2 | II | NP001020018.1 isoform a |
| | | _ NP_003233.4isoform b |
| Anti-Mullerian hormone receptor type 2, AMHR2, AMHR2 | II | NP_065434.1 isoform 1 |
| | | NP_001158162.1 isoform 2 |
| | | NP 001158163.1 isoform 3 |

Exemplary sequences for CDRs and scFV are provided herein, e.g., in Table 1. Sequences used in the present antibodies can be at least 80%, 85%, 90%, 95%, 97%, or 99% identical to a sequence provided herein, so long as they retain the ability to bind the target and the agonistic activity of the reference antibody, e.g., at least 50%, 60%, 70%, 75%, 80%, 85%, 90%, or 95% of the level of activity of the reference antibody. Calculations of "identity" between two sequences can be performed as follows. The sequences are aligned for optimal comparison purposes (e.g., gaps can be introduced in one or both of a first and a second nucleic acid sequence for optimal alignment and non-identical sequences can be disregarded for comparison purposes). The length of a sequence aligned for comparison purposes is at least 70% (e.g., at least 80%, 90% or 100%) of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In some embodiments, the percent identity between two nucleotide sequences is determined using the Needleman and Wunsch ((1970) J. Mol. Biol. 48:444-453 ) algorithm, which has been incorporated into the GAP program in the GCG software package (available at gcg.com), using either a Blossum 62 matrix, a PAM250 matrix, a NWSgapdna.CMP matrix. In some embodiments, the percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

In some embodiments, the bispecific and multi-specific reagents described herein can also include, or do not include, one or more antigen-binding domains from known anti-type I or anti-type II receptor antibodies, e.g., as provided in Tables B or C.

**Table B - Exemplary anti-type I or anti-type II receptor antibody sequences**

| **Pub. No/Journal** | **Authors/Inventors/Applicant** | **Target** | **Date** |
|---|---|---|---|
| Nature Structural Biology, 7(6):492-496 | Kirsch T, Sebald W, and Dreyer MK | Anti-BMPRIA | 2000 |
| US 2013/0089560 | Chartier-Courtaud BC and Gurney AL | Morphogenetic Protein Receptor binding agents and methods of their use | 2013 |
| WO 2018/183376 | Rigel Pharmaceuticals, Inc. | ACVR2A-Specific Antibody and method of use thereof | 2018 |
| US 2013/0344067 | Acceleron Pharma, Inc. | ACVRL1 Receptor and Ligand Antagonists and uses thereof | 2013 |
| US 7,537,762 | Amgen Fremont Inc., Pfizer Inc. | Human Monoclonal Antibodies to Activin Receptor-Like Kinase-1 | 2009 |
| US 10,428,148 | Saitama Medical University, Daiichi Sankyo Company | Anti-ACVR1 | 2019 |
| US 2016/0075772 | Regeneron Pharmaceuticals | Treatment of Fibrodysplasia Ossificans Progressiva (Anti-ACVR1) | 2016 |
| US 2015/0125471 | INSERM, Universite Paul Sabater Toulose III, Centre National De La Recherche Scientifi ue | Method and Pharmaceutical Composition for use in the Treatment and Diagnosis of Anemia pf Inflammation | 2015 |
| US 9,611,321 | Fox Chase Cancer Center | Rationally-Designed Anti-Mulleran Inhibiting Substance Type Ii Receptor Antibodies | 2017 |
| WO 2019/086331 | BAYER AKTIENGESELLSCHAFT | Bispecific Antibodies Binding ACVRL1 and BMPR2 | 2019 |
| US 9,969,806 | Novartis AG | Administration of BIMAGRUMAB for improving muscle mass and function after hip fracture surgery (Anti-ACVR2A/B) | 2018 |
| WO 2011/056497 | Genentech Inc. | ACTIVIN RECEPTOR TYPE IIB Compositions And Methods Of Use | 2010 |
| US 10,260,068 | Sumitomo Dainippon Pharma Co. Lt., Kyoto University | Prophylactic Agent and Therapeutic Agent for Fibrodysplasia Ossificans Progressiva (Anti-ACVR1) | 2019 |
| US 9,493,556 | Acceleron Pharma Inc. | ACVR2A Binding Agents and uses thereof | 2016 |
| WO 95/30003 | Creative Biomolecules Inc.,The Ludwig Institute for Cancer Research | Morphogenic Protein-Specific Cell Surface Receptors and uses | 1995 |
| | | therefor (Type I and II receptor Targets) | |
| US 2011/0182904 | Zimmerman D, Selby M, Srinivasan M, Bell M, Singh S, Leblanc HN, Zens KD, Sproul TW | Antibodies to Bone Morphogenic Proteins and Receptors therefor and methods for their use | 2011 |
| EP 3345921 | Knopf J, Seehra J, Kumar R | Anti-ACTRIIB antibodies | 2018 |
| US 9,365,65 | Feige J, Glass D, Hatakeyama S, Richardson BP, Trifilieff E | Anti-ACTRIIB antibodies | 2016 |
| US 2017/0306027 | Knopf J, Kumar R, Grinberg A, Pearsall RS, Sako DS, Castonguay R, Li G, Dagon Y | Anti-ALK7 antibodies | 2017 |

**Table C - Sequences from WO2019086331**

| **CDR** | **SEQUENCE** | **SEQ ID NO:** |
|---|---|---|
| **1st Bispecific Antibody Construct (TPP-14696)** | | |
| Chain 1 (TPP-13654/Anti-ACVRL1) | | |
| CDR H1 | IYAMS | 184. |
| CDR H2 | AISGSGGSTYYADSVKG | 185. |
| CDR H3 | DFDY | 186. |
| CDR L1 | SGSSSNIGSNYVY | 187. |
| CDR L2 | GNINRPS | 188. |
| CDR L3 | AAWDDSLNGRV | 189. |
| | | |

| Chain 2 (TPP-13667/Anti-BMPR2) | | |
|---|---|---|
| CDR H1 | NAWMN | 190. |
| CDR H2 | SISSSSSYIYYADSVKG | 191. |
| CDR H3 | AVAAGGMFWGLDQ | 192. |
| CDR L1 | SGSRSNIGSNSVH | 193. |
| CDR L2 | GNSNRPS | 194. |
| CDR L3 | QSYDSSLNDHVV | 195. |

| **CDR** | **SEQUENCE** | **SEQ ID NO:** |
|---|---|---|
| **2nd Bispecific Antibody Construct (TPP-14719)** | | |
| Chain 1 (TPP-13660/Anti-ACVRL1) | | |
| CDR H1 | SYAMS | 196. |
| CDR H2 | NINQDGSEKNYVDSMRG | 197. |
| CDR H3 | EFDY | 198. |
| CDR L1 | SGSSSNIGSNYVY | 199. |
| CDR L2 | GNNKRPS | 200. |
| CDR L3 | AAWDDSLNGRV | 201. |
| | | |

| Chain 2 (TPP-13469/Anti-BMPR2) | | |
|---|---|---|
| CDR H1 | DYYMT | 202. |
| CDR H2 | SISGGSTYYADSRKG | 203. |
| CDR H3 | DFGVAGWFGQYGMDV | 204. |
| CDR L1 | TGSSSNIGAGYDVH | 205. |
| CDR L2 | RSNQRPS | 206. |
| CDR L3 | SSYAGNYNLV | 207. |
| | | |

| **Individual Anti-ACVRL1 Antibody Clones** | | |
|---|---|---|
| TPP-13660 | | |
| CDR H1 | SYAMS | 208. |
| CDR H2 | NINQDGSEKNYVDSMR | 209. |
| CDR H3 | EFDY | 210. |
| CDR L1 | SGSSSNIGSNYVY | 211. |
| CDR L2 | GNNKRPS | 212. |
| CDR L3 | AAWDDSLNGRV | 213. |

| **CDR** | **SEQUENCE** | **SEQ ID NO:** |
|---|---|---|
| TPP-13654 | | |
| CDR H1 | IYAMS | 214. |
| CDR H2 | AISGSGGSTYYADSVKG | 215. |
| CDR H3 | DFDY | 216. |
| CDR L1 | SGSSSNIGSNYVY | 217. |
| CDR L2 | GNINRPS | 218. |
| CDR L3 | AAWDDSLNGRV | 219. |
| | | |

| **Individual anti-BMPR2 Antibody Clones** | | |
|---|---|---|
| TPP-13667 | | |
| CDR H1 | NAWMN | 220. |
| CDR H2 | SISSSSSYIYYADSVKG | 221. |
| CDR H3 | AVAAGGMFWGLDQ | 222. |
| CDR L1 | SGSRSNIGSNSVH | 223. |
| CDR L2 | GNSNRPS | 224. |
| CDR L3 | QSYDSSLNDHVV | 225. |
| | | |
| TPP-13469 | | |
| CDR H1 | DYYMT | 226. |
| CDR H2 | SISGGSTYYADSRKG | 227. |
| CDR H3 | DFGVAGWFGQYGMDV | 228. |
| CDR L1 | TGSSSNIGAGYDVH | 229. |
| CDR L2 | RSNQRPS | 230. |
| CDR L3 | SSYAGNYNLV | 231. |

The bispecific and multi-specific antibodies can be expressed, e.g., from nucleic acids comprising sequences encoding the antibodies, e.g., in genetically engineered cells or animals, and then purified. Thus also provided herein are nucleic acids encoding the antibodies as well as vectors, preferably expression vectors, containing a nucleic acid encoding an antibody as described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a nucleic acid encoding an antibody as described herein in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein encoding an antibody as described herein.

The recombinant expression vectors can be designed for expression of an antibody as described herein in prokaryotic or eukaryotic cells. For example, polypeptides of the invention can be expressed in *E*. *coli,* insect cells (e.g., using baculovirus expression vectors), yeast cells or mammalian cells. Suitable host cells are discussed further in Goeddel, (1990) Gene Expression Technology: Methods in Enzymology 185, Academic Press, San Diego, CA. Alternatively, the recombinant expression vector can be transcribed and translated in vitro, for example using T7 promoter regulatory sequences and T7 polymerase. Methods known in the art can be used to purify the antibody as described herein after expression or translation, for use in a method or composition described herein.

Alternatively, a nucleic acid encoding an antibody as described herein, or a vector (e.g., a viral vector) for expression of the nucleic acid, can be delivered to a subject in need thereof.

### V. Expression of Antibody Polypeptides

Following manipulation of the isolated genetic material to provide antibodies, or antigen-binding fragments, variants, or derivatives thereof described herein, the polynucleotides encoding the antibodies are typically inserted in an expression vector for introduction into host cells that may be used to produce the desired quantity of antibody. Recombinant expression of an antibody, or fragment, derivative or analog thereof, e.g., a heavy or light chain of an antibody which binds to a target molecule is described herein. Once a polynucleotide encoding an antibody molecule or a heavy or light chain of an antibody, or portion thereof (preferably containing the heavy or light chain variable domain), described herein has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. Thus, provided herein are replicable vectors comprising a nucleotide sequence encoding an antibody molecule, or antigen-binding fragment thereof, described herein, or a heavy or light chain thereof, or a heavy or light chain variable domain, operably linked to a promoter. Such vectors may include the nucleotide sequence encoding the constant region of the antibody molecule (see, e.g., international applications WO 86/05807 and WO 89/01036; and US patent no. 5,122,464) and the variable domain of the antibody may be cloned into such a vector for expression of the entire heavy or light chain.

The term "vector" or "expression vector" is used herein to mean vectors used in accordance methods described herein as a vehicle for introducing into and expressing a desired gene in a host cell. As known to those skilled in the art, such vectors may easily be selected from the group consisting of plasmids, phages, viruses and retroviruses. In general, vectors described herein will comprise a selection marker, appropriate restriction sites to facilitate cloning of the desired gene and the ability to enter and/or replicate in eukaryotic or prokaryotic cells. Numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Others involve the use of polycistronic systems with internal ribosome binding sites. Additionally, cells which have integrated the DNA into their chromosomes may be selected by introducing one or more markers which allow selection of transfected host cells. The marker may provide for prototrophy to an auxotrophic host, biocide resistance (e.g., antibiotics) or resistance to heavy metals such as copper. The selectable marker gene can either be directly linked to the DNA sequences to be expressed, or introduced into the same cell by co-transformation. Additional elements may also be needed for optimal synthesis of mRNA. These elements may include signal sequences, splice signals, as well as transcriptional promoters, enhancers, and termination signals.

In particular embodiments, the cloned variable region genes are inserted into an expression vector along with the heavy and light chain constant region genes (e.g., human heavy and light chain constant region genes) as discussed above. Any expression vector which is capable of eliciting expression in eukaryotic cells may be used. Examples of suitable vectors include, but are not limited to plasmids pcDNA3, pHCMV/Zeo, pCR3.1, pEF1/His, pIND/GS, pRc/HCMV2, pSV40/Zeo2, pTRACER-HCMV, pUB6/V5-His, pVAX1, and pZeoSV2 (available from Invitrogen, San Diego, CA), and plasmid pCI (available from Promega, Madison, WI). In general, screening large numbers of transformed cells for those which express suitably high levels if immunoglobulin heavy and light chains is routine experimentation which can be carried out, for example, by robotic systems. Vector systems are also taught in US patent nos. 5,736,137 and 5,658,570, each of which is incorporated by reference in its entirety herein. This system provides for high expression levels, e.g., > 30 pg/cell/day. Other exemplary vector systems are disclosed e.g., in US patent no. 6,413,777.

In other embodiments the antibodies, or antigen-binding fragments, variants, or derivatives thereof described herein may be expressed using polycistronic constructs such as those disclosed in US patent application publication no. 2003-0157641 A1 and incorporated herein in its entirety. In these expression systems, multiple gene products of interest such as heavy and light chains of antibodies may be produced from a single polycistronic construct. These systems advantageously use an internal ribosome entry site (IRES) to provide relatively high levels of antibodies. Compatible IRES sequences are disclosed in US patent no. 6,193,980 which is also incorporated herein. Those skilled in the art will appreciate that such expression systems may be used to effectively produce the full range of antibodies disclosed in the instant application.

More generally, once the vector or DNA sequence encoding a monomeric subunit of the antibody has been prepared, the expression vector may be introduced into an appropriate host cell. Introduction of the plasmid into the host cell can be accomplished by various techniques well known to those of skill in the art. These include, but are not limited to, transfection including lipotransfection using, e.g., Fugene^{®} or lipofectamine, protoplast fusion, calcium phosphate precipitation, cell fusion with enveloped DNA, microinjection, and infection with intact virus. Typically, plasmid introduction into the host is via standard calcium phosphate co-precipitation method. The host cells harboring the expression construct are grown under conditions appropriate to the production of the light chains and heavy chains, and assayed for heavy and/or light chain protein synthesis. Exemplary assay techniques include enzyme-linked immunosorbent assay (ELISA), radioimmunoassay (RIA), or fluorescence-activated cell sorter analysis (FACS), immunohistochemistry and the like.

The expression vector is transferred to a host cell by conventional techniques and the transfected cells are then cultured by conventional techniques to produce an antibody for use in the methods described herein. Thus, also provided herein are host cells containing a polynucleotide encoding an antibody or antigen binding fragment thereof described herein, or a heavy or light chain thereof, operably linked to a heterologous promoter. In particular embodiments, for the expression of double-chained antibodies, vectors encoding both the heavy and light chains may be co-expressed in the host cell for expression of the entire immunoglobulin molecule, as detailed below.

The host cell may be co-transfected with two expression vectors described herein, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain; see Proudfoot, Nature 322 (1986), 52; Kohler, Proc. Natl. Acad. Sci. USA 77 (1980), 2197. The coding sequences for the heavy and light chains may comprise cDNA or genomic DNA.

As used herein, "host cells" refers to cells which harbor vectors constructed using recombinant DNA techniques and encoding at least one heterologous gene. In descriptions of processes for isolation of antibodies from recombinant hosts, the terms "cell" and "cell culture" are used interchangeably to denote the source of antibody unless it is clearly specified otherwise. In other words, recovery of polypeptide from the "cells" may mean either from spun down whole cells, or from the cell culture containing both the medium and the suspended cells.

A variety of host-expression vector systems may be utilized to express antibody molecules for use in the methods described herein. Such host-expression systems represent vehicles by which the coding sequences of interest may be produced and subsequently purified, but also represent cells which may, when transformed or transfected with the appropriate nucleotide coding sequences, express an antibody molecule described herein in situ. These include but are not limited to microorganisms such as bacteria (e.g., *E. coli, B. subtilis*) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, NSO, BLK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). In some embodiments, bacterial cells such as Escherichia coli, and more preferably, eukaryotic cells, especially for the expression of whole recombinant antibody molecule, are used for the expression of a recombinant antibody molecule. For example, mammalian cells such as Chinese Hamster Ovary (CHO) cells, in conjunction with a vector such as the major intermediate early gene promoter element from human cytomegalovirus is an effective expression system for antibodies; see, e.g., Foecking et al., Gene 45 (1986), 101; Cockett et al., Bio/Technology 8 (1990), 2.

The host cell line used for protein expression is often of mammalian origin; those skilled in the art are credited with ability to determine particular host cell lines which are best suited for the desired gene product to be expressed therein. Exemplary host cell lines include, but are not limited to, CHO (Chinese Hamster Ovary), DG44 and DUXB11 (Chinese Hamster Ovary lines, DHFR minus), HELA (human cervical carcinoma), CVI (monkey kidney line), COS (a derivative of CVI with SV40 T antigen), VERY, BHK (baby hamster kidney), MDCK, WI38, R1610 (Chinese hamster fibroblast) BALBC/3T3 (mouse fibroblast), HAK (hamster kidney line), SP2/O (mouse myeloma), P3x63-Ag3.653 (mouse myeloma), BFA-1c1BPT (bovine endothelial cells), RAJI (human lymphocyte) and 293 (human kidney). In a specific embodiment, host cell lines are CHO or 293 cells. Host cell lines are typically available from commercial services, the American Tissue Culture Collection or from published literature.

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product may be used.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which stably express the antibody molecule.

A number of selection systems may be used, including but not limited to the herpes simplex virus thymidine kinase (Wigler et al., Cell 11 (1977), 223), hypoxanthine-guanine phosphoribosyltransferase (Szybalska & Szybalski, Proc. Natl. Acad. Sci. USA 48 (1992), 202), and adenine phosphoribosyltransferase (Lowy et al., Cell 22 (1980), 817) genes can be employed in tk-, hgprt- or aprt-cells, respectively. Also, antimetabolite resistance can be used as the basis of selection for the following genes: dhfr, which confers resistance to methotrexate (Wigler et al., Natl. Acad. Sci. USA 77 (1980), 357; O'Hare et al., Proc. Natl. Acad. Sci. USA 78 (1981), 1527); gpt, which confers resistance to mycophenolic acid (Mulligan & Berg, Proc. Natl. Acad. Sci. USA 78 (1981), 2072); neo, which confers resistance to the aminoglycoside G-418 Goldspiel et al., Clinical Pharmacy 12 (1993), 488-505; Wu and Wu, Biotherapy 3 (1991), 87-95; Tolstoshev, Ann. Rev. Pharmacol. Toxicol. 32 (1993), 573-596; Mulligan, Science 260 (1993), 926-932; and Morgan and Anderson, Ann. Rev. Biochem. 62 (1993), 191-217; TIB TECH 11 (1993), 155-215; and hygro, which confers resistance to hygromycin (Santerre et al., Gene 30 (1984), 147. Methods commonly known in the art of recombinant DNA technology which can be used are described in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981), which are incorporated by reference herein in their entireties.

The expression levels of an antibody molecule can be increased by vector amplification, for a review, see Bebbington and Hentschel, The use of vectors based on gene amplification for the expression of cloned genes in mammalian cells in DNA cloning, Academic Press, New York, Vol. 3. (1987). When a marker in the vector system expressing antibody is amplifiable, increase in the level of inhibitor present in culture of host cell will increase the number of copies of the marker gene. Since the amplified region is associated with the antibody gene, production of the antibody will also increase; see Crouse et al., Mol. Cell. Biol. 3 (1983), 257.

In vitro production allows scale-up to give large amounts of the desired polypeptides. Techniques for mammalian cell cultivation under tissue culture conditions are known in the art and include homogeneous suspension culture, e.g. in an airlift reactor or in a continuous stirrer reactor, or immobilized or entrapped cell culture, e.g. in hollow fibers, microcapsules, on agarose microbeads or ceramic cartridges. If necessary and/or desired, the solutions of polypeptides can be purified by the customary chromatography methods, for example gel filtration, ion-exchange chromatography, chromatography over DEAE-cellulose or (immuno-)affinity chromatography, e.g., after preferential biosynthesis of a synthetic hinge region polypeptide or prior to or subsequent to the HIC chromatography step described herein.

Genes encoding antibodies, or antigen-binding fragments, variants, or derivatives thereof described herein can also be expressed in non-mammalian cells such as bacteria or insect or yeast or plant cells. Bacteria which readily take up nucleic acids include members of the enterobacteriaceae, such as strains of Escherichia coli or Salmonella; Bacillaceae, such as Bacillus subtilis; Pneumococcus; Streptococcus, and Haemophilus influenzae. It will further be appreciated that, when expressed in bacteria, the heterologous polypeptides typically become part of inclusion bodies. The heterologous polypeptides must be isolated, purified and then assembled into functional molecules. Where tetravalent forms of antibodies are desired, the subunits will then self-assemble into tetravalent antibodies; see, e.g., international application WO02/096948.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., EMBO J. 2 (1983), 1791), in which the antibody coding sequence may be ligated individually into the vector in frame with the lacZ coding region so that a fusion protein is produced; pIN vectors (Inouye & Inouye, Nucleic Acids Res. 13 (1985), 3101-3109; Van Heeke & Schuster, J. Biol. Chem. 24 (1989), 5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix of glutathione-agarose beads followed by elution in the presence of free glutathione. The pGEX vectors are designed to include thrombin or factor Xa protease cleavage sites so that the cloned target gene product can be released from the GST moiety.

In addition to prokaryotes, eukaryotic microbes may also be used. Saccharomyces cerevisiae, or common baker's yeast, is the most commonly used among eukaryotic microorganisms although a number of other strains are commonly available, e.g., Pichia pastoris. For expression in Saccharomyces, the plasmid YRp7, for example, (Stinchcomb et al., Nature 282 (1979), 39; Kingsman et al., Gene 7 (1979), 141; Tschemper et al., Gene 10 (1980), 157) is commonly used. This plasmid already contains the TRP1 gene which provides a selection marker for a mutant strain of yeast lacking the ability to grow in tryptophan, for example ATCC No. 44076 or PEP4-1 (Jones, Genetics 85 (1977), 12). The presence of the trpl lesion as a characteristic of the yeast host cell genome then provides an effective environment for detecting transformation by growth in the absence of tryptophan.

In an insect system, Autographa californica nuclear polyhedrosis virus (AcNPV) is typically used as a vector to express foreign genes. The virus grows in Spodoptera frugiperda cells. The antibody coding sequence may be cloned individually into non-essential regions (for example the polyhedrin gene) of the virus and placed under control of an AcNPV promoter (for example the polyhedrin promoter).

Once an antibody described herein has been recombinantly expressed, the whole antibodies, their dimers, individual light and heavy chains, or other immunoglobulin forms described herein, can be purified according to standard procedures of the art, including for example, by chromatography (e.g., ion exchange, affinity, particularly by affinity for the specific antigen after Protein A, and sizing column chromatography), centrifugation, differential solubility, e.g. ammonium sulfate precipitation, or by any other standard technique for the purification of proteins; see, e.g., Scopes, "Protein Purification", Springer Verlag, N.Y. (1982). Alternatively, another method for increasing the affinity of antibodies described herein is disclosed in US patent publication 2002-0123057 A1.

### VI. Methods of Use

The present compositions and methods can be used in a variety of ways, including for research as well as therapeutically.

For example, heterotopic ossification (HO), the formation of ectopic endochondral bone in skeletal muscle and soft tissues, is a significant cause of morbidity from joint immobility and pain. The precise mechanisms responsible for HO are not known; however, its association with trauma, inflammation and biomechanical stress suggests a process of disordered injury repair and homeostasis. We have explored the underlying mechanisms of a monogenic cause of HO, fibrodysplasia ossificans progressiva (FOP), caused by activating mutations of the bone morphogenetic protein (BMP) type I receptor ALK2, whereas trauma-induced HO appears to be regulated by ALK2, ALK3 and potentially ALK6. FOP and acquired forms of HO share a common mechanism of inappropriate BMP signaling, but the manner by which BMP signals are interpreted to regulate ossification versus tissue regeneration remain incompletely understood.

BMP9 is a multifunctional ligand that can elicit vascular endothelial quiescence and is protective against pulmonary vascular disease in animal models of pulmonary hypertension. However, in some contexts treatment of animals with BMP9 can lead to heterotopic ossification, or undesired formation of ectopic bone in soft tissues at the sites of injection. BMP9 engages type II receptors BMPRII, ACTRIIA and ACTRIIB. and engages type I receptors ALK1 and ALK2. It is proposed that by engaging BMPR2 and ALK1, a bispecific antibody reagent could elicit vascular endothelial quiescence effects of BMP9, attributed to the activation of BMPR2 and ALK1, without causing heterotopic ossification, attributed to the activation of any of these type II receptors with ALK2 in cells with osteogenic potential, such as tissue-resident fibroadipogenic progenitor cells, and without causing liver necrosis, potentially due to activation of any of the type II receptors with ALK2 in the liver. Specificity of function is regulated by targeting ALK1, which is endothelially restricted, and associated with endothelial BMP9 signaling function, as compared to ALK2, which is expressed throughout all tissues and particularly on mesenchymal stem cells that are responsible for heterotopic ossification, and is associated with the signaling of highly osteogenic molecules such as BMP6 and BMP7.

Thus, an exemplary bispecific antibody is provided that recognizes BMPR2 and ALK1 to replicate signaling of BMP9, for the treatment of vascular conditions including pulmonary arterial hypertension and hereditary hemorrhagic telangiectasia (HHT) syndrome. Other examples are provided below in Table D.

**Table D - Potential musculoskeletal and connective tissue disease applications of bispecific BMP/TGFβ signaling Abs**

| Target combination | Therapeutic use |
|---|---|
| anti-ACTRIIA + anti-ALK2 | chondrogenesis, cartilage repair; fracture healing, non-union, prosthetic engineering; hemochromatosis |
| anti-ACTRIIA + anti-ALK3 | brown fat adipogenesis; metabolic syndrome; renal fibrosis |
| anti-BMPRII + anti-ALK3 | osteogenesis; fracture healing; non-union; prosthetic bone engineering; hemochromatosis |
| anti-BMPRII + anti-ALK6 | tendon repair, healing, and reconstruction |
| anti-TGFBRII + anti-ALK5 | Marfan's syndrome, Loeys-Dietz Syndrome; solid tumors |
| anti-BMPRII + anti-ALK7 | adipogenesis for reconstructive or aesthetic surgery |
| anti-AMHRII + anti-ALK6 | ovarian cancer; modulate fertility/contraception |

Thus provided herein are therapeutic molecules comprising anti-BMPRII/anti-ALK1 bispecific antibodies, which can be used, e.g., for the treatment of pulmonary arterial hypertension via anti-BMPRII/anti-ALK1 bispecific antibodies to augment deficient BMP9 signaling in this disease without heterotopic ossification effects of BMP9 due to ALK2 signaling, avoiding undesirable impact on liver necrosis and regeneration by avoiding the recruitment of ALK2 signaling that is also associated with endogenous BMP9.

The methods described herein can also include treatment of hereditary hemorrhagic telangiectasia via anti-BMPRII/anti-ALK1 bispecific antibodies to augment deficient BMP9 signaling in this disease; treatment of Acute Respiratory Distress Syndrome (ARDS), Acute Lung Injury (ALI) or other pulmonary vascular leak syndromes via anti-BMPRII/anti-ALK1 bispecific antibodies to replicate deficient BMP9 signaling; treatment of various other vascular disease conditions in which endothelial dysfunction may be receptive to modulation by anti-BMPRII/anti-ALK1 bispecific antibodies to replicate deficient BMP9 signaling; treatment of liver fibrosis via anti-BMPRII/anti-ALK1 bispecific antibodies to replicate deficient intrahepatic BMP9 signaling.

The methods can also include treatment of kidney fibrosis via anti-BMPRII/anti-ALK3, anti-BMPRII/anti-ALK2, anti-ACTRIIA/anti-ALK3, or anti-ACTRIIA/anti-ALK2 bispecific antibodies. Identification of the ideal targeting molecule will depend on identification of the the signaling molecule with the most anti-fibrotic effects with the least heterotopic ossification effects.

The methods can also include treatment of bone fractures, correcting fracture non-union, or induction of bone fusion using anti-BMPR2/anti-ALK3 bispecific antibodies to replicate BMP2, BMP4, or BMP6 signaling to promote osteoblast differentiation and bone mineralization, thereby promoting endochondral bone formation.

The methods can also include treatment of bone fractures, correcting fracture non-union, or induction of bone fusion using anti-BMPR2 or anti-ACTRIIA with anti-ALK2 bispecific antibodies to replicate BMP6, BMP7 or BMP8 signaling to promote cartilage formation via chondrocyte differentiation, thereby promoting endochondral bone formation.

The methods can also include *ex vivo* or *in vitro* induction of osteogenic or chondrogenic differentiation using anti-BMPR2/anti-ALK3 or anti-BMPR2/anti-ALK2 bispecific antibodies for the engineering of cartilage or bone tissues.

Therapeutic molecule comprises anti-BMPR2/anti-ALK3, anti-BMPR2/anti-ALK2, anti-ACTRIIA/anti-ALK3, or anti-ACTRIIA/anti-ALK2 bispecific antibodies can be used, e.g., for inducing bone formation *ex vivo* via the activation of BMPRII and ALK3 (or ALK2), or via the activation of ACTRIIA and ALK2 (or ALK3), for the therapeutic engineering of bone following trauma, reconstructive surgery, or orthopedic, spinal or neurosurgical procedures. Also provided herein are therapeutic molecules comprising anti-BMPR2/anti-ALK3 or anti- ACTRIIA/anti-ALK2 bispecific antibodies for inducing hepcidin expression in the liver, and decreasing iron overload in conditions like transfusion dependent anemias, beta-thalassemia and hemochromatosis, by stimulating the activity of BMPRII and ALK3, or ACTRIIA and ALK2 in the liver.

An exemplary therapeutic molecule comprising anti-ACTRIIB/anti-ALK7 bispecific antibodies can be used for inducing adipogenesis via the activation of ACTRIIB and ALK7, for reconstructive and aesthetic surgery applications. These agents could be used for plastic surgery, orthopedic and reconstructive surgical applications in situ, or ex vivo for the manufacture of biological implants from patient-derived or human progenitor cells.

Therapeutic molecules comprising anti-BMPR2/anti-ALK2 or anti-ACTRIIA/anti-ALK2, for targeting BMPRII or ACTRIIA together with ALK2, can be used to promote brown fat adipogenesis, to improve or correct metabolic syndrome, obesity, or diabetes mellitus and related disorders by improving energy utilization.

Therapeutic molecules comprising anti-BMPR2/anti-ALK6 or anti-ACTRIIA/anti-ALK6 bispecific antibodies can be used, e.g., for inducing tendon formation via the activation of BMPRII or ACTRIIA and ALK6, by simulating the tissue-specific effects of GDF5, GDF6, and GDF7 in tendon-progenitor tissues, or mesenchymal stem cells with tendon potential. These agents could be used for orthopedic and reconstructive surgical applications in situ, or ex vivo for the manufacture of biological implants from patient-derived or human progenitor cells.

Therapeutic molecules comprising anti-TGFBRII/anti-ALK5, anti-TGFBRII/anti-ALK4, anti-ACTRIIA/anti-ALK5 or anti-ACTRIIA/anti-ALK4, anti-ACTRIIB/anti-ALK5 or anti-ACTRIIB/anti-ALK4 bispecific antibodies can be used, e.g., for inducing growth arrest of tumor cells by the activation of TGFBRII and ALK5, or via the activation of TGFBRII and ALK4, or alternatively ACTRIIA or ACTRIIB with ALK4 or ALK5.

Therapeutic molecules comprising anti-TGFBRII/anti-ALK5 anti-TGFBRII/anti-ALK4 bispecific antibodies can be used, e.g., for inducing the formation of fibrotic tissue for reconstructive or aesthetic surgical applications, via the activation of TGFBRII and ALK5, or via the activation of TGFBRII and ALK4, or for the treatment of Marfan's disease, Loeys-Dietz Syndrome, and other aortopathies due to dysregulated or deficient TGFBRII signaling, via the activation of TGFBRII and ALK5, or via the activation of TGFBRII and ALK4.

Therapeutic molecules comprising anti-ACTRIIA/anti-ALK5 or anti-ACTRIIA/anti-ALK4 bispecific antibodies can be used, e.g., for targeting ACTRIIA and ALK4, or ALK5, to inhibit pathologic hypertrophy, such as that seen in hypertrophic cardiomyopathy, or hypertensive heart disease.

Other combinations of receptors could be targeted via bispecific antibodies to induce novel physiologic effects in tissues and cells. For example: Anti-TGFBR2/anti-ALK3; anti-BMPR2/anti-ALK4; anti-BMPR2/anti-ALK5 may induce physiology dictated by the type I receptors (osteogenesis, anti-myogenesis, or fibrosis respectively simulating BMP2/4 via ALK3, GDF8/11 via ALK4, and TGFb1/3 via ALK5), but in a highly restricted subset of tissues expressing this specific complement of type II and type I receptors. Alternatively, these novel or non-native signaling combinations may elicit unique biology not seen with endogenous ligands, but may be therapeutically useful. Such biology could include chondrogenesis decoupled from mineralization, or mineralization decoupled from chondrogenesis.

In any of the above bispecific antibodies or applications, anti-AMHRII, anti-ACTRIIA or anti-ACTRIIB may be interchangeable with anti-BMPRII in combination with given anti-type I receptor antibodies to accomplish similar, more potent, or more tissue-selective effects.

As used in this context, to "treat" means to ameliorate at least one symptom of the disorder. Administration of a therapeutically effective amount of a compound described herein for the treatment of a condition will result in improvement in at least one symptom or clinical parameter.

### VII. Pharmaceutical Compositions and Methods of Administration

The methods described herein include the use of pharmaceutical compositions comprising bispecific antibodies described herein as an active ingredient.

Pharmaceutical compositions typically include a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral, e.g., intravenous, intradermal, subcutaneous, oral (e.g., inhalation), transdermal (topical), transmucosal, and rectal administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressured container or dispenser that contains a suitable propellant, e.g., a gas such as carbon dioxide, or a nebulizer. Such methods include those described in U.S. Patent No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The pharmaceutical compositions can also be prepared in the form of suppositories (e.g., with conventional suppository bases such as cocoa butter and other glycerides) or retention enemas for rectal delivery.

Therapeutic compounds that are or include nucleic acids can be administered by any method suitable for administration of nucleic acid agents, such as a DNA vaccine. These methods include gene guns, bio injectors, and skin patches as well as needle-free methods such as the micro-particle DNA vaccine technology disclosed in U.S. Patent No. 6,194,389, and the mammalian transdermal needle-free vaccination with powder-form vaccine as disclosed in U.S. Patent No. 6,168,587. Additionally, intranasal delivery is possible, as described in, *inter alia,* Hamajima et al., Clin. Immunol. Immunopathol., 88(2), 205-10 (1998). Liposomes (e.g., as described in U.S. Patent No. 6,472,375) and microencapsulation can also be used. Biodegradable targetable microparticle delivery systems can also be used (e.g., as described in U.S. Patent No. 6,471,996).

In one embodiment, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or obtained commercially, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

The pharmaceutical compositions can be included in a container, pack, or dispenser together with instructions for administration.

### EXAMPLES

The invention is further described in the following examples, which do not limit the scope of the invention described in the claims.

### Material and Methods

The following materials and methods were used in the Examples below.

### Cell Culture and Western Blot Analysis

Pulmonary Artery Endothelial Cells (PAEC, Patient #593089, Lonza) were seeded in complete medium (Lonza #CC-3156 and #CC-4178) at 400,000 cells per well in 12-well plates (Falcon^{™} Polystyrene Microplates, Corning #353043). After overnight incubation at 37°C with 5.0% CO₂, cells were washed with PBS (GIBCO #14190-144), fed with fresh media, and incubated for 24 hours. PAEC were rinsed twice with PBS and starved in basal media with 0.1% FBS (GIBCO #A3160402) for 24 hours. Selected wells were preincubated for 60 minutes with the two most promising biotinylated scFv combination (ALK1.8 and BMPR2.12) at 10 ng/mL each; and then for 1 hour with 1 ng/mL of rhBMP9 (R&D 3209BP-CF), 1 mg/mL Streptavidin (ThermoFisher #21122), and 10 ng/mL monoclonal BMP9 neutralizing antibody (R&D MAB3209) in various co-treatments. The wells were washed with PBS and harvested in 80 mL of 1x NuPAGE LDS Sample Buffer (ThermoFisher #NP0007). Samples were incubated at 100°C for 5 minutes then separated by electrophoresis and analyzed by western blotting using specific antibodies recognizing phosphorylated forms of SMAD1/3 (Abcam 52903), SMAD1/5/8 (Cell Signaling, Technology, CST #9516), and SMAD2 (CST #3108), using total Smad1 (CST#6944) and GAPDH (Thermo Fisher MA5-15738-HRP) as housekeeping markers. Western blot was detected with SuperSignal^{™} West Femto Maximum Sensitivity Substrate (ThermoFisher #34096) and Kodak Carestream In Vivo MS-FX Pro Multispectral Imaging System.

Pulmonary Microvascular Endothelial Cells (PMVEC) from *wild type* and *Acvrl1 KO* Mice were seeded in complete medium (Lonza #CC-3156 and #CC-4178) at 400,000 cells per well in 12-well plates (Falcon^{™} Polystyrene Microplates, Corning #353043). After overnight incubation at 37°C with 5.0% CO₂, cells were washed with PBS (GIBCO #14190-144), fed with fresh media, and incubated for 24 hours. PAEC were rinsed twice with PBS and starved in basal media with 0.1% FBS (GIBCO #A3160402) for 24 hours. Selected wells were preincubated for 60 minutes with the two most promising biotinylated scFv combination (ALK1.8 and BMPR2.12) at 10 ng/mL each; and then for 1 hour with 1 ng/mL of rhBMP9 (R&D 3209BP-CF), 1 ug/mL Streptavidin (ThermoFisher #21122), and 10 ng/mL monoclonal BMP9 neutralizing antibody (R&D MAB3209) in various co-treatments. The wells were washed with PBS and harvested in 80 mL of 1x NuPAGE LDS Sample Buffer (ThermoFisher #NP0007). Samples were incubated at 100°C for 5 minutes then separated by electrophoresis and analyzed by western blotting using specific antibodies recognizing phosphorylated forms of SMAD1/3 (Abcam #52903), SMAD1/5/8 (CST #9516), and SMAD2 (CST #3108), using total SMAD1 (CST#6944) and GAPDH (Thermo Fisher MA5-15738-HRP) as housekeeping markers. Western blot was detected with SuperSignal^{™} West Femto Maximum Sensitivity Substrate (ThermoFisher #34096) and Kodak Carestream In Vivo MS-FX Pro Multispectral Imaging System

### Biotinylation Procedure

The most promising scFv clones were biotinylated in order to cross-link receptors when stimulated with streptavidin. The samples were dialyzed using a Zeba^{™} Spin Desalting Colum with a cut-off of 7 KDa (ThermoFisher #89889) and then conjugated using the DSB-X^{™} Biotin Protein Labeling Kit (ThermoFisher #D20655).

The efficacy of the biotinylation procedure was demonstrated by Western Blot using Streptavidin-HRP (ThermoFisher #21130). Preservation of binding affinity and specificity was demonstrated by Bio-Layer Interferometry (BLI, Octet Red) analysis to confirm the ability of biotinylated-scFv proteins to bind to immobilized ALK1 and BMPR2 receptors in a specific fashion.

### In-Cell Western

Telomerase-Immortalized Microvascular Endothelial (TIME) Cell cultures were seeded in complete media (ATCC # 100-030 and 100-041) onto high-bind 96 well plates (Corning #3340), grown to confluence, and deprived of serum for 24 hours. TIME Cells were treated with rhBMP9 (20 pM), scFv proteins (500 pg/mL), and streptavidin (1 mg/mL) in various co-treatments. The plate was washed twice with PBS and fixed with cold methanol (Fisher #412), washed, permeabilized, washed, blocked with 2% BSA (Fisher #BP1600) in TBS (ThermoFisher #J75892). Primary antibodies specific for p-SMAD1/5 (CST #9516) or p-SMAD2 (CST #8828) were added (1:1000 dilution) followed by secondary antibodies (HRP anti-rabbit IgG, CST 1:10000). After washing, assays were developed using BioFx UltraSensitive Chemiluminescent Substrate (Surmodics) and developed on a SpectraMax Plate Luminometer with 0.25-second integrations.

### Luciferase Assay

Bovine Aortic Endothelial Cells (BAEC, Sigma-Aldrich #B304-05) were seeded in complete media (Lonza, #CC-3156 and CC-4176) onto 96 well plates (Corning #3340) at a concentration of 31,000 cells per well. The day after, the cells were transfected using 60 ng/mL of PolyJet (SignaGen, SL100688) and 20 ng/mL of *BRE-LUC* plasmid per well as described in the transfection reagent protocol. After 16 hours, the cells were washed twice with PBS and fed with reduced serum media for 24 hours recovery. The following day, BAEC were washed with PBS and starved for 6 hours (FBS 0.1%); treated with 20 pM rhBMP9, 20 nM of ALK1-Fc (R&D #370AL) and 20 nM of different scFvs; then incubated overnight at 37°C with 5.0% CO₂.

The next day, we tested cell viability using an MTS assay (Promega #G3582) and performed a Luciferase activity assay as described by the Promega Kit (#1500). The plate was read on a SpectraMax Plate Luminometer with 0.25-second integrations.

### Octet Red

To characterize the biochemical interactions of the scFv clones with recombinant human ALK1 and BMPR2 receptors, we used Bio-Layer Interferometry (Octet Red 384, ForteBio). Receptors, expressed as IgG Fc fusion proteins (i.e., ALK1-Fc, and BMPR2-Fc) were immobilized onto Anti-Human IgG Fc Capture Sensors (ForteBio, #18-0015) at a concentration of 10 µg/mL in an equilibration buffer of HEPES 20 mM (GIBCO #15630), 0.05% Tween-20 (Promega #H5151), and Imidazole 25 mM (SIGMA #I0250) in PBS. All the clones were tested at different ranges of concentration in equilibration buffer: 50 nM, 100 nM, 200 nM, and 400 nM. The ForteBio program methodology was set as follows: Baseline equilibration of 60 s, loading time of 60 s, washing time of 120 s, association time of 300 s, and dissociation time of 600 s. The binding kinetics were analyzed using the ForteBio data analysis software.

### Example 1. Single chain Fv (scFv) Abs for BMP/TGFβ receptor extracellular domains can modulate signaling and form functional ligands.

We performed screening to identify a panel of human scFv Abs that bind to the extracellular domains for ALK1 and BMPR2, the type I and type II receptors that form the signaling complex for endothelial specific ligand BMP9.^{6,7} A combinatorial diversity library of ~10¹² distinct phage particles expressing single chain variable region (scFv) Abs derived from human germline V_{H} and V_{L} genes was screened for phage clones binding to recombinant human ALK1-Fc or BMPR2-Fc extracellular domain-IgG Fc fusion proteins, but not IgG Fc itself using sequential affinity purification for immobilized recombinant ALK1 or BMPR2 proteins, and depletion of non-specific clones, revealing several clones of interest. Three rounds of enrichment generated a panel of scFv binding to ALK1 or BMPR2 but not both, and with unique sequences. These phage sequences were subcloned into scFV expression vectors for scale up and purification in E. coli. For each target, several scFv fragments exhibited moderate to high affinity (K_{D}~20-200 nM) for their intended targets based on Octet Red Bio-Layer Interferometry (BLI, Fortebio), and retained this affinity and specificity even after low substitution ratio biotinylation (average 1.5-2 sites per molecule), with preserved binding to one receptor but not the other, and not to control ACTRIIA-Fc protein. CDR Sequence were predicted using the PARATOME On-Line Tool (Kunik et al., PLoS Comput Biol 8(2): e1002388 (2012); Kunik et al., Nucleic Acids Res. 2012 Jul;40(Web Server issue): W521-4 (2012)). Thus, several scFv candidates binding to each antigen were selected, activity confirmed by ELISA, and sequences were cloned into an expression vector and expressed as single chain Fv proteins.

### Screening of binding efficiency for the Single Chain Variable Fragments positive clones against ALK1 and BMPR2 by Bio-Layer Interferometry (BLI).

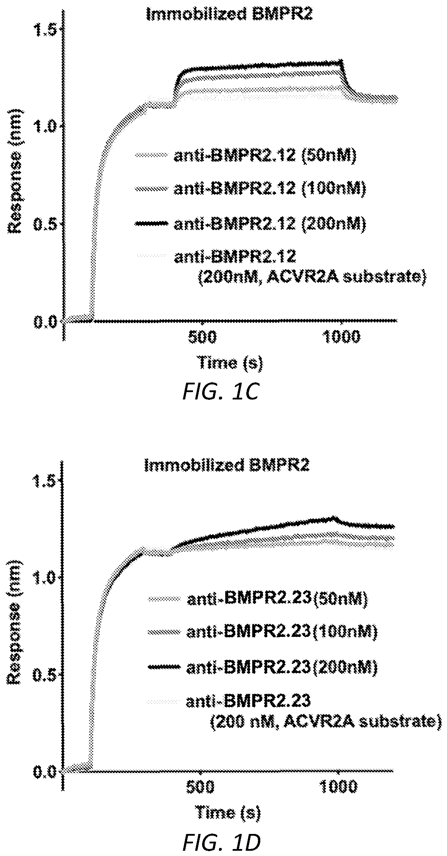

Bio-Layer Interferometry was used to determine the affinities of scFv clones for human BMPR2 and ALK1 receptors. The binding kinetics of scFv clones ALK1.3 (**Figure 1A**) and ALK1.8 (**Figure 1B**) versus immobilized ALK1; and BMPR2.12 (**Figure 1C**) and BMPR2.23 (**Figure 1D**) against BMPR2 are shown. The specificity of these clones against immobilized ALK1 (**Figure 2A**) vs. BMPR2 (**Figure 2B**) was also tested, revealing that each of these clones exhibited affinity for their intended target, but not a highly homologous control protein, the human ACVR2A receptor (**Figures 1** **and** **2C**).

### scFv directed against BMPR2 or ALK1 inhibit BMP9 signaling in vitro.

To test the ability of the selected single-chain antibodies to recognize the extracellular ligand-binding domains of human ALK1 and BMPR2 receptors, we used a cell-based assay of BNW-transcriptional activity using the BMP-response element luciferase (BRE-Luciferase) reporter, via a plasmid expressing firefly luciferase regulated by the BMP Responsive Element promoter sequence cloned from the human *ID1* gene. Transfected cells were stimulated overnight with rhBMP9 (1 ng/mL) in presence/absence of varying concentrations of scFv proteins. The BMP9 ligand trap ALK1-Fc was also used as positive control. The scFV clones BMPR2.12, BMPR2.23, ALK1.3, and ALK1.8 exhibited the most efficient inhibition of BRE-Luciferase activity, suggesting that these scFv proteins bind regions of human ALK1 and BMPR2 receptors associated with ligand binding (**Figure 3**).

### Biotinylation of scFv proteins.

The ALK1.3, ALK1.8, BMPR2.12, and BMPR2.23 scFv were biotinylated as described in the materials and methods section. The resulting biotinylated scFvs were tested by western blot analysis to demonstrate their successful biotinylation (**Figure 4A**).

### Binding efficiency and specificity of biotinylated scFv against ALK1 and BMPR2.

To verify that the biotinylation of the scFvs did not interfere with their binding capacity, the positive biotinylated and wild type clones were again screened using Bio-Layer Interferometry. Biotinylated ALK1.3 and ALK1.8 scFv proteins (ALK1.3-Bio, ALK1.8-Bio, **Figure 4B** **and** **4D**) and BMPR2.12 scFv proteins (BMPR2.12-Bio, **Figure 4C**) retained affinity for their intended target, while maintaining specificity based on lack of affinity for highly homologous control proteins.

### Biotinylated scFV complexes targeting ALK1 and BMPR2 induced SMAD1/5/9 activation comparable to the activity of BMP9 in endothelial cells.

In order to demonstrate specific activation of BMPR2:ALK1 signaling *in vitro,* human and murine microvascular endothelial cell lines were preincubated with different combinations of biotinylated scFv and treated with Streptavidin. Treatment of cultured human pulmonary microvascular endothelial cells (PMVEC) with biotinylated ALK1.8/BMPR2.12 scFv complexes in in the presence of Streptavidin for 30' at 37C induced the phosphorylation of SMAD1/5/9, whereas incubation with biotinylated ALK1.8 or BMPR2.12 individually did not activate signaling (**Figure 5**). The signaling via biotinylated ALK1.8/BMPR2.12 streptavidin complexes was similar to that seen following treatment with recombinant human BMP9, but in contrast to rhBMP9, was not affected the BMP9 neutralizing antibodies (mAb3209) or the BMP9 ligand trap ALK1-Fc as seen by Western Blot (**Figure 5**) and In-Cell Western analysis (**Figure 6B**). A similar experiment has been repeated using *Wild-Type (WT)* and *Acvrl1 KO* murine PMVEC, showing activation of SMAD1/5/9 signaling in response to the ALK1.8/BMPR2.12 complexes in WT murine cells comparable to results in human PMVEC, but not in *Acvrl1* KO PMVEC (**Figure 6A**), consistent with a requirement of ALK1 for signaling via the bispecific scFv complex.

Exemplary sequences for some of the identified scFV CDRs are shown in Table 1.

**Table 1. Sequences for single chain Fv BMPRI/BMPRII antibody CDRs**

| **CDR** | **SEQUENCE** | **SEQ ID NO** |
|---|---|---|
| Clone ALK1.2 (anti-ACVRL1/ALK1) * | | |
| CDR L1 | KLGDKYAS | 1. |
| CDR L2 | LVIYQDSKRPS | 2. |
| CDR L3 | QAWDSSTA | 3. |
| CDR H1 | GSISSDDYYWS | 4. |
| CDR H2 | WIGYIYYSGITYY | 5. |
| CDR H3 | REGCNDGVCYNGVFDY | 6. |

| Clone ALK1.3 (anti-ACVRL1/ALK1) | | |
|---|---|---|
| CDR L1 | SSDVGTYNYVS | 7. |
| CDR L2 | LMIYDVSKRPS | 8. |
| CDR L3 | YSYTTSSTW | 9. |
| CDR H1 | DSIGSGDYYWS | 10. |
| CDR H2 | WIGYSYHTGSTDY | 11. |
| CDR H3 | RDYYGYLGY | 12. |

| Clone ALK1.8 (anti-ACVRL1/ALK1)* | | |
|---|---|---|
| CDR L1 | QSISSYLN | 13. |
| CDR L2 | LLIYAASSLQS | 14. |
| CDR L3 | QQSYSTPR | 15. |
| CDR H1 | GSISSDDYYWS | 16. |
| CDR H2 | WIGYIYYSGITYY | 17. |
| CDR H3 | REGCNDGVCYNGVFDY | 18. |

| Clone ALK1.8b (anti-ACVRL1/ALK1) | | |
|---|---|---|
| CDR L1 | SSNIGGNTVN | 19. |
| CDR L2 | LLIYGDDLRPS | 20. |
| CDR L3 | AAWDDSLNAY | 21. |
| CDR H1 | FTFSNYAMHS | 22. |
| CDR H2 | WVAVISYDGSNKYY | 23. |
| CDR H3 | RVRYYGSGSPIGY | 24. |

| Clone BMPR2.12 (anti-BMPR2/BMPRII) | | |
|---|---|---|
| CDR L1 | SSDVGGYKSVS (26-36) | 25. |
| CDR L2 | LMIYDVSNRPS (48-58) | 26. |
| CDR L3 | SSYTSSSSLW (91-100) | 27. |
| CDR H1 | GTFSSYAIS | 28. |
| CDR H2 | WMGRIIPILGIANY | 29. |
| CDR H3 | TDLWGVGAD | 30. |

| Clone B15 (Anti-BMPR1A/ALK3) | | |
|---|---|---|
| CDR L1 | NTDIGYYNYVS | 31. |
| CDR L2 | LLIFEVDNRPS | 32. |
| CDR L3 | SSYSTYNIL | 33. |
| CDR H1 | YTFTGYYMH | 34. |
| CDR H2 | WMGRINPNSGGTNYA | 35. |
| CDR H3 | RDPTYYDILTGPEY | 36. |

| Clone B16 (Anti-BMPR1A/ALK3) | | |
|---|---|---|
| CDR L1 | QSVSSNLA | 37. |
| CDR L2 | LLIYGASSRAT | 38. |
| CDR L3 | QQYIYSPY | 39. |
| CDR H1 | YNFAGYYVH | 40. |
| CDR H2 | WMGWINPNSGGTDYA | 41. |
| CDR H3 | RNLQFFRP | 42. |

| Clone RI-2 (Anti-ACVR1/ALK2) | | |
|---|---|---|
| CDR L1 | NIGSKNVH | 43. |
| CDR L2 | LVIYDDSDRPS | 44. |
| CDR L3 | QAWDSSTAV | 45. |
| CDR H1 | GSISSSSYYWG | 46. |
| CDR H2 | WIGSIYYSGSTYY | 47. |
| CDR H3 | RQVGPVLKSVS | 48. |

| Clone RI-3 (Anti-ACVR1/ALK2) | | |
|---|---|---|
| CDR H1 | FTFSSYAMS | 49. |
| CDR H2 | WVSAISGSGGSTYY | 50. |
| CDR H3 | KSASFFDY | 51. |
| CDR L1 | QSVSSSYLA | 52. |
| CDR L2 | LLIYGASSRAT | 53. |
| CDR L3 | QQHFRWPW | 54. |

| Clone RI-4 (Anti-ACVR1/ALK2) | | |
|---|---|---|
| CDR L1 | SSNIGSNTVN | 55. |
| CDR L2 | LLIYSNNQRPS | 56. |
| CDR L3 | AAWDDSLNGV | 57. |
| CDR H1 | ASISSGGYYWS | 58. |
| CDR H2 | WIGYSYYSGSTYY | 59. |
| CDR H3 | RGGVRFDL | 60. |

| Clone RI-2-7 (Anti- ACVR1/ALK2) | | |
|---|---|---|
| CDR L1 | ALPKKYAY | 61. |
| CDR L2 | LVIYEDSKRPS | 62. |
| CDR L3 | YSTDSSGNP | 63. |
| CDR H1 | FTFSSYAMS | 64. |
| CDR H2 | WVSAISGSGGSTYY | 65. |
| CDR H3 | KIPWGDYDFWSGYYTRPGHSGMDV | 66. |

| Clone RI-9 (Anti-ACVR1/ALK2) | | |
|---|---|---|
| CDR L1 | SSNIGAGYDVH | 67. |
| CDR L2 | LLIFDSSNRPS | 68. |
| CDR L3 | QSYDFRLSGSM | 69. |
| CDR H1 | FTFRAYPMS | 70. |
| CDR H2 | WVSSITSSGSYY | 71. |
| CDR H3 | RVGVTDFDY | 72. |

| Clone RII-19 (Anti-ACVR2A/ACTRIIA) | | |
|---|---|---|
| CDR L1 | SSNIGTNYVY | 73. |
| CDR L2 | LLIYRNNQRPS | 74. |
| CDR L3 | AAWDDSLSGW | 75. |
| CDR H1 | FTFSSYAMS | 76. |
| CDR H2 | WVSAISGSGGSTYYA | 77. |
| CDR H3 | AKSIRTGSGYFDY | 78. |

| Clone H1-2 (Anti-ACVR2A/ACTRIIA) | | |
|---|---|---|
| CDR L1 | SSNIGSNYVY | 79. |
| CDR L2 | LLIYRNNQRPS | 80. |
| CDR L3 | AAWDDSLSGW | 81. |
| CDR H1 | YTFTSYYMH | 82. |
| CDR H2 | WMGIINPSGGSTSYA | 83. |
| CDR H3 | RDLLLYGRGGGFQH | 84. |

| Clone H2-11 (Anti-ACVR2B/ACTRIIB) | | |
|---|---|---|
| CDR L1 | SLRSYYAS | 85. |
| CDR L2 | LVIYGKNNRPS | 86. |
| CDR L3 | NSRDNSGKHLW | 87. |
| CDR H1 | FTFDDYGMS | 88. |
| CDR H2 | WVSGINWNGGSTGY | 89. |
| CDR H3 | RKSTGDY | 90. |

| Clone H2-15 (Anti-ACVR2B/ACTRIIB) | | |
|---|---|---|
| CDR L1 | SSNIGAGYDVH | 91. |
| CDR L2 | LLIYGNSNRPS | 92. |
| CDR L3 | AAWDDSLNGPRM | 93. |
| CDR H1 | FTFSDYYMS | 94. |
| CDR H2 | WVSYISSSSSYTNY | 95. |
| CDR H3 | RVNYYYGMDV | 96. |

| Clone H5-2 (Anti-ACVR1B/ALK4) | | |
|---|---|---|
| CDR L1 | SLRKYYAS | 97. |
| CDR L2 | LVIYGKNKRPS | 98. |
| CDR L3 | NSWDSSGNHV | 99. |
| CDR H1 | GSISSGGYYWS | 100. |
| CDR H2 | WIGYIYYSGSTYY | 101. |
| CDR H3 | REEVGDWFDP | 102. |

| Clone H3-2 (Anti-BMPR1B/ALK6) | | |
|---|---|---|
| CDR L1 | QGDSLRKYYAS | 109. |
| CDR L2 | LVIYGKSKRPS | 110. |
| CDR L3 | NSWDSSGNHVV | 111. |
| CDR H1 | GGSISSGGYYWS | 112. |
| CDR H2 | WIGYIYYSGSTYY | 113. |
| CDR H3 | REEVGDWFDP | 114. |

| Clone BMPR2.23 (anti-BMPR2/BMPRII) | | |
|---|---|---|
| CDR L1 | TLHSGINVATYRIY | 115. |
| CDR L2 | YKSDLDKHQGS | 116. |
| CDR L3 | LIWHDYVWY | 117. |
| CDR H1 | CAASGFTFDDYAMH | 118. |
| CDR H2 | WVSGISWNSGSIGY | 119. |
| CDR H3 | KGGWGSSTHYYYGMDV | 120. |

| Clone H4-1 (Anti-ACVR1C/ALK7) | | |
|---|---|---|
| CDR L1 | SSNIGSNYVY | 121. |
| CDR L2 | LLIYRNNQRPS | 122 |
| CDR L3 | AAWDDSLSGV | 123 |
| CDR H1 | YTFTGYYMH | 124. |
| CDR H2 | WMGWINPNGGGTKYA | 125. |
| CDR H3 | TLSDYSSGGP | 126. |

| Clone H4-4 (Anti-ACVR1C/ALK7) | | |
|---|---|---|
| CDR L1 | SSNIGSNYVY | 127. |
| CDR L2 | LLIYRNNQRPS | 128. |
| CDR L3 | AAWDDSLSGV | 129. |
| CDR H1 | YTFTGYHVH | 130. |
| CDR H2 | WMGWINPDSGGTNFA | 131. |
| CDR H3 | RRSLDY | 132. |

| Clone H4-7 (Anti-ACVR1C/ALK7)) | | |
|---|---|---|
| CDR L1 | SSNIGSNYVY | 133. |
| CDR L2 | LLIYRNNQRPS | 134. |
| CDR L3 | AAWDDSLNGV | 135. |
| CDR H1 | FTFSNAWMS | 136. |
| CDR H2 | WVGRIKSKTDGGTTDY | 137. |
| CDR H3 | ASLVDY | 138. |

| Clone H4-8 (Anti-ACVR1C/ALK7) | | |
|---|---|---|
| CDR L1 | SSNIGSNYVY | 139. |
| CDR L2 | LLIYRNNQRPS | 140. |
| CDR L3 | AAWDDSLSGP | 141. |
| CDR H1 | YTFTKYGIS | 142. |
| CDR H2 | WMGWISAYNVDTKY | 143. |
| CDR H3 | RRGLDY | 144. |

| | | |
|---|---|---|
| *Alk1.2 and Alk1.8 share 98% of identity at the heavy chain CDRs but have distinct light chain CDRs. | | |

Examples of sequences of anti-BMPRI/BMPRII antibodies are shown below. The sequences are shown as VL-**linker**-VH, where the linker is in bold.
**Clone ALK1.2 (Anti-ALK1 (ACVRL1))**
   Nucleotide Sequence (SEQ ID NO: 145) 
CAGCTGANTACNGNCGACGATGACAAGCTTTCCTATGAGCTGACACAGCCAC
CCTCAATGTCCGTGTCCCCAGGACAGACAGCCAGCATCACCTGCTCTGGAGA
TAAATTGGGAGATAAATATGCTTCCTGGTATCAGCAGAAGCCAGGCCAGTCC
CCTGTGCTGGTCATCTATCAAGATAGCAAGCGGCCCTCAGGGATCCCTGAGC
GATTCTCTGGCTCCAACTCTGGGAACACAGCCACTCTGACCATCAGCGGGAC
CCAGGCTATGGATGAGGCTGACTATTACTGTCAGGCGTGGGACAGCAGCACT
GCGGTATTCGGTGGAGGGACCAAGCTCACCGTCCTAGGTGAGGGTAAATCT
TCCGGATCTGGTTCCGAATCCAAAGCTAGCCAGGTGCAGCTGCAGGAGTC
GGGCCCAGGACTGGTGAAGCCTTCACAGACCCTGTCCCTCACCTGCACTGTCT
CTGGTGGCTCCATCAGCAGTGATGATTACTACTGGAGTTGGATCCGCCAGAC
CCCAGGGAAGGGCCTGGAGTGGATTGGGTACATCTATTACAGTGGGATCACC
TACTACAACCCGTCCCTCAAGAGTCGAGTTACCATATCAGTAGACACGTCGA
AGAACCAGTTCTCCCTGAAGCTGAGCTCTGTGACTGCCGCAGACACGGCCGT
GTATTACTGTGCCAGAGAGGGGTGTAATGATGGTGTATGCTATAATGGGGTC
TTTGACTACTGNGGCCAGGGCACCCTGGTCACCGTCTCCTCAGTCGACCCATT
CGTTTCTGAATATCAAGGCCAATCGTCTGACCTGCCTCAACCTCCTGTCAATG
CTGGCGGCGGCTCTGANGGAGGCGGTTCCGGTGGTGGCTCTGGTTCCGGTGA
TTTTGATTATGAAAAGATGGC
   Amino acid Sequence (SEQ ID NO: 146) 
SYELTQPPSMSVSPGQTASITCSGDKLGDKYASWYQQKPGQSPVLVIYQDSKRPS
GIPERFSGSNSGNTATLTISGTQAMDEADYYCQAWDSSTAVFGGGTKLTVLGEG
KSSGSGSESKASQVQLQESGPGLVKPSQTLSLTCTVSGGSISSDDYYWSWIRQTP
GKGLEWIGYIYYSGITYYNPSLKSRVTISVDTSKNQFSLKLSSVTAADTAVYYCA
REGCNDGVCYNGVFDYWGQGTLVTVSS
**Clone ALK1.3 (Anti-ALK1 (ACVRL1))**
   Nucleotide Sequence (SEQ ID NO: 147) 
CAGTCTGCCCTGACTCAGCCTGCCTCCGTGTCTGGGTCTCCTGGACAGTCGAT
CACCATCTCCTGCGCTGGAACCAGCAGTGACGTTGGTACTTATAACTATGTCT
CCTGGTACCAACAACACCCAGGCAAAGCCCCCAAACTCATGATTTATGATGT
CAGTAAGCGGCCCTCAGGGGTCCCTGATCGCTTCTCTGGCTCCAAGTCTGGCA
ACACGGCCTCCCTGACCATCTCTGGGCTCCAGGCTGAGGACGAGGCTGATTA
TTACTGCTACTCATATACAACCAGCAGCACTTGGGTGTTCGGCGGAGGGACC
AAGGTCACCGTCCTAGGAGAGGGTAAATCTTCCGGATCTGGTTCCGAAAG
CAAGGCTAGCCAGGTGCAGCTGCAGGAGTCGGGCCCAGGACTGCTGAAGCC
TTCGCAGACCCTGTCCCTCACCTGCACTGTCTCCGGTGACTCCATCGGCAGTG
GTGATTACTACTGGAGTTGGATCCGCCAGCCCCCAGGGAAGGGCCTGGAATG
GATTGGGTACAGCTATCACACTGGGAGCACCGACTACAACCCGTCCCTCAAG
AGTCGAGTTTCCATATCAGTGGACAGGTCCAAGAACCAGTTCTCCCTTAAACT
GAGGTCTGTGACTGCCGCAGACACGGCCGTGTATTACTGTGCCAGAGATTAT
TATGGATATTTGGGTTACTGGGGCCAGGGAACCCTGGTCACCGTCTCCTCA
   Amino acid Sequence (SEQ ID NO: 148) 
QSALTQPASVSGSPGQSITISCAGTSSDVGTYNYVSWYQQHPGKAPKLMIYDVSK
RPSGVPDRFSGSKSGNTASLTISGLQAEDEADYYCYSYTTSSTWVFGGGTKVTVL
GEGKSSGSGSESKASQVQLQESGPGLLKPSQTLSLTCTVSGDSIGSGDYYWSWIR
QPPGKGLEWIGYSYHTGSTDYNPSLKSRVSISVDRSKNQFSLKLRSVTAADTAVY
YCARDYYGYLGYWGQGTLVTVSS
**Clone ALK1.8 (Anti-ALK1 (ACVRL1))**
   Nucleotide Sequence (SEQ ID NO: 149) 
GACATCCAGATGACCCAGTCTCCATCCTCCCTGTCTGCATCTGTAGGAGACAG
AGTCACCATCACTTGCCGGGCAAGTCAGAGCATTAGCAGCTATTTAAATTGG
TATCAGCAGAAACCAGGGAAAGCCCCTAAGCTCCTGATCTATGCTGCATCCA
GTTTGCAAAGTGGGGTCCCATCAAGGTTCAGTGGCAGTGGATCTGGGACAGA
TTTCACTCTCACCATCAGCAGTCTGCAACCTGAAGATTTTGCAACTTACTACT
 
GTCAACAGAGTTACAGTACCCCTCGAACGTTCGGCCAAGGGACCAAAGTGGA
TATCAAAGAGGGTAAATCTTCCGGATCTGGTTCCGAATCCAAAGCTAGCC
AGGTGCAGCTGCAGGAGTCGGGCCCAGGACTGGTGAAGCCTTCACAGACCCT
GTCCCTCACCTGCACTGTCTCTGGTGGCTCCATCAGCAGTGATGATTACTACT
GGAGTTGGATCCGCCAGACCCCAGGGAAGGGCCTGGAGTGGATTGGGTACAT
CTATTACAGTGGGATCACCTACTACAACCCGTCCCTCAAGAGTCGAGTTACC
ATATCAGTAGACACGTCGAAGAACCAGTTCTCCCTGAAGCTGAGCTCTGTGA
CTGCCGCAGACACGGCCGTGTATTACTGTGCCAGAGAGGGGTGTAATGATGG
TGTATGCTATAATGGGGTCTTTGACTACTGGGGCCAGGGCACCCTGGTCACCG
TCTCCTCA
   Amino acid Sequence (SEQ ID NO: 150) 
DIQMTQSPSSLSASVGDRVTITCRASQSISSYLNWYQQKPGKAPKLLIYAASSLQS
GVPSRFSGSGSGTDFTLTISSLQPEDFATYYCQQSYSTPRTFGQGTKVDIKEGKSS
GSGSESKASQVQLQESGPGLVKPSQTLSLTCTVSGGSISSDDYYWSWIRQTPGKG
LEWIGYIYYSGITYYNPSLKSRVTISVDTSKNQFSLKLSSVTAADTAVYYCAREG
CNDGVCYNGVFDYWGQGTLVTVSS
**Clone ALK1.8b (Anti-ALK1 (ACVRL1))**
   Nucleotide Sequence (SEQ ID NO: 151) 
CTGCCTGTGCTGACTCAGCCACCCTCAGCGTCGGGGACCCCCGGGCAGAGGG
TCACCATCTCTTGTTCTGGAAGCAGCTCCAACATCGGAGGTAATACTGTAAAC
TGGTATCACCAACTCCCAGGATCGGCCCCCAAACTCCTCATCTATGGTGATGA
TCTGCGGCCCTCAGGGGTCCCTGACCGATTCTCCGGCTCCAAGTCTGGCACGT
CAGCCTCCCTGGCCATCAGTAGGTTCCAGTCTGGGGATGAGGCTGATTATTAC
TGTGCAGCATGGGATGACAGCCTGAATGCTTATGTCTTCGGAACTGGGACCA
AGCTCACCGTCCTAGGTGAGGGTAAATCTTCCGGATCTGGTTCCGACTCC
AAAGCTAGCGAGGTGCAGCTGGTGGGGTCTGGGGGAGGCGTGGTCCAGCCT
GGGAGGTCCCTGAGACTCTCCTGTGCAGCCTCTGGATTCACCTTCAGTAACTA
TGCTATGCACTGGGTCCGCCAGGCTCCAGGCAAGGGGCTGGAGTGGGTGGCA
GTTATATCATATGATGGAAGTAATAAATACTACGCAGACTCCGTGAAGGGCC
GATTCACCATCTCCAGAGACAATTCCAAGAACACGCTGTATCTGCAAATGAA
 
CAGCCTGAGAGCTGAGGACACGGCTGTGTATTACTGTGCGAGAGTCCGCTAC
TATGGTTCGGGGAGTCCCATTGGGTACTGGGGCCAGGGAACCCTGGTCACCG
TCTCCTCA
   Amino acid Sequence (SEQ ID NO: 152) 
LPVLTQPPSASGTPGQRVTISCSGSSSNIGGNTVNWYHQLPGSAPKLLIYGDDLRP
SGVPDRFSGSKSGTSASLAISRFQSGDEADYYCAAWDDSLNAYVFGTGTKLTVL
GEGKSSGSGSDSKASEVQLVGSGGGVVQPGRSLRLSCAASGFTFSNYAMHWVR
QAPGKGLEWVAVISYDGSNKYYADSVKGRFTISRDNSKNTLYLQMNSLRAEDT
AVYYCARVRYYGSGSPIGYWGQGTLVTVSS
**Clone BMPR2.12 (Anti-BMPR2 (BMPRII))**
   Nucleotide Sequence (SEQ ID NO: 153) 
CAGTCTGCCCTGACTCAGCCTGCCTCCGTGTCTGGGTCTCCTGGACAGTCGAT
CACCATCTCCTGCACTGGAACCAGCAGTGACGTTGGTGGTTATAAGTCTGTCT
CCTGGTACCAACAACACCCAGGCAAAGCCCCCAAACTCATGATTTATGATGT
CAGTAATCGGCCCTCAGGGGTTTCTGATCGCTTCTCTGGCTCCAAGTCTGGCA
ACACGGCCTCCCTGACCATCTCTGGGCTCCAGGCTGAGGACGAGGCTGATTA
TTACTGCAGCTCTTACACAAGCAGCAGCTCCCTTTGGGTGTTCGGCGGAGGG
ACCAAGCTCACCGTCCTAGGAGAGGGTAAATCTTCCGGATCTGGTTCCGA
AAGCAAGGCTAGCCAGGTTCAGCTGGTGCAGTCTGGGGCTGAGGTGAAGAA
GCCTGGGTCCTCGGTGAAGGTCTCCTGCAAGGCTTCTGGAGGCACCTTCAGC
AGCTATGCTATCAGCTGGGTGCGACAGGCCCCTGGACAAGGGCTTGAGTGGA
TGGGAAGGATCATCCCTATCCTTGGTATAGCAAACTACGCACAGAAGTTCCA
GGGCAGAGTCACCATGACCGAGGACACATCTACAGACACAGCCTACATGGA
GCTGAGCAGCCTGAGATCTGAGGACACGGCCGTGTATTACTGTGCAACAGAT
CTGTGGGGAGTGGGAGCAGATTGGGGCCAGGGAACCCTGGTCACCGTCTCCT
CA
   Amino acid Sequence (SEQ ID NO: 154) 
QSALTQPASVSGSPGQSITISCTGTSSDVGGYKSVSWYQQHPGKAPKLMIYDVSN
RPSGVSDRFSGSKSGNTASLTISGLQAEDEADYYCSSYTSSSSLWVFGGGTKLTV
LGEGKSSGSGSESKASQVQLVQSGAEVKKPGSSVKVSCKASGGTFSSYAISWVR
 
QAPGQGLEWMGRIIPILGIANYAQKFQGRVTMTEDTSTDTAYMELSSLRSEDTAV
YYCATDLWGVGADWGQGTLVTVSS
**Clone BMPR2.23 (Anti-BMPR2 (BMPRII))**
   Nucleotide Sequence (SEQ ID NO:211) 
GACGACGATGACAAGCTTCAGGCTGTGCTCACTCAGCCGTCTTCCCTCTCTGC
ATCTCCTGGAGCTTCAGCCAGTCTCACCTGCACCTTACACAGTGGCATCAATG
TTGCTACCTACAGGATATACTGGTACCAGCAGAAGCCAGGGAGTCCTCCCCA
GTATCTGCTGACTTATAAGTCAGACTTAGATAAACATCAGGGGTCTGGAGTC
CCCAGTCGCTTCTCTGGATCCAAAGATGCGTCGGCCAATGCAGGCATATTGCT
CATTTCTGGACTCCAGTCTGACGACGAGGGTGACTATTATTGTTTAATCTGGC
ACGACTACGTCTGGTACTTCGGCGGTGGGACCAAGGTCACCGTCCTAGGTGA
GGGTAAATCGTCCGGATCTGGTTCCGAATCCAAAGCTAGCGAGGTGCAG
CTGTTGGAGTCTGGGGGAGGCTTGGTACAGCCTGGCAGGTCCCTGAGACTCT
CCTGTGCAGCCTCTGGATTCACCTTTGATGATTATGCCATGCACTGGGTCCGG
CAAGCTCCAGGGAAGGGCCTGGAGTGGGTCTCAGGTATTAGTTGGAATAGTG
GTAGCATAGGCTATGCGGACTCTGTGAAGGGCCGATTCACCATCTCCAGAGA
CAACGCCAAGAACTCCCTGTATCTGCAAATGAACAGTCTGAGAGCTGAGGAC
ACGGCTCTGTATTATTGTGCAAAAGGCGGGTGGGGCAGCTCGACCCACTACT
ACTACGGTATGGACGTCTGGGGCCAAGGGACCACGGTCACCGTCTCCTCAGT
CGACCCATTCGTTTCTGAATATCAAGGCCAATCGTCTGACCTGCCTCAACCTC
CTGTCAATGCTGGCGGCGGCTCTGANGGAGGCGGGTNCCGGTGGTGGCTCTG
GTTCCGG
   Amino acid Sequence (SEQ ID NO: 212) 
DDDDKLQAVLTQPSSLSASPGASASLTCTLHSGINVATYRIYWYQQKPGSPPQYL
LTYKSDLDKHQGSGVPSRFSGSKDASANAGILLISGLQSDDEGDYYCLIWHDYV
WYFGGGTKVTVLGEGKSSGSGSESKASEVQLLESGGGLVQPGRSLRLSCAASGF
TFDDYAMHWVRQAPGKGLEWVSGISWNSGSIGYADSVKGRFTISRDNAKNSLY
LQMNSLRAEDTALYYCAKGGWGSSTHYYYGMDVWGQGTTVTVSSVDPFVSEY
QGQSSDLPQPPVNAGGGSXGGGXRWWLWFR
**Clone B15: (Anti-ALK3 (BMPRIA))**
   Nucleotide Sequence (SEQ ID NO: 155) 
CTGCCTGTGCTGACTCAGCCTGCCTCCGTGTCTGGGTCTCCTGGACAGTCGAT
CACCTTCTCCTGCACTGGAACCAACACTGACATTGGTTATTATAATTATGTCT
CCTGGTACCAACAACTCCCAGGCAAAGCCCCCAAACTCTTGATTTTTGAGGTC
GATAATCGGCCCTCAGGGGTCCCTAGTCGCTTCTCTGGCTCCAAGTCTGGCAA
CACGGCCTCCCTGACCATCTCTGGGCTCCAGGCTGAGGACGAGGCTGATTAT
TACTGCAGCTCATATTCAACCTATAATATTTTGCTCTTCGGCGGAGGGACCAA
GCTCACCGTCCTAGGTGAGGGTAAATCTTCCGGATCTGGTTCCGAATCCA
AAGCTAGCGAGGTGCAGCTGTTGGAGTCTGAGGCTGAGGTGAAGAAGCCTG
GGGCCTCAGTGAAGGTCTCCTGCAAGGCTTCTGGATACACCTTCACCGGCTA
CTATATGCACTGGGTGCGACAGGCCCCTGGACAAGGGCTTGAGTGGATGGGA
CGGATCAACCCTAACAGTGGTGGCACAAACTATGCACAGAAGTTTCAGGGCA
GGGTCACCATGACCAGGGACACGTCCATCAGCACAGCCTACATGGAGCTGAG
CAGGCTGAGATCTGACGACACGGCCGTGTATTACTGTGCGAGAGACCCCACG
TATTACGATATTTTGACTGGTCCGGAGTACTGGGGCCAGGGAACCGTCACCG
TCTCCTCA
   Amino acid Sequence (SEQ ID NO:156) 
LPVLTQPASVSGSPGQSITFSCTGTNTDIGYYNYVSWYQQLPGKAPKLLIFEVDN
RPSGVPSRFSGSKSGNTASLTISGLQAEDEADYYCSSYSTYNILLFGGGTKLTVLG
EGKSSGSGSESKASEVQLLESEAEVKKPGASVKVSCKASGYTFTGYYMHWVRQ
APGQGLEWMGRINPNSGGTNYAQKFQGRVTMTRDTSISTAYMELSRLRSDDTA
VYYCARDPTYYDILTGPEYWGQGTVTVSS
**Clone B16: (Anti-ALK3 (BMPRIA))**
   Nucleotide Sequence (SEQ ID NO: 157) 
GAAATAGTGATGACACAGTCTCCAGCCACCCTGTCTGTGTCTCCAGGGGAAA
GAGCCACCCTCTCCTGCAGGGCCAGTCAGAGTGTTAGCAGCAACTTAGCCTG
GTACCAGCAGAAACCTGGCCAGGCTCCCAGGCTCCTCATCTATGGTGCATCC
AGCAGGGCCACTGGCATCCCAGACAGGTTCAGTGGCAGTGGGTCTGGGACAG
 
ACTTCACTCTCACCATCAGCAGACTGGAGCCTGAGGATTTTGCAGTGTATTAC
TGTCAGCAATATATTTACTCACCGTACACTTTTGGCCAGGGGACCAAAGTGG
ATATCAAAGAGGGTAAATCTTCCGGATCTGGTTCCGAATCCAAAGCTAGC
GAGGTGCAGCTGGTGCAGTCTGGGGCTGAGGTGAAGAAGCCTGGGGCCTCAG
TGAAGGTCTCCTGCAAGGCCTCTGGATACAACTTCGCCGGCTACTATGTTCAC
TGGGTGCGACAGGCCCCTGGACAAGGGCTTGAGTGGATGGGATGGATCAACC
CTAACAGTGGTGGCACAGACTATGCACAGAAGTTTCAGGGCAGGGTCACCAT
GACCAGGGACACGTCCATCAGCACAGCCTACATGGACCTGAGTAGGCTGAGA
TCTGACGACACGGCCGTCTATTACTGTGCGAGGAATCTACAGTTTTTTCGGCC
AGGGACCCTGGTCACCGTCTCCTCA
   Amino acid Sequence (SEQ ID NO:158) 
EIVMTQSPATLSVSPGERATLSCRASQSVSSNLAWYQQKPGQAPRLLIYGASSRA
TGIPDRFSGSGSGTDFTLTISRLEPEDFAVYYCQQYIYSPYTFGQGTKVDIKEGKS
SGSGSESKASEVQLVQSGAEVKKPGASVKVSCKASGYNFAGYYVHWVRQAPG
QGLEWMGWINPNSGGTDYAQKFQGRVTMTRDTSISTAYMDLSRLRSDDTAVYY
CARNLQFFRPGTLVTVSS
**Clone RI-2: (Anti-ALK2 (ACVR1))**
   Nucleotide Sequence (SEQ ID NO: 159) 
TCCTATGAGCTGACTCAGCCACTCTCAGTGTCAGTGGCCCTGGGACAGACGG
CCAGGATTACCTGTGGGGGAAACAACATTGGAAGTAAAAATGTGCACTGGTA
CCAGCAGAAGCCAGGCCAGGCCCCTGTGCTGGTCATCTATGATGATAGCGAC
CGGCCCTCAGGGACCTCCGAGCGATTCTCTGGCTCCAACTCTGGGAACACAG
CCACTCTGACCATCAGCGGGACCCAGGCTATGGATGAGGCTGACTATTACTG
TCAGGCGTGGGACAGCAGCACTGCAGTGGTATTCGGCGGAGGGACCAAGCTC
ACCGTCCTAGGTGAGGGTAAATCTTCCGGATCTGGTTCCGAATCCAAAGC
TAGCCAGGTGCAGCTGCAGGAGTCGGGCCCAGGACTGGTGAAGCCTTCGGA
GACCCTGTCCCTCACCTGCACTGTCTCTGGTGGCTCCATCAGCAGTAGTAGTT
ACTACTGGGGCTGGATCCGCCAGCCCCCAGGGAAGGGGCTGGAGTGGATTGG
GAGTATCTATTATAGTGGGAGCACCTACTACAACCCGTCCCTCAAGAGTCGA
GCCACCATATCCGTAGACACGTCCAAGAACCAGTTCTCCCTGAAGATGACCT
 
CAGTGACCGCCGCAGACACGGCCATATATTACTGTGCGAGACAGGTGGGCCC
TGTGTTAAAGTCGGTATCTCAGGGCACCCTGGTCACCGTCACCTCA
   Amino acid Sequence (SEQ ID NO:160) 
SYELTQPLSVSVALGQTARITCGGNNIGSKNVHWYQQKPGQAPVLVIYDDSDRP
SGTSERFSGSNSGNTATLTISGTQAMDEADYYCQAWDSSTAVVFGGGTKLTVLG
EGKSSGSGSESKASQVQLQESGPGLVKPSETLSLTCTVSGGSISSSSYYWGWIRQ
PPGKGLEWIGSIYYSGSTYYNPSLKSRATISVDTSKNQFSLKMTSVTAADTAIYYC
ARQVGPVLKSVSQGTLVTVTS
**Clone RI-3: (Anti-ALK2 (ACVR1))** *Note different linker sequence
   Nucleotide Sequence (SEQ ID NO: 161) 
GAAGTGCAGCTGCTGGAAAGCGGCGGTGGTCTGGTTCAGCCGGGTGGCAGCC
TGCGTCTGAGCTGTGCGGCGAGCGGCTTTACCTTTAGCAGCTATGCCATGAGC
TGGGTGCGTCAGGCACCGGGCAAAGGCCTGGAATGGGTGAGCGCGATTAGC
GGCAGCGGCGGCAGCACCTATTATGCGGATAGCGTGAAAGGCCGTTTTACCA
TTAGCCGTGATAACAGCAAAAACACCCTGTATCTGCAGATGAACAGCCTGCG
TGCGGAAGATACCGCGGTGTATTATTGCGCGAAAAGCGCCAGCTTCTTTGATT
ATTGGGGCCAGGGCACCCTGGTTACCGTTAGCAGCGGTGGAGGCGGTTCTG
GTGGAGGCGGTTCGGGTGACGGAAGTTCAGAAATTGTGCTGACCCAGAGC
CCGGGCACGCTGTCTCTGAGCCCGGGTGAACGTGCGACCCTGAGCTGTCGTG
CGAGCCAGAGCGTGAGCAGCAGCTATCTGGCCTGGTATCAGCAGAAACCGG
GCCAGGCACCGCGTCTGCTGATTTATGGCGCGAGCAGCCGTGCGACCGGCAT
TCCGGATCGTTTTAGCGGCAGCGGTAGCGGCACCGATTTTACCCTGACCATTA
GCCGTCTGGAACCGGAAGATTTTGCGGTGTATTATTGCCAGCAGCATTTCCGT
TGGCCGTGGACCTTTGGCCAGGGCACCAAAGTGGAAATTAAA
   Amino acid Sequence (SEQ ID NO:162) 
EVQLLESGGGLVQPGGSLRLSCAASGFTFSSYAMSWVRQAPGKGLEWVSAISGS
GGSTYYADSVKGRFTISRDNSKNTLYLQMNSLRAEDTAVYYCAKSASFFDYWG
QGTLVTVSSGGGGSGGGGSGDGSSEIVLTQSPGTLSLSPGERATLSCRASQSVSS
SYLAWYQQKPGQAPRLLIYGASSRATGIPDRFSGSGSGTDFTLTISRLEPEDFAVY
YCQQHFRWPWTFGQGTKVEIK
**Clone RI-4: (Anti-ALK2 (ACVR1))**
   Nucleotide Sequence (SEQ ID NO: 163) 
TCCTATGAGCTGACTCAGCCACCCTCAGCGTCTGGGACCCCCGGGCAGAAGG
TCACCATCTCTTGTTCTGGAAGCAGCTCCAACATCGGAAGTAATACTGTAAAC
TGGTACCAGCAGCTCCCAGGAACGGCCCCCAAACTCCTCATCTATAGTAATA
ATCAGCGGCCCTCAGGGGTCCCTGACCGATTCTCTGGCTCCAAGTCTGGCACC
TCAGCCTCCCTGGCCATCAGTGGGCTCCAGTCTGAGGATGAGGCTGATTATTA
CTGTGCAGCGTGGGATGACAGCCTGAATGGTGTGGTTTTCGGCGGAGGGACC
AAGGTGACCGTCCTAGGTGAGGGTAAATCTTCCGGATCTGGTTCCGAATC
CAAAGCTAGCCAGGTGCAGCTGCAGGAGTCGGGCCCAGGACTGGTGAAGCC
TTCACAGACCCTGTCCCTCACCTGCACTGTCTCTGGTGCCTCCATCAGCAGTG
GTGGTTACTACTGGAGCTGGATCCGCCAGCACCCAGGGAAGGGCCTGGAGTG
GATTGGGTACAGCTATTACAGTGGGAGCACCTACTACAACCCGTCCCTCAAG
AGTCGAGTTACCATATCAGTAGACACGTCCAAGAACCAGTTCTCCCTGTACCT
GACCTCTGTGACCGCTGCGGACACGGCCGTATATCATTGTGCGAGAGGGGGG
GTCAGGTTTGACCTCTGGGGCCAGGGCACCCTGGTCACCGTCTCCTCA
   Amino acid Sequence (SEQ ID NO:164) 
SYELTQPPSASGTPGQKVTISCSGSSSNIGSNTVNWYQQLPGTAPKLLIYSNNQRP
SGVPDRFSGSKSGTSASLAISGLQSEDEADYYCAAWDDSLNGVVFGGGTKVTVL
GEGKSSGSGSESKASQVQLQESGPGLVKPSQTLSLTCTVSGASISSGGYYWSWIR
QHPGKGLEWIGYSYYSGSTYYNPSLKSRVTISVDTSKNQFSLYLTSVTAADTAVY
HCARGGVRFDLWGQGTLVTVSS
**Clone RI-2-7 (Anti-ALK2 (ACVR1))**
   Nucleotide Sequence (SEQ ID NO: 165) 
TCCTATGAGCTGACTCAGCCACCCTCGGTGTCAGTGTCCCCAGGACAAACGG
CCAGGATCACCTGCTCTGGAGATGCATTGCCAAAAAAATATGCTTATTGGTA
CCAGCAGAAGTCAGGCCAGGCCCCTGTGCTGGTCATCTATGAGGACAGCAAA
CGACCCTCCGGGATCCCTGAGAGATTCTCTGGCTCCAGCTCAGGGACAATGG
CCACCTTGACTGTCAGTGGGGCCCAGGTGGAGGATGAAGCTGACTACTACTG
TTACTCAACAGACAGCAGTGGTAATCCAGTGTTCGGCGGAGGGACCAAGCTG
 
ACCGTCCTAGGTGAGGGTAAATCTTCCGGATCTGGTTCCGAATCCAAAGC
TAGCGAGGTGCAGCTGTTGGAGTCTGGGGGAGGCTTGGTACAGCCTGGGGG
GTCCCTGAGACTCTCCTGTGCAGCCTCTGGATTCACCTTTAGCAGCTATGCCA
TGAGCTGGGTCCGCCAGGCTCCAGGGAAGGGGCTGGAGTGGGTCTCAGCTAT
TAGTGGTAGTGGTGGTAGCACATACTACGCAGACTCCGTGAAGGGCCGGTTC
ACCATCTCCAGAGACAATTCCAAGAACACGCTGTATCTGCAAATGAACAGCC
TGAGAGCCGAGGACACGGCCGTATATTACTGTGCGAAAATTCCTTGGGGTGA
TTACGATTTTTGGAGTGGTTATTATACCAGGCCCGGTCACTCCGGTATGGACG
TCTGGGGCCAAGGCACCCTGGTCACCGTCTCCTCA
   Amino acid Sequence (SEQ ID NO:166) 
SYELTQPPSVSVSPGQTARITCSGDALPKKYAYWYQQKSGQAPVLVIYEDSKRPS
GIPERFSGSSSGTMATLTVSGAQVEDEADYYCYSTDSSGNPVFGGGTKLTVLGE
GKSSGSGSESKASEVQLLESGGGLVQPGGSLRLSCAASGFTFSSYAMSWVRQAP
GKGLEWVSAISGSGGSTYYADSVKGRFTISRDNSKNTLYLQMNSLRAEDTAVYY
CAKIPWGDYDFWSGYYTRPGHSGMDVWGQGTLVTVSS
**Clone RI-9: (Anti-ALK2 (ACVR1))**
   Nucleotide Sequence (SEQ ID NO: 167) 
CAGGCTGTGCTGACTCAGCCGCCCTCAGTGTCTGGGGCCCCAGGGCAGAGGG
TCACCATCTCCTGCGCTGGGAGCAGCTCCAACATCGGGGCAGGTTATGATGT
ACACTGGTACCAACAGCTTCCAGGAACAGCCCCCAAGCTCCTCATCTTTGAT
AGCTCCAATCGGCCCTCAGGGGTCCCTGACCGATTCTCTGGCTCCAAGTCCGG
CACCTCAGCCTCCCTGGCCATCACTGGGCTCCAGGCTGAGGATGAGGCTGAT
TATTACTGCCAGTCCTATGACTTCAGGCTGAGTGGTTCTATGGTCTTCGGCGG
AGGGACCAAGCTGACCGTCCTAGGTGAGGGTAAATCTTCCGGATCTGGTT
CCGAATCCAAAGCTAGCGAGGTGCAGCTGGTGGAGTCTGGGGGAGGCCTGG
TCAAGCCTGGGGGGTCCCTGAGACTCTCCTGTGCAGCCTCTGGATTCACCTTC
AGGGCCTATCCCATGAGCTGGGTCCGCCAGGCTCCAGGGAAGGGGCTGGAAT
GGGTCTCATCCATTACTTCTAGTGGCAGTTACTACGCAGACTCAGTGAAGGGT
CGATTTACCATCTCCAGAGACAACGCCAAGAGTTCACTGTATCTCCAAATGA
 
ACAGCCTGAGAGCCGACGACACGGCTGTCTATTACTGTGCGAGAGTGGGAGT
TACTGACTTTGACTACTGGGGCCAGGGAACCCTGGTCACCGTCTCCTCA
   Amino acid Sequence (SEQ ID NO:168) 
QAVLTQPPSVSGAPGQRVTISCAGSSSNIGAGYDVHWYQQLPGTAPKLLIFDSSN
RPSGVPDRFSGSKSGTSASLAITGLQAEDEADYYCQSYDFRLSGSMVFGGGTKLT
VLGEGKSSGSGSESKASEVQLVESGGGLVKPGGSLRLSCAASGFTFRAYPMSW
VRQAPGKGLEWVSSITSSGSYYADSVKGRFTISRDNAKSSLYLQMNSLRADDTA
VYYCARVGVTDFDYWGQGTLVTVSS
**Clone RII-19 (Anti-ACTRIIA (ACVR2A))**
   Nucleotide Sequence (SEQ ID NO: 169) 
CAGTCTGTGCTGACTCAGCCGCCCTCAGCGTCTGGGACCCCCGGGCAGAGGG
TCACCATCTCTTGTTCTGGAAGCAGCTCCAACATCGGAACTAATTATGTATAC
TGGTACCAGCAGCTCCCAGGAACGGCCCCCAAACTCCTCATCTATAGGAATA
ATCAGCGGCCCTCAGGGGCCCCTGACCGATTCTCTGGCTCCAAGTCTGGCAC
CTCAGCCTCCCTGGCCATCAGTGGGCTCCGGTCCGAGGATGAGGCTGATTATT
ACTGTGCAGCATGGGATGACAGCCTGAGTGGTTGGGTGTTCGGCGGAGGGAC
CAAGGTGACCGTCCTAGGTGAGGGTAAATCTTCCGGATCTGGTTCCGAAT
CCAAAGCTAGCGAGGTGCAGCTGTTGGAGTCTGGGGGAGGCTTGGTACAGC
CTGGGGGGTCCCTGAGACTCTCCTGTGCAGCCTCTGGATTCACCTTTAGCAGC
TATGCCATGAGCTGGGTCCGCCAGGCTCCAGGGAAGGGGCTGGAGTGGGTCT
CAGCTATTAGTGGTAGTGGTGGTAGCACATACTACGCAGACTCCGTGAAGGG
CCGGTTCACCATCTCCAGAGACAATTCCAAGAACACGCTGTATCTGCAAATG
AACAGCCTGAGAGCCGAGGACACGGCCGTATATTACTGTGCGAAAAGTATCA
GAACTGGGAGCGGGTACTTTGACTACTGGGGCCAGGGCACCCTGGTCACCGT
CTCCTCA
   Amino acid Sequence (SEQ ID NO:170) 
QSVLTQPPSASGTPGQRVTISCSGSSSNIGTNYVYWYQQLPGTAPKLLIYRNNQRP
SGAPDRFSGSKSGTSASLAISGLRSEDEADYYCAAWDDSLSGWVFGGGTKVTVL
GEGKSSGSGSESKASEVQLLESGGGLVQPGGSLRLSCAASGFTFSSYAMSWVRQ
 
APGKGLEWVSAISGSGGSTYYADSVKGRFTISRDNSKNTLYLQMNSLRAEDTAV
YYCAKSIRTGSGYFDYWGQGTLVTVSS
**Clone H1-2 (Anti-Activin RIIA (ACVR2A))**
   Nucleotide Sequence (SEQ ID NO: 171) 
CAGTCTGTGCTGACGCAGCCGCCCTCAGCGTCTGGGACCCCCGGGCAGAGGG
TCACCATCTCTTGTTCTGGAAGCAGCTCCAACATCGGAAGTAATTATGTATAC
TGGTACCAGCAGCTCCCAGGAACGGCCCCCAAACTCCTCATCTATAGGAATA
ATCAGCGGCCCTCAGGGGTCCCTGACCGATTCTCTGGCTCCAAGTCTGGCACC
TCAGCCTCCCTGGCCATCAGTGGGCTCCGGTCCGAGGATGAGGCTGATTATTA
CTGTGCAGCATGGGATGACAGCCTGAGTGGTTGGGTGTTCGGCGGAGGGACC
AAGCTCACCGTCCTAGGTGAGGGTAAATCTTCCGGATCTGGTTCCGAATC
CAAAGCTAGCCAGGTTCAGCTGCAGTCTGGGGCTGAGGTGAAGAAGCCTGG
GGCCTCAGTGAAGGTTTCCTGCAAGGCATCTGGATACACCTTCACCAGCTACT
ATATGCACTGGGTGCGACAGGCCCCTGGACAAGGGCTTGAGTGGATGGGAAT
AATCAACCCTAGTGGTGGTAGCACAAGCTACGCACAGAAGTTCCAGGGCAGA
GTCACCATGACCAGGGACACGTCCACGAGCACAGTCTACATGGAGCTGAGCA
GCCTGAGATCTGAGGACACGGCCGTGTATTACTGTGCGAGAGATCTCCTCCTC
TATGGTCGCGGGGGCGGTTTCCAGCACTGGGGCCAGGGAACCCTGGTCACCG
TCTCCTCA
   Amino acid Sequence (SEQ ID NO:172) 
QSVLTQPPSASGTPGQRVTISCSGSSSNIGSNYVYWYQQLPGTAPKLLIYRNNQRP
SGVPDRFSGSKSGTSASLAISGLRSEDEADYYCAAWDDSLSGWVFGGGTKLTVL
GEGKSSGSGSESKASQVQLQSGAEVKKPGASVKVSCKASGYTFTSYYMHWVR
QAPGQGLEWMGIINPSGGSTSYAQKFQGRVTMTRDTSTSTVYMELSSLRSEDTA
VYYCARDLLLYGRGGGFQHWGQGTLVTVSS
**Clone H2-11 (Anti-Activin RIIB (ACVR2B))**
   Nucleotide Sequence (SEQ ID NO: 173) 
TCTTCTGAGCTGACTCAGGACCCTGCTGTGTCTGTGGCCTTAGGACAGACAGT
CAGGATCACATGCCAAGGAGACAGCCTCAGAAGCTATTATGCAAGCTGGTAC
CAGCAGAAGCCAGGACAGGCCCCTGTACTTGTCATCTATGGTAAAAACAACC
 
GGCCCTCAGGGATCCCAGCCCGATTCTCTGGCTCCAGCTCAGGAAACACAGC
TTCCTTGACCATCACTGGGGCTCAGGCGGAAGATGAGGCTGACTATTACTGT
AACTCCCGGGACAACAGTGGTAAGCATCTTTGGGTGTTCGGCGGAGGGACCA
AGCTCACCGTCCTAGGTGAGGGTAAATCTTCCGGATCTGGTTCCGAATCC
AAAGCTAGCGAGGTGCAGCTGGTGCAGTCTGGGGGAGGTGTGGTACGGCCT
GGGGGGTCCCTGAGACTCTCCTGTGCAGCCTCTGGATTCACCTTTGATGATTA
TGGCATGAGCTGGGTCCGCCAAGCTCCAGGGAAGGGGCTGGAGTGGGTCTCT
GGTATTAATTGGAATGGTGGTAGCACAGGTTATGCAGACTCTGTGAAGGGCC
GATTCACCATCTCCANAGACAACGCCAAGAACTCCCTGTATCTGCAAATGAA
CAGTCTGAGAGCCGAGGACACGGCCTTGTATTACTGTGCGAGGAAATCTACA
GGTGACTACTGGGGCCAGGGAACCCTGGTCACCGTCTCTTCA
   Amino acid Sequence (SEQ ID NO:174) 
SSELTQDPAVSVALGQTVRITCQGDSLRSYYASWYQQKPGQAPVLVIYGKNNRP
SGIPARFSGSSSGNTASLTITGAQAEDEADYYCNSRDNSGKHLWVFGGGTKLTVL
GEGKSSGSGSESKASEVQLVQSGGGVVRPGGSLRLSCAASGFTFDDYGMSWVR
QAPGKGLEWVSGINWNGGSTGYADSVKGRFTISTDNAKNSLYLQMNSLRAEDT
ALYYCARKSTGDYWGQGTLVTVSS
**Clone H2-15 (Anti-Activin RIIB (ACVR2B))**
   Nucleotide Sequence (SEQ ID NO: 175) 
CAGTCTGTGCTGACGCAGCCGCCCTCAGTGTCTGGGGCCCCAGGGCAGAGGG
TCACCATCTCCTGCACTGGGAGCAGCTCCAACATCGGGGCAGGTTATGATGT
ACACTGGTACCAGCAGCTTCCAGGAACAGCCCCCAAACTCCTCATCTATGGT
AACAGCAATCGGCCCTCAGGGGTCCCTGACCGATTCTCTGGCTCCAAGTCTG
GCACCTCAGCCTCCCTGGCCATCAGTGGGCTCCAGTCTGAGGATGAGGCTGA
TTATTACTGTGCAGCATGGGATGACAGCCTGAATGGTCCGAGAATGGTATTC
GGCGGAGGGACCAAGCTGACCGTCCTAGGTGAGGGTAAATCTTCCGGATCT
GGTTCCGAATCCAAAGCTAGCGAGGTGCAGCTGGTGCAGTCTGGGGGAGG
CTTGGTCAAGCCTGGAGGGTCCCTGAGACTCTCCTGTGCAGCCTCTGGATTCA
CCTTCAGTGACTACTACATGAGCTGGATCCGCCAGGCTCCAGGGAAGGGGCT
GGAGTGGGTTTCATACATTAGTAGTAGTAGTAGTTACACAAACTACGCAGAC
 
TCTGTGAAGGGCCGATTCACCATCTCCAGAGACAACGCCAAGAACTCACTGT
ATCTGCAAATGAACAGCCTGAGAGCCGAGGACACGGCTGTGTATTACTGTGC
GAGAGTTAACTACTACTACGGTATGGACGTCTGGGGCCAAGGCACCCTGGTC
ACCGTCTCTTCA
   Amino acid Sequence (SEQ ID NO:176) 
QSVLTQPPSVSGAPGQRVTISCTGSSSNIGAGYDVHWYQQLPGTAPKLLIYGNSN
RPSGVPDRFSGSKSGTSASLAISGLQSEDEADYYCAAWDDSLNGPRMVFGGGTK
LTVLGEGKSSGSGSESKASEVQLVQSGGGLVKPGGSLRLSCAASGFTFSDYYMS
WIRQAPGKGLEWVSYISSSSSYTNYADSVKGRFTISRDNAKNSLYLQMNSLRAE
DTAVYYCARVNYYYGMDVWGQGTLVTVSS
**Clone H5-2 (Anti-ALK4 (Activin RIB))**
   Nucleotide Sequence (SEQ ID NO: 177) 
TCTTCTGAGCTGACTCAGGACCCTGCTGTGTCTGTGGCCTTGGGACAGACAGT
CAGAATCACATGCCAAGGAGACAGCCTCAGAAAATATTATGCAAGCTGGTAC
CAGCAGAAGGCAGGACAGGCCCCTGTACTTGTCATCTATGGTAAAAACAAAC
GGCCCTCAGGGATCCCAGACCGATTCTCTGGCTCCAGCTCAGGAAACACAGC
TTCCTTGACCATCACTGGGGCTCAGGCGGAAGATGAGGCTGACTATTACTGT
AACTCCTGGGACAGCAGTGGTAACCACGTGGTATTCGGCGGAGGGACCAAGC
TCACCGTCCTAGGTGAGGGTAAATCTTCCGGATCTGGTTCCGAATCCAAA
GCTAGCCAGGTGCAGCTGCAGGAGTCGGGCCCAGGACTGGTGAAGCCTTCA
CAGACCCTGTCCCTCACCTGCACTGTCTCTGGTGGCTCCATCAGCAGTGGTGG
TTACTACTGGAGCTGGATCCGCCAGCACCCAGGGAAGGGCCTGGAGTGGATT
GGGTACATCTATTACAGTGGGAGCACCTACTACAACCCGTCCCTCAAGGGTC
GAGTTACCATATCAGTAGACACGTCTAAGAACCAGTTCTCCCTGAAGCTGAG
CTCTGTGACTGCCGCGGACACGGCCGTGTATTACTGTGCGAGAGAAGAAGTG
GGAGACTGGTTCGACCCCTGGGGCCAAGGCACCCTGGTCACCGTCTCCTCA
   Amino acid Sequence (SEQ ID NO:178) 
SSELTQDPAVSVALGQTVRITCQGDSLRKYYASWYQQKAGQAPVLVIYGKNKR
PSGIPDRFSGSSSGNTASLTITGAQAEDEADYYCNSWDSSGNHVVFGGGTKLTVL
GEGKSSGSGSESKASQVQLQESGPGLVKPSQTLSLTCTVSGGSISSGGYYWSWIR
 
QHPGKGLEWIGYIYYSGSTYYNPSLKGRVTISVDTSKNQFSLKLSSVTAADTAVY
YCAREEVGDWFDPWGQGTLVTVSS
**Clone H3-2 (Anti-ALK6 (BMPR1B))**
   Nucleotide Sequence (SEQ ID NO:179) 
TCTTCTGAGCTGACTCAGGACCCTGCTGTGTCTGTGGCCTTGGGACAGACAGT
CAGAATCACATGCCAAGGAGACAGCCTCAGAAAATATTATGCAAGCTGGTAC
CAGCAGAAGGCAGGACAGGCCCCTGTACTTGTCATCTATGGTAAAAGCAAAC
GGCCCTCAGGGATCCCAGACCGATTCTCTGGCTCCAGCTCAGGAAACACAGC
TTCCTTGACCATCACTGGGGCTCAGGCGGAAGATGAGGCTGACTATTACTGT
AACTCCTGGGACAGCAGTGGTAACCACGTGGTATTCGGCGGAGGGACCAAGC
TCACCGTCCTAGGTGAGGGTAAATCTTCCGGATCTGGTTCCGAATCCAAA
GCTAGCCAGGTGCAGCTGCAGGAGTCGGGCCCAGGACTGGTGAAGCCTTCA
CAGACCCTGTCCCTCACCTGCACTGTCTCTGGTGGCTCCATCAGCAGTGGTGG
TTACTACTGGAGCTGGATCCGCCAGCACCCAGGGAAGGGCCTGGAGTGGATT
GGGTACATCTATTACAGTGGGAGCACCTACTACAACCCGTCCCTCAAGGGTC
GAGTTACCATATCAGTAGACACGTCTAAGAACCAGTTCTCCCTGAAGCTGAG
CTCTGTGACTGCCGCGGACACGGCCGTGTATTACTGTGCGAGAGAAGAAGTG
GGAGACTGGTTCGACCCCTGGGGCCAGGGCACCCTGGTCACCGTCTCCTCA
   Amino acid Sequence (SEQ ID NO:180) 
SSELTQDPAVSVALGQTVRITCQGDSLRKYYASWYQQKAGQAPVLVIYGKSKRP
SGIPDRFSGSSSGNTASLTITGAQAEDEADYYCNSWDSSGNHVVFGGGTKLTVLG
EGKSSGSGSESKASQVQLQESGPGLVKPSQTLSLTCTVSGGSISSGGYYWSWIRQ
HPGKGLEWIGYIYYSGSTYYNPSLKGRVTISVDTSKNQFSLKLSSVTAADTAVYY
CAREEVGDWFDPWGQGTLVTVSS
**Clone H4-1 (Anti-ALK7 (ACVR1C))**
   Nucleotide Sequence (SEQ ID NO:181) 
CAGTCTGTGCTGACTCAGCCGCCCTCAGCGTCTGGGACCCCCGGGCAGAGGG
TCACCATCTCTTGTTCTGGAAGCAGCTCCAACATCGGAAGTAATTACGTATAC
TGGTACCAGCAGCTCCCAGGAACGGCCCCCAAACTCCTCATCTATAGGAATA
ATCAGCGGCCCTCAGGGGTCCCTGACCGATTCTCTGGCTCCAAGTCTGGCACC
 
TCAGCCTCCCTGGCCATCAGTGGGCTCCGGTCCGAGGATGAGGCTGATTATTA
CTGTGCAGCATGGGATGACAGCCTGAGTGGTGTGGTATTCGGCGGAGGGACC
AAGCTGACCGTCCTAGGTGAGGGTAAATCCTCCGGATCTGGTTCCGAATC
CAAAGCTAGCCAGGTTCAGCTGCAGTCTGGGGCTGAGGTGAAGAAGCCTGG
GGCCTCAGTGAAGGTCTCCTGCAAGGCTTCTGGATACACCTTCACCGGCTACT
ACATGCACTGGGTGCGACAGGCCCCTGGACAAGGGCTTGAGTGGATGGGATG
GATCAACCCTAACGGTGGTGGCACAAAGTATGCACAGAAGTTTCAGGGCAGG
GTCACCATGACCAGGGACACGTCCATCAGCACAGCCTACATGGAGCTGAGCA
GTCTGAGATCTGACGACACGGCCGTGTATTACTGTGCGACGCTTTCTGACTAT
AGCAGTGGCGGACCCTGGGGCCAGGGCACCCTGGTCACCGTCTCCTCA
   Amino acid Sequence (SEQ ID NO:182) 
QSVLTQPPSASGTPGQRVTISCSGSSSNIGSNYVYWYQQLPGTAPKLLIYRNNQRP
SGVPDRFSGSKSGTSASLAISGLRSEDEADYYCAAWDDSLSGVVFGGGTKLTVL
GEGKSSGSGSESKASQVQLQSGAEVKKPGASVKVSCKASGYTFTGYYMHWVR
QAPGQGLEWMGWINPNGGGTKYAQKFQGRVTMTRDTSISTAYMELSSLRSDDT
AVYYCATLSDYSSGGPWGQGTLVTVSS
**Clone H4-4 (Anti-ALK7 (ACVR1C))**
   Nucleotide Sequence (SEQ ID NO:183) 
CAGGCTGTGCTGACTCAGCCACCCTCAGCGTCTGGGACCCCCGGGCAGAGGG
TCACCATCTCTTGTTCTGGAAGCAGCTCCAACATCGGAAGTAATTATGTATAC
TGGTACCAGCAGCTCCCAGGAACGGCCCCCAAACTCCTCATCTATAGGAATA
ATCAGCGGCCCTCAGGGGTCCCTGACCGATTCTCTGGCTCCAAGTCTGGCACC
TCAGCCTCCCTGGCCATCAGTGGGCTCCGGTCCGAGGATGAGGCTGATTATTA
CTGTGCAGCATGGGATGACAGCCTGAGTGGTGTGGTATTCGGCGGAGGGACC
AAGCTCACCGTCCTAGGTGAGGGTAAATCTTCCGGATCTGGTTCCGAATC
CAAAGCTAGCGAGGTGCAGCTGGTGCAGTCTGGGACTGAGGTGAAGAGGCC
TGGGGCCTCAGTGAAGGTCTCCTGCAAGGCTTCTGGATACACCTTCACCGGCT
ACCATGTGCACTGGGTGCGACAGGCCCCTGGACAAGGGCTTGAGTGGATGGG
GTGGATCAACCCTGACAGTGGTGGCACAAACTTTGCACAGAAGTTTCAGGGC
AGGGTCACCATGACCAGGGACACGTCCATCAGCACAGCCTACATGGAACTTA
 
CCAGCCTGAGATCTGACGACACGGCCATTTATTACTGTGCGAGACGTTCACTT
GACTACTGGGGCCAGGGCACCCTGGTCACCGTCTCCTCA
   Amino acid Sequence (SEQ ID NO: 184) 
QAVLTQPPSASGTPGQRVTISCSGSSSNIGSNYVYWYQQLPGTAPKLLIYRNNQR
PSGVPDRFSGSKSGTSASLAISGLRSEDEADYYCAAWDDSLSGVVFGGGTKLTVL
GEGKSSGSGSESKASEVQLVQSGTEVKRPGASVKVSCKASGYTFTGYHVHWVR
QAPGQGLEWMGWINPDSGGTNFAQKFQGRVTMTRDTSISTAYMELTSLRSDDT
AIYYCARRSLDYWGQGTLVTVSS
**Clone H4-7 (Anti-ALK7 (ACVR1C))**
   Nucleotide Sequence (SEQ ID NO:185) 
TCCTATGAGCTGACACAGCCACCCTCAGCGTCTGGGACCCCCGGGCAGAGGG
TCACCATCTCTTGTTCTGGAAGCAGCTCCAACATCGGAAGTAATTATGTATAC
TGGTACCAGCAGCTCCCAGGAACGGCCCCCAAACTCCTCATCTATAGGAATA
ATCAGCGGCCCTCAGGGGTCCCTGACCGATTCTCTGCCTCCAAGTCTGGCACC
TCAGCCTCCCTGGCCATCAGTGGGCTCCAGTCTGCGGATGAGGCTGATTATTA
CTGTGCAGCATGGGATGACAGCCTGAATGGTGTGGTATTCGGCGGAGGGACC
AAGCTGACCGTCCTAGGTGAGGGTAAATCTTCCGGATCTGGTTCCGAATC
CAAAGCTAGCGAGGTGCAGCTGGTGCAGTCTGGGGGAGGCTTGGTCCAGCC
TGGGGGGTCCCTTAGACTCTCCTGTGCAGCCTCTGGATTCACTTTCAGTAACG
CCTGGATGAGCTGGGTCCGCCAGGCTCCAGGGAAGGGGCTGGAGTGGGTTGG
CCGTATTAAAAGCAAAACTGATGGTGGGACAACAGACTACGCTGCACCCGTG
AAAGGCAGATTCACCATCTCAAGAGATGATTCAAAAAACACGCTGTATCTGC
AAATGAACAGCCTGAAAACCGAGGACACAGCCGTGTATTACTGTGCTAGCCT
GGTTGACTACTGGGGCCAGGGCACCCTGGTCACCGTCTCCTCA
   Amino acid Sequence (SEQ ID NO:186) 
SYELTQPPSASGTPGQRVTISCSGSSSNIGSNYVYWYQQLPGTAPKLLIYRNNQRP
SGVPDRFSASKSGTSASLAISGLQSADEADYYCAAWDDSLNGVVFGGGTKLTVL
GEGKSSGSGSESKASEVQLVQSGGGLVQPGGSLRLSCAASGFTFSNAWMSWVR
QAPGKGLEWVGRIKSKTDGGTTDYAAPVKGRFTISRDDSKNTLYLQMNSLKTED
TAVYYCASLVDYWGQGTLVTVSS
**Clone H4-8 (Anti-ALK7 (ACVR1C))**
   Nucleotide Sequence (SEQ ID NO:187) 
CAGTCTGTGCTGACTCAGCCGCCCTCAGCGTCTGGGACCCCCGGGCAGAGGG
TCACCATCTCTTGTTCTGGAAGCAGCTCCAACATCGGAAGTAATTATGTATAC
TGGTACCAGCAGCTCCCAGGAACGGCCCCCAAACTCCTCATCTATAGGAATA
ATCAGCGGCCCTCAGGGGTCCCTGACCGATTCTCTGGCTCCAAGTCTGGCACC
TCAGCCTCCCTGGCCATCAGTGGGCTCCGGTCCGAGGATGAGGCTGATTATTA
CTGTGCAGCATGGGATGACAGCCTGAGTGGTCCGGTGTTCGGCGGAGGGACC
AAGGTGACCGTCCTAGGTGAGGGTAAATCTTCCGGATCTGGTTCCGAATC
CAAAGCTAGCGAGGTGCAGCTGGTGCAGTCTGGGGCTGTTGTGAAGAAGCC
TGGGGCCTCAGTGAAGGTCTCCTGCAAGGCTTCTGGCTACACCTTTACCAAGT
ATGGTATCAGCTGGGTGCGACAGGCCCCTGAACAAGGGCTTGAGTGGATGGG
ATGGATCAGCGCTTACAATGTTGACACAAAGTATGCACAGAAGTTCCAGGGC
AGAGTCACCATGACCACAGACACATCCACGAGCACAGCCTACATGGAACTGA
GGAGCCTTAGATCTGACGACACGGCCGTGTATTACTGTGCGAGAAGAGGGCT
TGACTACTGGGGCCAGGGCACCCTGGTCACCGTCTCCTCA
   Amino acid Sequence (SEQ ID NO:188)

QSVLTQPPSASGTPGQRVTISCSGSSSNIGSNYVYWYQQLPGTAPKLLIYRNNQRP
SGVPDRFSGSKSGTSASLAISGLRSEDEADYYCAAWDDSLSGPVFGGGTKVTVL
GEGKSSGSGSESKASEVQLVQSGAVVKKPGASVKVSCKASGYTFTKYGISWVR
QAPEQGLEWMGWISAYNVDTKYAQKFQGRVTMTTDTSTSTAYMELRSLRSDDT
AVYYCARRGLDYWGQGTLVTVSS

### Example 2. Bispecific Constructs.

Several examples of bispecific constructs were made from scFv clones binding to ACVRL1 (ACVRL1.8) and BMPR2 (BMPR2.12). These intermediate complexes were made to generate bispecific molecules against ALK1/ACVRL1 and BMPR2. These complexes were made by either expressing two scFv as a single molecule separated by a linker, and with a C-terminal His Tag, or alternatively by expressing each scFv separately as Fc-fusion molecules, and then combining into one scFv-Fc heterodimer.

Neither the single molecule bispecifics or the heterodimeric bispecifics had significant BMP transcriptional activity on their own in an in vitro assay; see FIG. 8. Rather than having biological activity as single molecule bi-specifics, these molecules had activity when they were linked as tetramers, using Ni++, or Ca++, or anti-His to join the His tags thereby forming heterotetramers. These results show that a heterotetrameric artificial ligand engages signaling in this pathway; this discovery is key to making these BMP mimetic signaling molecules work.
**Clone ACVRL1.8 (anti-ACVRL1)**
   Amino acid Sequence: 
DIQMTQSPSSLSASVGDRVTITCRASQSISSYLNWYQQKPGKAPKLLIYAASSLQS
GVPSRFSGSGSGTDFTLTISSLQPEDFATYYCQQSYSTPRTFGQGTKVDIKEGKSS
GSGSESKASQVQLQESGPGLVKPSQTLSLTCTVSGGSISSDDYYWSWIRQTPGKG
LEWIGYIYYSGITYYNPSLKSRVTISVDTSKNQFSLKLSSVTAADTAVYYCAREG
CNDGVCYNGVFDYWGQGTLVTVSS (SEQ ID NO:150)
**Clone BMPR2.12 (anti-BMPR2)**
   Amino acid Sequence 
QSALTQPASVSGSPGQSITISCTGTSSDVGGYKSVSWYQQHPGKAPKLMIYDVSN
RPSGVSDRFSGSKSGNTASLTISGLQAEDEADYYCSSYTSSSSLWVFGGGTKLTV
LGEGKSSGSGSESKASQVQLVQSGAEVKKPGSSVKVSCKASGGTFSSYAISWVR
QAPGQGLEWMGRIIPILGIANYAQKFQGRVTMTEDTSTDTAYMELSSLRSEDTAV
YYCATDLWGVGADWGQGTLVTVSS (SEQ ID NO:154)
**Bispecific Constructs:**
   **BMPR2.12-Medium-ACVRL1.8-His**
      Amino acid Sequence: 
GDEMGTGTQSALTQPASVSGSPGQSITISCTGTSSDVGGYKSVSWYQQHPGKAP
KLMIYDVSNRPSGVSDRFSGSKSGNTASLTISGLQAEDEADYYCSSYTSSSSLWV
FGGGTKLTVLGEGKSSGSGSESKASQVQLVQSGAEVKKPGSSVKVSCKASGGTF
SSYAISWVRQAPGQGLEWMGRIIPILGIANYAQKFQGRVTMTEDTSTDTAYMELS
 
SLRSEDTAVYYCATDLWGVGADWGQGTLVTVSSSGGGGSGGGGSSGSGGGG
DGGGGSGGTDIQMTQSPSSLSASVGDRVTITCRASQSISSYLNWYQQKPGKAPK
LLIYAASSLQSGVPSRFSGSGSGTDFTLTISSLQPEDFATYYCQQSYSTPRTFGQGT
KVDIKEGKSSGSGSESKASQVQLQESGPGLVKPSQTLSLTCTVSGGSISSDDYYW
SWIRQTPGKGLEWIGYIYYSGITYYNPSLKSRVTISVDTSKNQFSLKLSSVTAADT
AVYYCAREGCNDGVCYNGVFDYWGQGTLVTVSSSGGGGSHHHHHHHH* (SEQ
ID NO:189)
   **ACVRL1.8-Long-BMPR2.12-His**
      Amino acid Sequence: 
GDEMGTGTDIQMTQSPSSLSASVGDRVTITCRASQSISSYLNWYQQKPGKAPKLL
IYAASSLQSGVPSRFSGSGSGTDFTLTISSLQPEDFATYYCQQSYSTPRTFGQGTK
VDIKEGKSSGSGSESKASQVQLQESGPGLVKPSQTLSLTCTVSGGSISSDDYYWS
WIRQTPGKGLEWIGYIYYSGITYYNPSLKSRVTISVDTSKNQFSLKLSSVTAADTA
VYYCAREGCNDGVCYNGVFDYWGQGTLVTVSSSGGSGGGGSSGGGGSGGGG
SSGGGGDGGGGSGGTQSALTQPASVSGSPGQSITISCTGTSSDVGGYKSVSWYQ
QHPGKAPKLMIYDVSNRPSGVSDRFSGSKSGNTASLTISGLQAEDEADYYCSSYT
SSSSLWVFGGGTKLTVLGEGKSSGSGSESKASQVQLVQSGAEVKKPGSSVKVSC
KASGGTFSSYAISWVRQAPGQGLEWMGRIIPILGIANYAQKFQGRVTMTEDTSTD
TAYMELSSLRSEDTAVYYCATDLWGVGADWGQGTLVTVSSSSGGGGSHHHHH
HHH* (SEQ ID NO:190)
   **BMPR2.12-Long-ACVRL1.8-His**
      Amino acid Sequence:

GDEMGTGTQSALTQPASVSGSPGQSITISCTGTSSDVGGYKSVSWYQQHPGKAP
KLMIYDVSNRPSGVSDRFSGSKSGNTASLTISGLQAEDEADYYCSSYTSSSSLWV
FGGGTKLTVLGEGKSSGSGSESKASQVQLVQSGAEVKKPGSSVKVSCKASGGTF
SSYAISWVRQAPGQGLEWMGRIIPILGIANYAQKFQGRVTMTEDTSTDTAYMELS
SLRSEDTAVYYCATDLWGVGADWGQGTLVTVSSSGGGGSGGGGSSGGGGSG
GGGSSGGGGDGGGGSGGTDIQMTQSPSSLSASVGDRVTITCRASQSISSYLNW
YQQKPGKAPKLLIYAASSLQSGVPSRFSGSGSGTDFTLTISSLQPEDFATYYCQQS

YSTPRTFGQGTKVDIKEGKSSGSGSESKASQVQLQESGPGLVKPSQTLSLTCTVS
GGSISSDDYYWSWIRQTPGKGLEWIGYIYYSGITYYNPSLKSRVTISVDTSKNQFS
LKLSSVTAADTAVYYCAREGCNDGVCYNGVFDYWGQGTLVTVSSSGGGGSHH
HHHHH* (SEQ ID NO:191)

### Example 3. Tetravalent Constructs.

Tetravalent constructs were made from scFv clones binding to ACVRL1 (ACVRL1.8) and BMPR2 (BMPR2.12). These were designed to be tetravalent complexes expressed as a single molecule, with spacers of varying lengths as specified. Alternatively, they were designed as tetravalent IgG-Fc proteins generated by two bispecific scFvs expressed as an IgG Fc fusion protein, then associated as two disulfide linked homodimeric Fc complexes.

These constructs were able to generate potent BMP signaling in cells (see FIG. 8) Tetravalent complexes of anti-ACVRL1 and anti-BMPR2 elicited BMP transcriptional activity in endothelial cells. TIME cells (human telomerase immortalized microvascular endothelial cells) were cultured and transiently transfected with plasmid expressing the BMP response element (BRE-luciferase) reporter. BMP9 (1 ng/mL) elicited BMP transcriptional activity in these cells, based on the detection of luciferase activity, divided by the relative number of cells, based on an MTS colorimetric viability assay (RLU/viability). A bispecific construct consisting of a single anti-ACVRL1 scFv and a single anti-BMPR2 scFv joined by a spacer, and with a C-terminal His tag domain ("ACVRL1.8-L-BMPR2.12-His") did not elicit signaling activity by itself, but was active when combined with monoclonal anti-His antibodies to complex these bispecific molecules into a tetravalent complex. To demonstrate the signaling of a tetravalent complex when expressed as a disulfide linked heterotetramer, a tetravalent complex consisting of the same bispecific molecule expressed as an IgG Fc domain fusion protein and assembled into disulfide linked homodimers ("ACVRL1.8-L-BMPR2.12-Short-Fc") elicited potent signaling.

Consistent with in vitro signaling activity, in vivo signaling BMP signaling activity was demonstrated (Fig. 9) based on Id1 gene transcriptional activity assayed in whole lung tissues from 8-week old mice 24 hours after tail vein injection of saline (50 uL), recombinant human BMP9 (rhBMP9, 150 ug/kg in 50 uL), or recombinant tetrameric Fc fusion protein ACVRL1.8-L-BMPR2.12-Short-Fc (150 ug/kg in 50 uL). The injection of BMP9 or ACVRL1.8-L-BMPR2.12-Short-Fc elicited an increase in BMP transcriptional activity as compared to saline in whole lung tissues.

Demonstration of such signaling in vivo is accomplished by injecting recombinant signaling proteins such as ACVRL1.8-L-BMPR2.12-Short-Fc or similar molecules intravenously, intramuscularly, or subcutaneously into a suitable animal model such as a mouse or a rat. After 30 min to 24 hours, the impact of these treatments versus saline on the BMP signaling activity is assayed by performing immunohistochemistry on animal tissues for the phosphorylation of SMAD1/5/9 effector proteins, or by Western blotting of tissue extracts for phosphorylated SMAD1/5/9, or by assaying for the expression of ID1, ID2, and/or ID3 in target tissues by quantitative RT-PCR, or by assaying the expression of GFP in a transgenic reporter mouse expression GFP under the regulation of the BMP transcriptional response element (Monteiro et al., Genesis. 2008 Jul;46(7):335-46).

### Sequences of Exemplary Tetravalent constructs

scFv clone BMPR2.12 (anti-BMPR2): 
QSALTQPASVSGSPGQSITISCTGTSSDVGGYKSVSWYQQHPGKAPKLMIYDVSN
RPSGVSDRFSGSKSGNTASLTISGLQAEDEADYYCSSYTSSSSLWVFGGGTKLTV
LGEGKSSGSGSESKASQVQLVQSGAEVKKPGSSVKVSCKASGGTFSSYAISWVR
QAPGQGLEWMGRIIPILGIANYAQKFQGRVTMTEDTSTDTAYMELSSLRSEDTAV
YYCATDLWGVGADWGQGTLVTVSS (SEQ ID NO:154)
A = scFv clone ACVRL1.8 (anti-ACVRL1): 
DIQMTQSPSSLSASVGDRVTITCRASQSISSYLNWYQQKPGKAPKLLIYAASSLQS
GVPSRFSGSGSGTDFTLTISSLQPEDFATYYCQQSYSTPRTFGQGTKVDIKEGKSS
GSGSESKASQVQLQESGPGLVKPSQTLSLTCTVSGGSISSDDYYWSWIRQTPGKG
LEWIGYIYYSGITYYNPSLKSRVTISVDTSKNQFSLKLSSVTAADTAVYYCAREG
CNDGVCYNGVFDYWGQGTLVTVSS (SEQ ID NO:150)
M = medium spacer between scFv clones within a bivalent complex:
   SGGGGSGGGGSSGSGGGGDGGGGSG (SEQ ID NO:192)
   L = long spacer between ScFv clones within a bivalent complex:
      SGGSGGGGSSGGGGSGGGGSSGGGGDGGGGSG (SEQ ID NO:193)
   short = short spacer before IgG Fc domain for homodimeric Fc molecules:
      GSGGGGDGGGGSG (SEQ ID NO:194)
   medium = medium spacer before IgG Fc domain for homodimeric Fc molecules:
      GSGGGGDSGGGGSGGGGSSGGGGSG (SEQ ID NO: 195)
   long = long spacer before IgG Fc domain for homodimeric Fc molecules:
      GSGGGGDSGGGGSGGSGGSGGSGGGSGGGGSG (SEQ ID NO: 196)
   Fc = IgG Fc constant domain: 
DKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKAL
PAPIEKTISKAKGQPREPQVYTLPPSREELTKNQVSLTCLVKGFYPSDIAVEWESN
GQPENNYKTTPPVLDSDGSFFLYSKLTVDKSRWQQGNVFSCSVMHEALHNHYT
QKSLSLSPGS (SEQ ID NO:197)
   short(His) = short spacer between bivalent arms with His tag:
      GGHHHHHHHHGG (SEQ ID NO:198)
   medium(His) = medium spacer between bivalent arms with His tag:
      SGGGGSHHHHHHHHSGGGGS (SEQ ID NO: 199)
   long (His) = long spacer between bivalent arms with His tag:
      SGGGGSGGHHHHHHHHGGSGGGGS (SEQ ID NO:200)
   **BOLDED** = CDR regions of scFv

(1) *BMA*-short-Fc 
GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTA
YMELSSLRSEDTAVYYCATDLWGVGADWGQGTLVTVSSSGGGGSGGGGSSGS
GGGGDGGGGSGGTDIQMTQSPSSLSASVGDRVTITCRASQSISSYLNWYQQKPG
KAPKLLIYAASSLQSGVPSRFSGSGSGTDFTLTISSLQPEDFATYYCQQSYSTPRT
FGQGTKVDIKEGKSSGSGSESKASQVQLQESGPGLVKPSQTLSLTCTVSGGSISSD
 
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSGSGGGGDGGGGSG
DKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKAL
PAPIEKTISKAKGQPREPQVYTLPPSREELTKNQVSLTCLVKGFYPSDIAVEWESN
GQPENNYKTTPPVLDSDGSFFLYSKLTVDKSRWQQGNVFSCSVMHEALHNHYT
QKSLSLSPGS* (SEQ ID NO:201)
(2) BMA-medium-Fc 
GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTAY
MELSSLRSEDTAVYYCATDLWGVGADWGQGTLVTVSSSGGGGSGGGGSSGSG
GGGDGGGGSGGTDIQMTQSPSSLSASVGDRVTITCRASQSISSYLNWYQQKPGK
APKLLIYAASSLQSGVPSRFSGSGSGTDFTLTISSLQPEDFATYYCQQSYSTPRTF
GQGTKVDIKEGKSSGSGSESKASQVQLQESGPGLVKPSQTLSLTCTVSGGSISSD
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSGSGGGGDSGGGGS
GGGGSSGGGGSGDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLN
GKEYKCKVSNKALPAPIEKTISKAKGQPREPQVYTLPPSREELTKNQVSLTCLVK
GFYPSDIAVEWESNGQPENNYKTTPPVLDSDGSFFLYSKLTVDKSRWQQGNVFS
CSVMHEALHNHYTQKSLSLSPGS* (SEQ ID NO:202)
(3) BMA-long-Fc 
GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTAY
MELSSLRSEDTAVYYCATDLWGVGADWGQGTLVTVSSSGGGGSGGGGSSGSG
 
GGGDGGGGSGGTDIQMTQSPSSLSASVGDRVTITCRASQSISSYLNWYQQKPGK
APKLLIYAASSLQSGVPSRFSGSGSGTDFTLTISSLQPEDFATYYCQQSYSTPRTF
GQGTKVDIKEGKSSGSGSESKASQVQLQESGPGLVKPSQTLSLTCTVSGGSISSD
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSGSGGGGDSGGGGS
GGSGGSGGSGGGSGGGGSGDKTHTCPPCPAPELLGGPSVFLFPPKPKDTLMISRT
PEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREELTKNQV
SLTCLVKGFYPSDIAVEWESNGQPENNYKTTPPVLDSDGSFFLYSKLTVDKSRW
QQGNVFSCSVMHEALHNHYTQKSLSLSPGS* (SEQ ID NO:203)
(4) *ALB*-short-Fc 
GDEMGTDIQMTQSPSSLSASVGDRVTITCRASQSISSYLNWYQQKPGKAP
KLLIYAASSLQSGVPSRFSGSGSGTDFTLTISSLQPEDFATYYCQQSYSTPRTFGQ
GTKVDIKEGKSSGSGSESKASQVQLQESGPGLVKPSQTLSLTCTVSGGSISSDDY
YWSWIRQTPGKGLEWIGYIYYSGITYYNPSLKSRVTISVDTSKNQFSLKLSSVTA
ADTAVYYCAREGCNDGVCYNGVFDYWGQGTLVTVSSSGGSGGGGSSGGGGS
GGGGSSGGGGDGGGGSGGTQSALTQPASVSGSPGQSITISCTGTSSDVGGYKSVS
WYQQHPGKAPKLMIYDVSNRPSGVSDRFSGSKSGNTASLTISGLQAEDEADYYC
SSYTSSSSLWVFGGGTKLTVLGEGKSSGSGSESKASQVQLVQSGAEVKKPGSSV
KVSCKASGGTFSSYAISWVRQAPGQGLEWMGRIIPILGIANYAQKFQGRVTMTE
DTSTDTAYMELSSLRSEDTAVYYCATDLWGVGADWGQGTLVTVSSGSGGGGD
GGGGSGDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKC
KVSNKALPAPIEKTISKAKGQPREPQVYTLPPSREELTKNQVSLTCLVKGFYPSDI
AVEWESNGQPENNYKTTPPVLDSDGSFFLYSKLTVDKSRWQQGNVFSCSVMHE
ALHNHYTQKSLSLSPGS* (SEQ ID NO:204)
(5) *ALB*-medium-Fc 
GDEMGTDIQMTQSPSSLSASVGDRVTITCRASQSISSYLNWYQQKPGKAP
KLLIYAASSLQSGVPSRFSGSGSGTDFTLTISSLQPEDFATYYCQQSYSTPRTFGQ
 
GTKVDIKEGKSSGSGSESKASQVQLQESGPGLVKPSQTLSLTCTVSGGSISSDDY
YWSWIRQTPGKGLEWIGYIYYSGITYYNPSLKSRVTISVDTSKNQFSLKLSSVTA
ADTAVYYCAREGCNDGVCYNGVFDYWGQGTLVTVSSSGGSGGGGSSGGGGS
GGGGSSGGGGDGGGGSGGTQSALTQPASVSGSPGQSITISCTGTSSDVGGYKSVS
WYQQHPGKAPKLMIYDVSNRPSGVSDRFSGSKSGNTASLTISGLQAEDEADYYC
SSYTSSSSLWVFGGGTKLTVLGEGKSSGSGSESKASQVQLVQSGAEVKKPGSSV
KVSCKASGGTFSSYAISWVRQAPGQGLEWMGRIIPILGIANYAQKFQGRVTMT
EDTSTDTAYMELSSLRSEDTAVYYCATDLWGVGADWGQGTLVTVSSGSGGGG
DSGGGGSGGGGSSGGGGSGDKTHTCPPCPAPELLGGPSVFLFPPKPKDTLMISRT
PEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSREELTKNQV
SLTCLVKGFYPSDIAVEWESNGQPENNYKTTPPVLDSDGSFFLYSKLTVDKSRW
QQGNVFSCSVMHEALHNHYTQKSLSLSPGS* (SEQ ID NO:205)
(6) *ALB*-long-Fc 
GDEMGTDIQMTQSPSSLSASVGDRVTITCRASQSISSYLNWYQQKPGKAP
KLLIYAASSLQSGVPSRFSGSGSGTDFTLTISSLQPEDFATYYCQQSYSTPRTFGQ
GTKVDIKEGKSSGSGSESKASQVQLQESGPGLVKPSQTLSLTCTVSGGSISSDDY
YWSWIRQTPGKGLEWIGYIYYSGITYYNPSLKSRVTISVDTSKNQFSLKLSSVTA
ADTAVYYCAREGCNDGVCYNGVFDYWGQGTLVTVSSSGGSGGGGSSGGGGS
GGGGSSGGGGDGGGGSGGTQSALTQPASVSGSPGQSITISCTGTSSDVGGYKSVS
WYQQHPGKAPKLMIYDVSNRPSGVSDRFSGSKSGNTASLTISGLQAEDEADYYC
SSYTSSSSLWVFGGGTKLTVLGEGKSSGSGSESKASQVQLVQSGAEVKKPGSSV
KVSCKASGGTFSSYAISWVRQAPGQGLEWMGRIIPILGIANYAQKFQGRVTMT
EDTSTDTAYMELSSLRSEDTAVYYCATDLWGVGADWGQGTLVTVSSGSGGGG
DSGGGGSGGSGGSGGSGGGSGGGGSGDKTHTCPPCPAPELLGGPSVFLFPPKPKD
TLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSREE
LTKNQVSLTCLVKGFYPSDIAVEWESNGQPENNYKTTPPVLDSDGSFFLYSKLTV
DKSRWQQGNVFSCSVMHEALHNHYTQKSLSLSPGS* (SEQ ID NO:206)
(7) *BMA*-'short(His)'*-ALB* 
GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTA
YMELSSLRSEDTAVYYCATDLWGVGADWGQGTLVTVSSSGGGGSGGGGSSGS
GGGGDGGGGSGGTDIQMTQSPSSLSASVGDRVTITCRASQSISSYLNWYQQKPG
KAPKLLIYAASSLQSGVPSRFSGSGSGTDFTLTISSLQPEDFATYYCQQSYSTPRT
FGQGTKVDIKEGKSSGSGSESKASQVQLQESGPGLVKPSQTLSLTCTVSGGSISSD
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSGGHHHHHHHHGG
GTDIQMTQSPSSLSASVGDRVTITCRASQSISSYLNWYQQKPGKAPKLLIYAASS
LQSGVPSRFSGSGSGTDFTLTISSLQPEDFATYYCQQSYSTPRTFGQGTKVDIKEG
KSSGSGSESKASQVQLQESGPGLVKPSQTLSLTCTVSGGSISSDDYYWSWIRQTP
GKGLEWIGYIYYSGITYYNPSLKSRVTISVDTSKNQFSLKLSSVTAADTAVYYCA
REGCNDGVCYNGVFDYWGQGTLVTVSSSGGSGGGGSSGGGGSGGGGSSGGGG
DGGGGSGGTQSALTQPASVSGSPGQSITISCTGTSSDVGGYKSVSWYQQHPGKA
PKLMIYDVSNRPSGVSDRFSGSKSGNTASLTISGLQAEDEADYYCSSYTSSSSLW
VFGGGTKLTVLGEGKSSGSGSESKASQVQLVQSGAEVKKPGSSVKVSCKASGGT
FSSYAISWVRQAPGQGLEWMGRIIPILGIANYAQKFQGRVTMTEDTSTDTAYM
ELSSLRSEDTAVYYCATDLWGVGADWGQGTLVTVSS* (SEQ ID NO:207)
(8) *BMA*-'medium(His)'*-ALB* 
GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTA
YMELSSLRSEDTAVYYCATDLWGVGADWGQGTLVTVSSSGGGGSGGGGSSGS
GGGGDGGGGSGGTDIQMTQSPSSLSASVGDRVTITCRASQSISSYLNWYQQKPG
KAPKLLIYAASSLQSGVPSRFSGSGSGTDFTLTISSLQPEDFATYYCQQSYSTPRT
FGQGTKVDIKEGKSSGSGSESKASQVQLQESGPGLVKPSQTLSLTCTVSGGSISSD
 
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSSGGGGSHHHHHH
HSGGGGSGTDIQMTQSPSSLSASVGDRVTITCRASQSISSYLNWYQQKPGKAPKL
LIYAASSLQSGVPSRFSGSGSGTDFTLTISSLQPEDFATYYCQQSYSTPRTFGQGT
KVDIKEGKSSGSGSESKASQVQLQESGPGLVKPSQTLSLTCTVSGGSISSDDYYW
SWIRQTPGKGLEWIGYIYYSGITYYNPSLKSRVTISVDTSKNQFSLKLSSVTAAD
TAVYYCAREGCNDGVCYNGVFDYWGQGTLVTVSSSGGSGGGGSSGGGGSGG
GGSSGGGGDGGGGSGGTQSALTQPASVSGSPGQSITISCTGTSSDVGGYKSVSW
YQQHPGKAPKLMIYDVSNRPSGVSDRFSGSKSGNTASLTISGLQAEDEADYYCS
SYTSSSSLWVFGGGTKLTVLGEGKSSGSGSESKASQVQLVQSGAEVKKPGSSVK
VSCKASGGTFSSYAISWVRQAPGQGLEWMGRIIPILGIANYAQKFQGRVTMTE
DTSTDTAYMELSSLRSEDTAVYYCATDLWGVGADWGQGTLVTVSS* (SEQ ID
NO:208)
(9) *BMA*-'long(His)'*-ALB* 
GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTA
YMELSSLRSEDTAVYYCATDLWGVGADWGQGTLVTVSSSGGGGSGGGGSSGS
GGGGDGGGGSGGTDIQMTQSPSSLSASVGDRVTITCRASQSISSYLNWYQQKPG
KAPKLLIYAASSLQSGVPSRFSGSGSGTDFTLTISSLQPEDFATYYCQQSYSTPRT
FGQGTKVDIKEGKSSGSGSESKASQVQLQESGPGLVKPSQTLSLTCTVSGGSISSD
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSSGGGGSGGHHHHH
HHHGGSGGGGSGTDIQMTQSPSSLSASVGDRVTITCRASQSISSYLNWYQQKPG
KAPKLLIYAASSLQSGVPSRFSGSGSGTDFTLTISSLQPEDFATYYCQQSYSTPRT
FGQGTKVDIKEGKSSGSGSESKASQVQLQESGPGLVKPSQTLSLTCTVSGGSISSD
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSSGGSGGGGSSGGG
GSGGGGSSGGGGDGGGGSGGTQSALTQPASVSGSPGQSITISCTGTSSDVGGYKS
 
VSWYQQHPGKAPKLMIYDVSNRPSGVSDRFSGSKSGNTASLTISGLQAEDEADY
YCSSYTSSSSLWVFGGGTKLTVLGEGKSSGSGSESKASQVQLVQSGAEVKKPGS
SVKVSCKASGGTFSSYAISWVRQAPGQGLEWMGRIIPILGIANYAQKFQGRVT
MTEDTSTDTAYMELSSLRSEDTAVYYCATDLWGVGADWGQGTLVTVSS* (SEQ
ID NO:209)
(10) *BMA*-'short(His)'*-AMB*

GDEMGTQSALTQPASVSGSPGQSITISCTGTSSDVGGYKSVSWYQQHPGK
APKLMIYDVSNRPSGVSDRFSGSKSGNTASLTISGLQAEDEADYYCSSYTSSSSL
WVFGGGTKLTVLGEGKSSGSGSESKASQVQLVQSGAEVKKPGSSVKVSCKASG
GTFSSYAISWVRQAPGQGLEWMGRIIPILGIANYAQKFQGRVTMTEDTSTDTA
YMELSSLRSEDTAVYYCATDLWGVGADWGQGTLVTVSSSGGGGSGGGGSSGS
GGGGDGGGGSGGTDIQMTQSPSSLSASVGDRVTITCRASQSISSYLNWYQQKPG
KAPKLLIYAASSLQSGVPSRFSGSGSGTDFTLTISSLQPEDFATYYCQQSYSTPRT
FGQGTKVDIKEGKSSGSGSESKASQVQLQESGPGLVKPSQTLSLTCTVSGGSISSD
DYYWSWIRQTPGKGLEWIGYIYYSGITYYNPSLKSRVTISVDTSKNQFSLKLSSV
TAADTAVYYCAREGCNDGVCYNGVFDYWGQGTLVTVSSGGHHHHHHHHGG
GTDIQMTQSPSSLSASVGDRVTITCRASQSISSYLNWYQQKPGKAPKLLIYAASS
LQSGVPSRFSGSGSGTDFTLTISSLQPEDFATYYCQQSYSTPRTFGQGTKVDIKEG
KSSGSGSESKASQVQLQESGPGLVKPSQTLSLTCTVSGGSISSDDYYWSWIRQTP
GKGLEWIGYIYYSGITYYNPSLKSRVTISVDTSKNQFSLKLSSVTAADTAVYYCA
REGCNDGVCYNGVFDYWGQGTLVTVSSSGGSGGGGSSGSGGGGDGGGGSGG
TQSALTQPASVSGSPGQSITISCTGTSSDVGGYKSVSWYQQHPGKAPKLMIYDVS
NRPSGVSDRFSGSKSGNTASLTISGLQAEDEADYYCSSYTSSSSLWVFGGGTKLT
VLGEGKSSGSGSESKASQVQLVQSGAEVKKPGSSVKVSCKASGGTFSSYAISWV
RQAPGQGLEWMGRIIPILGIANYAQKFQGRVTMTEDTSTDTAYMELSSLRSEDT
AVYYCATDLWGVGADWGQGTLVTVSS* (SEQ ID NO:210)

### References Cited

1. Agarwal, S., et al. Strategic Targeting of Multiple BMP Receptors Prevents Trauma-Induced Heterotopic Ossification. Mol Ther 25, 1974-1987 (2017).
2. Dey, D., Goldhamer, D.J. & Yu, P.B. Contributions of Muscle-Resident Progenitor Cells to Homeostasis and Disease. Curr Mol Bio Rep, 1-14 (2015).
3. Dey, D., et al. Two tissue-resident progenitor lineages drive distinct phenotypes of heterotopic ossification. Sci Transl Med 8, 366ra163 (2016).
4. Shen, H., Gelberman, R.H., Silva, M.J., Sakiyama-Elbert, S.E. & Thomopoulos, S. BMP12 induces tenogenic differentiation of adipose-derived stromal cells. PLoS One 8, e77613 (2013).
5. Lin, S., Svoboda, K.K., Feng, J.Q. & Jiang, X. The biological function of type I receptors of bone morphogenetic protein in bone. Bone Res 4, 16005 (2016).
6. David, L., Mallet, C., Mazerbourg, S., Feige, J.J. & Bailly, S. Identification of BMP9 and BMP10 as functional activators of the orphan activin receptor-like kinase 1 (ALK1) in endothelial cells. Blood 109, 1953-1961 (2007).
7. Long, L., et al. Selective enhancement of endothelial BMPR-II with BMP9 reverses pulmonary arterial hypertension. Nature medicine 21, 777-785 (2015).
8. Dinter, T., Bocobo, G.A. & Yu, P.B. Pharmacologic Strategies for Assaying BMP Signaling Function. Methods Mol Biol 1891, 221-233 (2019).
9. Choi, E.J., et al. Enhanced responses to angiogenic cues underlie the pathogenesis of hereditary hemorrhagic telangiectasia 2. PLoS One 8, e63138 (2013).
10. Luu, H.H., et al. Distinct roles of bone morphogenetic proteins in osteogenic differentiation of mesenchymal stem cells. J Orthop Res 25, 665-677 (2007).
11. Leblanc, E., et al. BMP9-Induced muscle heterotopic ossification requires changes to the skeletal muscle microenvironment. J Bone Miner Res (2010).
12. Luo, J., et al. TGFbeta/BMP type I receptors ALK1 and ALK2 are essential for BMP9-induced osteogenic signaling in mesenchymal stem cells. J Biol Chem 285, 29588-29598 (2010).
13. Salingcarnboriboon, R., et al. Establishment of tendon-derived cell lines exhibiting pluripotent mesenchymal stem cell-like property. Exp Cell Res 287, 289-300 (2003).
14. Lejard, V., et al. Scleraxis and NFATc regulate the expression of the pro-alpha1(I) collagen gene in tendon fibroblasts. J Biol Chem 282, 17665-17675 (2007).
15. Berasi, S.P., et al. Divergent activities of osteogenic BMP2, and tenogenic BMP12 and BMP13 independent of receptor binding affinities. Growth Factors 29, 128-139 (2011).
16. Andersson, O., Korach-Andre, M., Reissmann, E., Ibanez, C.F. & Bertolino, P. Growth/differentiation factor 3 signals through ALK7 and regulates accumulation of adipose tissue and diet-induced obesity. Proc Natl Acad Sci U S A 105, 7252-7256 (2008).

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.
The invention will now be defined by reference to the following clauses:
1. A multi- or bi-specific antibody molecule, comprising at least:
   a first antigen binding domain that binds to a bone morphogenetic protein receptor (BMPR) type I (BMPRI); and
   a second antigen binding domain that binds to a bone morphogenetic protein receptor (BMPR) type II (BMPRII),
   wherein the first and second antigen binding domains are linked together by a flexible linker, and can be in any order,
   wherein each antigen binding domain can agonize the BMPR to which it binds, and
   preferably wherein each antigen binding domain is an scFv.
2. The antibody molecule of clause 1, wherein binding of the antibody molecule to a cell initiates BMP/TGF-beta/activin/GDF signaling.
3. The antibody molecule of clause 1, wherein the first antigen binding domain binds to a BMPRI selected from the group consisting of ALK1 (ACVRL1); ALK2 (ACVR1A); ALK3 (BMPR1A); ALK4 (ACVR1B); ALK5 (TGFBR1); ALK6 (BMPR1B); or ALK7 (ACVR1C).
4. The antibody molecule of clause 3, wherein the first antigen binding domain binds to ALK1 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 146, 148, 150, or 152; comprises VH and/or VL sequences at least 95% identical to sequences in Fig. 7; or comprises a sequence at least 95% identical to SEQ ID NO: 146, 148, 150, or 152.
5. The antibody molecule of clause 3, wherein the first antigen binding domain binds to ALK2 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 160, 162, 164, 166, or 168; comprises VH and/or VL sequences at least 95% identical to sequences in Fig. 7; or comprises a sequence at least 95% identical to SEQ ID NO:160, 162, 164, 166, or 168.
6. The antibody molecule of clause 3, wherein the first antigen binding domain binds to ALK3 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 156 or 158; comprises VH and/or VL sequences at least 95% identical to sequences in Fig. 7; or comprises a sequence at least 95% identical to SEQ ID NO:156 or 158.
7. The antibody molecule of clause 3, wherein the first antigen binding domain binds to ALK4 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:178; comprises VH and/or VL sequences at least 95% identical to sequences in Fig. 7; or comprises a sequence at least 95% identical to SEQ ID NO:178.
8. The antibody molecule of clause 3, wherein the first antigen binding domain binds to ALK6 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:180; comprises VH and/or VL sequences at least 95% identical to sequences in Fig. 7; or comprises a sequence at least 95% identical to SEQ ID NO:180.
9. The antibody molecule of clause 3, wherein the first antigen binding domain binds to ALK7 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 182, 184, 186, or 188; comprises VH and/or VL sequences at least 95% identical to sequences in Fig. 7; or comprises a sequence at least 95% identical to SEQ ID NOs: 182, 184, 186, or 188.
10. The antibody molecule of clause 1, wherein the second antigen binding domain binds to a BMPRII selected from the group consisting of ACTRIIA (ACVR2A); ACTRIIB (ACVR2B); BMPRII (BMPR2); TGFBRII (TGFBR2); or AMEIRII (AMHR2).
11. The antibody molecule of clause 10, wherein the second antigen binding domain binds to BMPR2 and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:154 or 212; comprises VH and/or VL sequences at least 95% identical to sequences in Fig. 7; or comprises a sequence at least 95% identical to SEQ ID NO: 154 or 212.
12. The antibody molecule of clause 10, wherein the second antigen binding domain binds to ACTRIIA (ACVR2A) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs:170 and 172; comprises VH and/or VL sequences at least 95% identical to sequences in Fig. 7; or comprises a sequence at least 95% identical to SEQ ID NOs: 170 or 172.
13. The antibody molecule of clause 10, wherein the second antigen binding domain binds to ACTRIIB (ACVR2B) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs:174 or176; comprises VH and/or VL sequences at least 95% identical to sequences in Fig. 7; or comprises a sequence at least 95% identical to SEQ ID NOs: 174 or 176.
14. The antibody molecule of clause 1, wherein:
   (i) the first antigen binding domain binds to BMPR2 and comprises CDR sequences at least 95% identical to CDR sequences in Table 1 or Fig. 7, and/or the second antigen binding domain binds to ALK1, ALK2, ALK3, ALK4, ALK5, ALK6, and ALK7and comprises CDR sequences at least 95% identical to CDR sequences in Table 1 or Fig. 7;
   (ii) the first antigen binding domain binds to ACVR2A and comprises CDR sequences at least 95% identical to CDR sequences in Table 1 or Fig. 7, and/or the second
      antigen binding domain binds to ALK1, ALK2, ALK3, ALK4, ALK5, ALK6, and ALK7 and comprises CDR sequences at least 95% identical to CDR sequences in Table 1 or Fig. 7;
   (iii) the first antigen binding domain binds to ACVR2B and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7;
   (iv) the first antigen binding domain binds to TGFBR2 and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7;
   (v) the first antigen binding domain binds to AIVIHR2 and comprises CDR sequences at least 95% identical to CDR sequences in Table 1 or Fig. 7, and/or the second antigen binding domain binds to ALK1, ALK2, ALK3, ALK4, ALK5, ALK6, and ALK7 and comprises CDR sequences at least 95% identical to CDR sequences in Table 1 or Fig. 7.
15.An antibody or antigen binding portion thereof that binds to ALK1 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 146, 148, 150, or 152; comprises VH and/or VL sequences at least 95% identical to sequences in Fig. 7; or comprises a sequence at least 95% identical to SEQ ID NOs: 146, 148, 150, or 152.
16.An antibody or antigen binding portion thereof that binds to ALK2 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 160, 162, 164, 166, or 168; comprises VH and/or VL sequences at least 95% identical to sequences in Fig. 7; or comprises a sequence at least 95% identical to SEQ ID NOs: 160, 162, 164, 166, or 168.
17. An antibody or antigen binding portion thereof that binds to ALK3 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 156 or 158; comprises VH and/or VL sequences at least 95% identical to sequences in Fig. 7; or comprises a sequence at least 95% identical to SEQ ID NO: 156 or 158.
18. An antibody or antigen binding portion thereof that binds to ALK4 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 178; comprises VH and/or VL sequences at least 95% identical to sequences in Fig. 7; or comprises a sequence at least 95% identical to SEQ ID NO: 178.
19. An antibody or antigen binding portion thereof that binds to ALK6 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO:180; comprises VH and/or VL sequences at least 95% identical to sequences in Fig. 7; or comprises a sequence at least 95% identical to SEQ ID NO: 180.
20. An antibody or antigen binding portion thereof that binds to ALK7 and optionally comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NOs: 182, 184, 186, or 188; comprises VH and/or VL sequences at least 95% identical to sequences in Fig. 7; or comprises a sequence at least 95% identical to SEQ ID NO: 182, 184, 186, or 188.
21. An antibody or antigen binding portion thereof that binds to BMPR2 and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 154 or 212; comprises VH and/or VL sequences at least 95% identical to sequences in Fig. 7; or comprises a sequence at least 95% identical to SEQ ID NO: 154 or 212.
22. An antibody or antigen binding portion thereof that binds to ACTRIIA (ACVR2A) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 170 or 172; comprises VH and/or VL sequences at least 95% identical to sequences in Fig. 7; or comprises a sequence at least 95% identical to SEQ ID NO: 170 and 172.
23. An antibody or antigen binding portion thereof that binds to ACTRIIB (ACVR2B) and comprises CDR sequences at least 95% identical to CDR sequences in Table 1 or Fig. 7; comprises VH CDRs 1, 2, 3 and VL CDRs 1, 2, 3 that are identical to complementary determining regions in SEQ ID NO: 174 or 176; comprises VH and/or VL sequences at least 95% identical to sequences in Fig. 7; or comprises a sequence at least 95% identical to SEQ ID NO: 174 or 176.
24. The antibody molecule of clauses 1-14, or the antibody or antigen binding portion thereof of clauses 15-23, for use in a method of treating a vascular condition in a subject, optionally wherein the vascular condition is pulmonary arterial hypertension or hereditary hemorrhagic telangiectasia (HHT) syndrome.
25. The antibody molecule of clauses 1-14, or the antibody or antigen binding portion thereof of clauses 15-23, for use in a method of treating a pulmonary vascular leak syndrome, optionally wherein the pulmonary vascular leak syndrome is Acute Respiratory Distress Syndrome (ARDS) or Acute Lung Injury (ALI).
26. The antibody molecule of clauses 1-14, or the antibody or antigen binding portion thereof of clauses 15-23, for use in a method of treating liver fibrosis in a subject with deficient intrahepatic BMP9 signaling.
27. A nucleic acid encoding the antibody molecule of clauses 1-14 or the antibody or antigen binding portion thereof of clauses 15-23.
28. A host cell comprising the nucleic acid of clause 11, and optionally expressing the antibody molecule of clauses 1-14 or the antibody or antigen binding portion thereof of clauses 15-23.
29. A pharmaceutical composition comprising the antibody molecule of clauses 1-14 or the antibody or antigen binding portion thereof of clauses 15-23.
30. A method of treating a vascular condition in a subject, optionally wherein the vascular condition is pulmonary arterial hypertension or hereditary hemorrhagic telangiectasia (HHT) syndrome, the method comprising administering a therapeutically effective amount of the antibody molecule of clauses 1-14 or the antibody or antigen binding portion thereof of clauses 15-23 to a subject in need thereof.
31. A method of treating a pulmonary vascular leak syndrome, optionally wherein the pulmonary vascular leak syndrome is Acute Respiratory Distress Syndrome (ARDS) or Acute Lung Injury (ALI), the method comprising administering a therapeutically effective amount of the antibody molecule of clauses 1-14 or the antibody or antigen binding portion thereof of clauses 15-23 to a subject in need thereof.
32. A method of treating liver fibrosis in a subject with deficient intrahepatic BMP9 signaling, the method comprising administering a therapeutically effective amount of the antibody molecule of clauses 1-14 or the antibody or antigen binding portion thereof of clauses 15-23 to a subject in need thereof.

## Claims

1. A tetraspecific construct comprising a pair of molecules,
(i) each molecule comprising:
a first antigen binding domain that binds to a bone morphogenetic protein receptor (BMPR) type I (BMPRI); and
a second antigen binding domain that binds to a bone morphogenetic protein receptor (BMPR) type II (BMPRII);
wherein the first and second antigen binding domains are linked together by a flexible linker, and can be in any order;
wherein the first or second antigen binding domain is linked to an Fc domain by a flexible linker; and
wherein the molecules are fused by disulfide bonds at the Fc domains to form a tetraspecific construct; or
(ii) a first molecule comprising a pair of first antigen binding domains that bind to a BMPRI linked together by a flexible linker, wherein one of the antigen binding domains is linked to an Fc domain by a flexible linker; and
a second molecule comprising a pair of second antigen binding domains that bind to a BMPRII linked together by a flexible linker, wherein one of the antigen binding domains is linked to an Fc domain by a flexible linker; and
wherein the molecules are fused by disulfide bonds at the Fc domains to form a tetraspecific construct.

2. The tetraspecific construct of claim 1, wherein each antigen binding domain is an scFv.

3. The tetraspecific construct of claim 1, wherein binding of the tetraspecific construct to a cell initiates BMP/TGF-beta/activin/GDF signaling in the cell.

4. The tetraspecific construct of claim 1, wherein the first antigen binding domains bind to a BMPRI selected from the group consisting of ALK1 (ACVRL1); ALK2 (ACVR1A); ALK3 (BMPR1A); ALK4 (ACVR1B); ALK5 (TGFBR1); ALK6 (BMPR1B); or ALK7 (ACVR1C).

5. The tetraspecific construct of claim 4, wherein the first antigen binding domain binds to ALK1 and optionally comprises CDR sequences at least 95% identical to SEQ ID NOs: 544, 549, 554, 557, 560 and 45; 564, 569, 574, 577, 580, and 582; 544, 549, 554, 585, 588, and 590; or 592, 597, 602, 605, 608, and 610; comprises VH CDRs 1, 2, 3 and VL CDRs 1, 2, 3 that are identical to complementary determining regions in SEQ ID NOs: 146, 148, 150, or 152; comprises VH and/or VL sequences at least 95% identical to SEQ ID NOs. 562 and 563; 583 and 584; 562 and 591, or 611 and 612; or comprises a sequence at least 95% identical to SEQ ID NO:146, 148, 150, or 152.

6. The tetraspecific construct of claims 1, wherein the second antigen binding domain binds to a BMPRII selected from the group consisting of BMPRII (BMPR2); ACTRIIA (ACVR2A); ACTRIIB (ACVR2B); TGFBRII (TGFBR2); or AMHRII (AMHR2).

7. The tetraspecific construct of claim 6, wherein the second antigen binding domain binds to BMPR2 and comprises CDR sequences at least 95% identical to SEQ ID NOs:1-6; 7-12; 13-18; 19-24; 503, 508, 513, 516, 519, or 521; or 394, 529, 534, 115, 116, or 117; comprises VH CDRs 1, 2, 3 and VL CDRs 1, 2, 3 that are identical to complementary determining regions in SEQ ID NO:154 or 212; comprises VH and/or VL sequences at least 95% identical to SEQ ID NO: 523 and 524, or 542 and 543; or comprises a sequence at least 95% identical to SEQ ID NO: 154 or 212.

8. The tetraspecific construct of claim 1, wherein the first antigen binding domain binds to ALK1 (ACVRL1) and comprises CDR sequences at least 95% identical to SEQ ID NOs: 1-6; 7-12; 13-18; 19-24; 544, 549, 554, 557, 560 and 45; 564, 569, 574, 577, 580, and 582; 544, 549, 554, 585, 588, and 590; or 592, 597, 602, 605, 608, and 610, and the second antigen binding domain binds to BMPRII (BMPR2) and comprises CDR sequences at least 95% identical to SEQ ID NO:25-30; 31-36; 503, 508, 513, 516, 519, or 521; or 394, 529, 534, 115, 116, or 117.

9. An antibody or antigen binding portion thereof that binds to:
(i)ALK1 and comprises CDR sequences at least 95% identical to SEQ ID NOs: 1-6; 7-12; 13-18; 19-24; 544, 549, 554, 557, 560 and 45; 564, 569, 574, 577, 580, and 582; 544, 549, 554, 585, 588, and 590; or 592, 597, 602, 605, 608, and 610; comprises VH CDRs 1, 2, 3 and VL CDRs 1, 2, 3 that are identical to complementary determining regions in SEQ ID NOs: 146, 148, 150, or 152; comprises VH and/or VL sequences at least 95% identical to SEQ ID NOs:562 and 563; 583 and 584; 562 and 591, or 611 and 612; or comprises a sequence at least 95% identical to SEQ ID NOs: 146, 148, 150, or 152; or
(ii) BMPR2 and comprises CDR sequences at least 95% identical to SEQ ID NOs: 25-30; 31-36; 503, 508, 513, 516, 519, or 521; or 394, 529, 534, 115, 116, or 117; comprises VH CDRs 1, 2, 3 and VL CDRs 1, 2, 3 that are identical to complementary determining regions in SEQ ID NO:154 or 212; comprises VH and/or VL sequences at least 95% identical to SEQ ID NO: 523 and 524, or 542 and 543; or comprises a sequence at least 95% identical to SEQ ID NO: 154 or 212.

10. The tetraspecific construct of claims 1-8, or the antibody or antigen binding portion thereof of claim 9, for use in a method of treating a vascular condition in a subject, optionally wherein the vascular condition is pulmonary arterial hypertension or hereditary hemorrhagic telangiectasia (HHT) syndrome.

11. The tetraspecific construct of claims 1-8, or the antibody or antigen binding portion thereof of claim 9, for use in a method of treating a pulmonary vascular leak syndrome, optionally wherein the pulmonary vascular leak syndrome is Acute Respiratory Distress Syndrome (ARDS) or Acute Lung Injury (ALI).

12. The tetraspecific construct of claims 1-8, or the antibody or antigen binding portion thereof of claim 9, for use in a method of treating liver fibrosis in a subject with deficient intrahepatic BMP9 signaling.

13. A nucleic acid encoding the tetraspecific construct of claims 1-8 or the antibody or antigen binding portion thereof of claim 9.

14. A host cell comprising the nucleic acid of claim 13, and optionally expressing the tetraspecific construct of claims 1-8 or the antibody or antigen binding portion thereof of claim 9.

15. A pharmaceutical composition comprising the tetraspecific construct of claims 1-8 or the antibody or antigen binding portion thereof of claim 9.
